# EUROPEAN PATENT SPECIFICATION

(11) **EP 2 155 747 B1**
(45) Date of publication and mention of the grant of the patent: **24.10.2012**
(21) Application number: 08769433.7
(22) Date of filing: 09.05.2008
(51) Int. Cl.: C07D 471/04, C07D 487/04, A61P 29/00, A61P 25/00, A61K 31/437, A61K 31/4985, A61K 31/519

(54) **IMIDAZOL (1,2-A)PYRIDINES AND RELATED COMPOUNDS WITH ACTIVITY AT CANNABINOID CB2 RECEPTORS**
IMIDAZOL (1,2-A)PYRIDINE UND ZUGEHÖRIGE VERBINDUNGEN MIT AKTIVITÄT AN CANNABINOID-CB2-REZPTOREN
IMIDAZOL (1,2-A)PYRIDINES ET COMPOSÉS ASSOCIÉS À ACTIVITÉ AU NIVEAU DES RÉCEPTEURS CANNABINOÏDES CB2

(30) Priority: 10.05.2007 US 917318 P; 08.06.2007 US 942746 P; 18.09.2007 US 973410 P
(43) Date of publication of application: 24.02.2010
(73) Proprietor: GE Healthcare Limited, Little Chalfont HP7 9NA (GB)
(72) Inventor: OLSSON, Roger, S-23044 Bunkeflostrand (SE); BURSTEIN, Ethan, San Diego, CA 92130 (US); KNAPP, Anne, Eeg, DK-1804 Frederiksberg C (DK); ESKILDSEN, Jorgen, DK-2300 Copenhagen S (DK); CASTILLO, Joel, S-211 28 Malmö (SE); NAIRNE, James, Amersham HP7 9LL (GB); IVESON, Veronique, Morisson, Amersham HP7 9LL (GB); ROBINS, Edward, London W12 0NN (GB); GIBSON, Alex, Amersham HP7 9LL (GB)
(74) Representative: Canning, Lewis R.
(86) International application number: PCT/US2008/063343
(87) International publication number: WO 2008/141249

(56) References cited:
- WO-A-2006/101455
- MIRONOV M A ET AL: "New catalytic system for the synthesis of imidazo[1,2-a]pyridines by the Ugi reaction" RUSSIAN CHEMICAL BULLETIN, KLUWER ACADEMIC PUBLISHERS-PLENUM PUBLISHERS, NE, vol. 55, no. 10, 1 October 2006 (2006-10-01), pages 1835-1839, XP019484107 ISSN: 1573-9171
- DATABASE CHEMCATS CHEMICAL ABSTRACTS SERVICE, COLUMBUS, OHIO, US; 7 April 2006 (2006-04-07), XP007905456 & "New Chemistry Horizons Laboratories Screening Library" 3 March 2008 (2008-03-03), NEW CHEMISTRY HORIZONS LABORATORIES LTD. POB 130 MOSCOW, 115522 RUSSIA
- DATABASE CHEMCATS CHEMICAL ABSTRACTS SERVICE, COLUMBUS, OHIO, US; 16 August 2006 (2006-08-16), XP007905454 & "Aurora Screening Library" 13 June 2008 (2008-06-13), AURORA FINE CHEMICALS LLC 7929- SILVERTON AVE. SUITE 609 SAN DIEGO, CA, 92126 USA
- DATABASE CHEMCATS CHEMICAL ABSTRACTS SERVICE, COLUMBUS, OHIO, US; 15 August 2006 (2006-08-15), XP007905453 & "Aurora Screening Library" 13 June 2008 (2008-06-13), AURORA FINE CHEMICALS LLC 7929- SILVERTON AVE. SUITE 609 SAN DIEGO, CA, 92126 USA
- DATABASE CHEMCATS CHEMICAL ABSTRACTS SERVICE, COLUMBUS, OHIO, US; 23 April 2004 (2004-04-23), XP007905455 & "Aurora Screening Library" 13 June 2008 (2008-06-13), AURORA FINE CHEMICALS LLC 7929- SILVERTON AVE. SUITE 609 SAN DIEGO, CA, 92126 USA
- KATOCH-ROUSE R ET AL: "Synthesis of N-(piperidin-1-yl)-5-(4-methoxyphenyl)-1-( 2-chloropheny" JOURNAL OF LABELLED COMPOUNDS AND RADIOPHARMACEUTICALS, JOHN WILEY, CHICHESTER, GB, vol. 46, no. 1, 1 January 2003 (2003-01-01), pages 93-98, XP008095423 ISSN: 0362-4803
- VOGEL: 'Drug discovery and evaluation: safety and pharmacokinetic assays',

### SEQUENCE LISTING

<110> ACADIA PHARMACUETICALS INC.
   GE Healthcare
   Olsson, Roger
   Burstein , Ethan
   Knapp, Anne Eeg
   Eskildsen, Jorgen Castillo, Joel
   Nairne, James
   Iveson, Veronique Morrison Robins, Edward
   Gibson, Alex
<120> COMPOUNDS WITH ACTIVITY AT CANNABINOID RECEPTORS
<130> ACADIA.131VPC
<140> Not Known
   <141> 2008-05-09
<150> 60/917,318
   <151> 2007-05-10
<150> 60/942,746
   <151> 2007-06-08
<150> 60/973,410
   <151> 2007-09-18
<160> 2
<170> PatentIn version 3.5
<210> 1
   <211> 1083
   <212> DNA
   <213> Homo sapiens
<400> 1
   atggaggaat gctgggtgac agagatagcc aatggctcca aggatggctt ggattccaac      60
   cctatgaagg attacatgat cctgagtggt ccccagaaga cagctgttgc tgtgttgtgc     120
   actcttctgg gcctgctaag tgccctggag aacgtggctg tgctctatct gatcctgtcc     180
   tcccaccaac tccgccggaa gccctcatac ctgttcattg gcagcttggc tggggctgac     240
   ttcctggcca gtgtggtctt tgcatgcagc tttgtgaatt tccatgtttt ccatggtgtg     300
   gattccaagg ctgtcttcct gctgaagatt ggcagcgtga ctatgacctt cacagcctct     360
   gtgggtagcc tcctgctgac cgccattgac cgatacctct gcctgcgcta tccaccttcc     420
   tacaaagctc tgctcacccg tggaagggca ctggtgaccc tgggcatcat gtgggtcctc     480
   tcagcactag tctcctacct gcccctcatg ggatggactt gctgtcccag gccctgctct     540
   gagcttttcc cactgatccc caatgactac ctgctgagct ggctcctgtt catcgccttc     600
   ctcttttccg gaatcatcta cacctatggg catgttctct ggaaggccca tcagcatgtg     660
   gccagcttgt ctggccacca ggacaggcag gtgccaggaa tggcccgaat gaggctggat     720
   gtgaggttgg ccaagaccct agggctagtg ttggctgtgc tcctcatctg ttggttccca     780
   gtgctggccc tcatggccca cagcctggcc actacgctca gtgaccaggt caagaaggcc     840
   tttgctttct gctccatgct gtgcctcatc aactccatgg tcaaccctgt catctatgct     900
   ctacggagtg gagagatccg ctcctctgcc catcactgcc tggctcactg gaagaagtgt     960
   gtgaggggcc ttgggtcaga ggcaaaagaa gaagccccga gatcctcagt caccgagaca    1020
   gaggctgatg ggaaaatcac tccgtggcca gattccagag atctagacct ctctgattgc    1080
   tga                                                                  1083
<210> 2
   <211> 360
   <212> PRT
   <213> Homo sapiens
<400> 2
   Met Glu Glu Cys Trp Val Thr Glu Ile Ala Asn Gly Ser Lys Asp Gly
   1               5                   10                  15
   Leu Asp Ser Asn Pro Met Lys Asp Tyr Met Ile Leu Ser Gly Pro Gln
               20                  25                  30
   Lys Thr Ala Val Ala Val Leu Cys Thr Leu Leu Gly Leu Leu Ser Ala
           35                  40                  45
   Leu Glu Asn Val Ala Val Leu Tyr Leu Ile Leu Ser Ser His Gln Leu
       50                  55                  60
   Arg Arg Lys Pro Ser Tyr Leu Phe Ile Gly Ser Leu Ala Gly Ala Asp
   65                  70                  75                  80
   Phe Leu Ala Ser Val Val Phe Ala Cys Ser Phe Val Asn Phe His Val
                   85                  90                  95
   Phe His Gly Val Asp Ser Lys Ala Val Phe Leu Leu Lys Ile Gly Ser
               100                 105                 110
   Val Thr Met Thr Phe Thr Ala Ser Val Gly Ser Leu Leu Leu Thr Ala
           115                 120                 125
   Ile Asp Arg Tyr Leu Cys Leu Arg Tyr Pro Pro Ser Tyr Lys Ala Leu
       130                 135                 140
   Leu Thr Arg Gly Arg Ala Leu Val Thr Leu Gly Ile Met Trp Val Leu
   145                 150                 155                 160
   Ser Ala Leu Val Ser Tyr Leu Pro Leu Met Gly Trp Thr Cys Cys Pro
                   165                 170                 175
   Arg Pro Cys Ser Glu Leu Phe Pro Leu Ile Pro Asn Asp Tyr Leu Leu
               180                 185                 190
   Ser Trp Leu Leu Phe Ile Ala Phe Leu Phe Ser Gly Ile Ile Tyr Thr
           195                 200                 205
   Tyr Gly His Val Leu Trp Lys Ala His Gln His Val Ala Ser Leu Ser
       210                 215                 220
   Gly His Gln Asp Arg Gln Val Pro Gly Met Ala Arg Met Arg Leu Asp
   225                 230                 235                 240
   Val Arg Leu Ala Lys Thr Leu Gly Leu Val Leu Ala Val Leu Leu Ile
                   245                 250                 255
   Cys Trp Phe Pro Val Leu Ala Leu Met Ala His Ser Leu Ala Thr Thr
               260                 265                 270
   Leu Ser Asp Gln Val Lys Lys Ala Phe Ala Phe Cys Ser Met Leu Cys
           275                 280                 285
   Leu Ile Asn Ser Met Val Asn Pro Val Ile Tyr Ala Leu Arg Ser Gly
       290                 295                 300
   Glu Ile Arg Ser Ser Ala His His Cys Leu Ala His Trp Lys Lys Cys
   305                 310                 315                 320
   Val Arg Gly Leu Gly Ser Glu Ala Lys Glu Glu Ala Pro Arg Ser Ser
                   325                 330                 335
   Val Thr Glu Thr Glu Ala Asp Gly Lys Ile Thr Pro Trp Pro Asp Ser
               340                 345                 350
   Arg Asp Leu Asp Leu Ser Asp Cys
           355                 360

## Description

### RELATED APPLICATIONS

This application claims priority to the U.S. Provisional Application Serial No. 60/917,318, filed on May 10, 2007, by Ethan Burstein et aL, and entitled "COMPOUNDS WITH ACTIVITY AT CANNABINOID CB2 RECEPTORS" (ACADIA.119PR), and the U.S. Provisional Application Serial No. 60/942,746, filed on June 8, 2007, by Ethan Burstein et al., and entitled "COMPOUNDS WITH ACTIVITY AT CANNABINOID CB2 RECEPTORS" (ACADIA.119PR2) and the U.S. Provisional Application Serial No. 60/973,410, filed on September 18, 2007, by Ethan Burstein et al., and entitled "COMPOUNDS WITH ACTIVITY AT CANNABINOID CB2 RECEPTORS" (ACADIA.119PR3), all of which are incorporated by reference herein in their entirety, including any drawings.

### FIELD OF THE INVENTION

The present invention is in the field of pharmaceuticals, and in particular in the field of compounds that bind to cannabinoid CB2 receptors and diagnosis of diseases with these compounds.

### BACKGROUND

The cannabinoids, which are bioactive lipids found in the cannabis sativa (marijuana) plant, have been used recreationally and therapeutically for at least 5000 years. In addition to their well-documented effects on mood, cannabinoids (often in the form of marijuana) have been prescribed to treat nausea, pain, migraine, epilepsy, glaucoma, hypertension, cachexia and pain associated with childbirth. Two cannabinoid receptors, CB1 and CB2, have been identified (reviewed in Howlett et al., 2004). Both are members of the G protein-coupled receptor superfamily, and are negatively coupled through Gi protein. The CB2 receptor has 44% sequence similarity to the CB1 receptor. Radiotracers are known for studying CB1 receptors (Katoch-Rouse et al 2003 J Lab Comp Radiopharm; 46(1): 93-98).

CB2 cannabinoid receptors were first cloned from differentiated human HL-60 myeloid cells, and are most highly expressed in spleen (Monro et al, 1993), and cells of the immune system such as B cells, T cells, natural killer cells, macrophages, monocytes, and neutrophils (Galiegue et al, 1995; Carlisle et al, 2002; Lee et al, 2001; Ueda et al, 2005). Lower levels of CB2 receptors are also found in epidermis including keratinocytes, hair follicles, sebocytes, and sweat glands (Stander et al, 2005; Ibrahim et al, 2005; Walczak et al, 2005), as well as osteoblasts, osteoclasts, and osteocytes (Ofec et al, 2006), and stomach, lung, heart and testis (Onaivi et al, 2006). CB2 receptor expression has been reported in dorsal root ganglion (DRG) neurons (Ross et al, 2001; Beltramo et al, 2006; Walczak et al, 2005; Wotherspoon et al, 2005), and evidence for CB2 receptor expression in other peripheral neurons such as C- and Adelta-fibers has been reported (Martin et al, 2000; Patel et al, 2003; Yoshihara et al; 2004; Elmes et al, 2004). Recently CB2 receptor expression within the CNS has been described, at both the spinal and supraspinal levels. Specifically, CB2 receptors are found in lumbar (L3-14) spinal cord (Beltramo et al, 2006; Walczak et al, 2005), and in cerebellar granule neurons (Skaper et al, 1996), cerebrovascular epithelium (Golech et al, 2004), microglia (Klegeris et al, 2003) and neurons of the brainstern (striatum, thalamic nuclei, hippocampus, amygdala, substantia nigra, periaqueductal gray, spinal trigeminal nucleus etc.), cortex and cerebellum (Ashton et al, 2006; Gong et al, 2006; Van Sickle et al; 2005).

CB2 receptors have been implicated in a number of physiological processes including inflammation and perception of pain (Whiteside et al, 2007), immune system regulation (Sipe et al, 2005), neurogenesis (Palazuelos et al, 2006), and bone physiology (Karsak et al, 2005). Upregulation of CB2 receptors is associated with certain pathophysiological states. Increased CB2 receptor expression has been detected in dorsal horn of the spinal cord as well as primary afferent, C-fiber neurons in chronic constriction injury (CCI), spinal nerve ligation (SNL), complete sciatic nerve section, and saphenous nerve partial ligation models of neuropathic pain (Zhang et al, 2003; Walczak et al, 2005; Wotherspoon et al, 2005). CB2 receptors are upregulated in microglia and astrocytes from neuritic plaques found in Alzheimer's diseased brains (Benito et al, 2003), or by interferon gamma (Carlisle et al, 2002) or lipopolysaccharide (Cabral et al, 2005), and in T-lymphocytes from simian immunodeficiency virus-infected macaques (Benito et al, 2005). CB2 receptors are found in T-lymphocytes, astrocytes and perivascular and reactive microglia in multiple sclerosis plaques (Benito et al, 2007).

### SUMMARY OF THE INVENTION

Disclosed herein is a compound of Formula I: 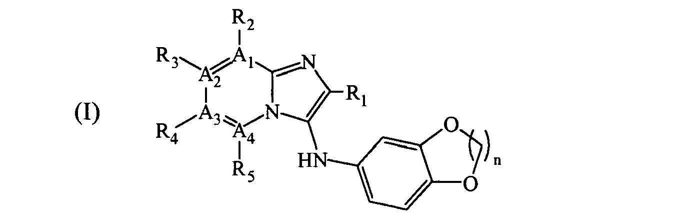 or a pharmaceutically acceptable salt thereof, wherein:
a) the 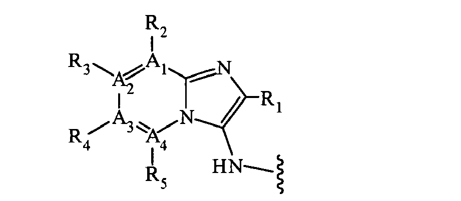 moiety is selected from the group consisting of 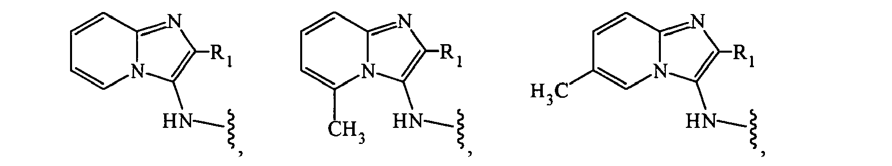 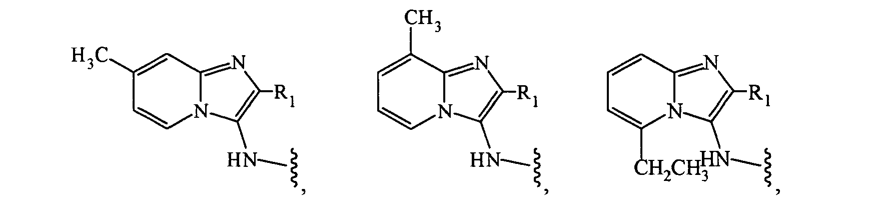 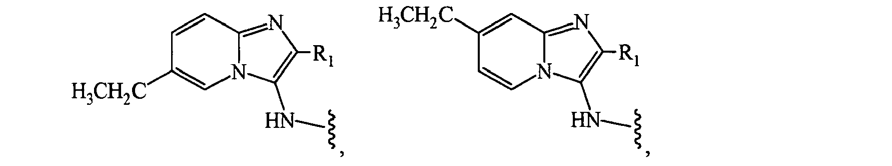 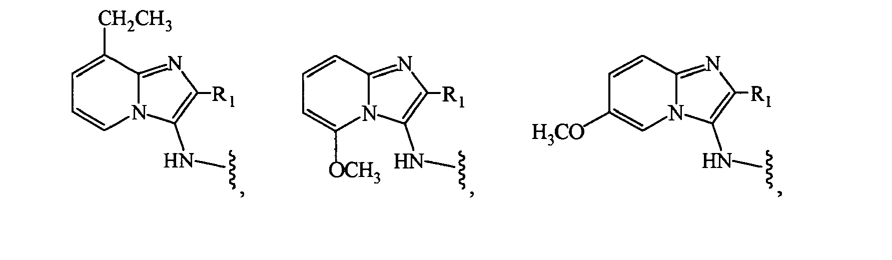 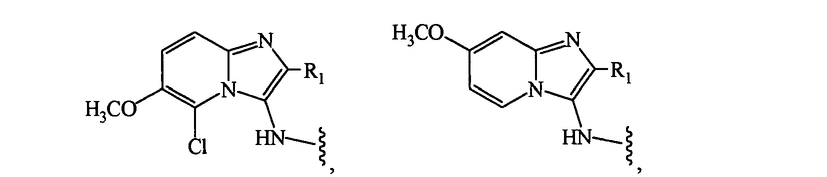 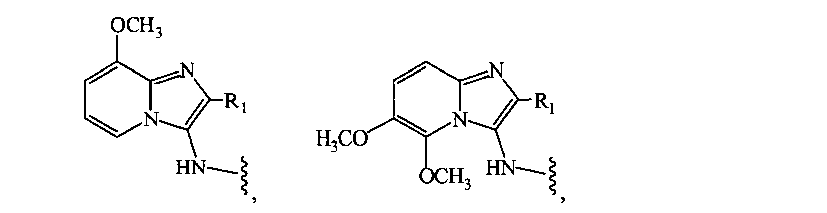 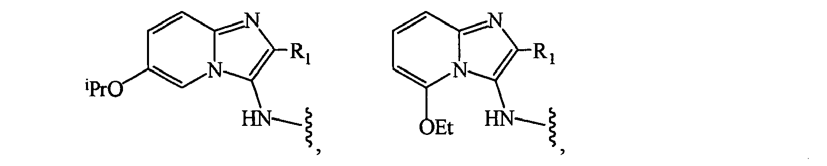 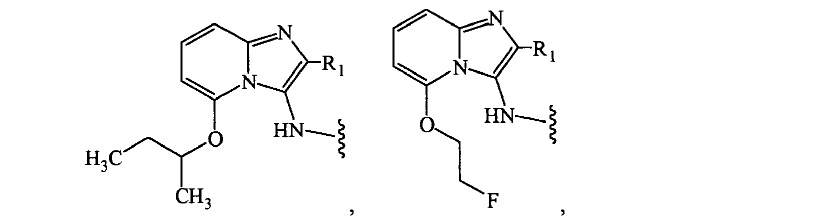 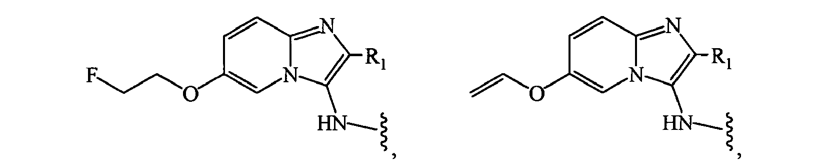 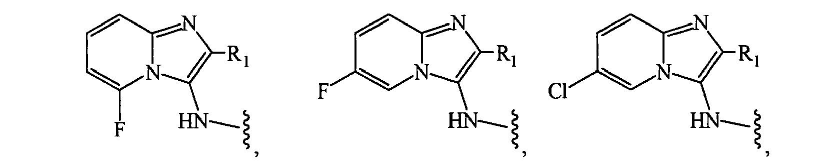 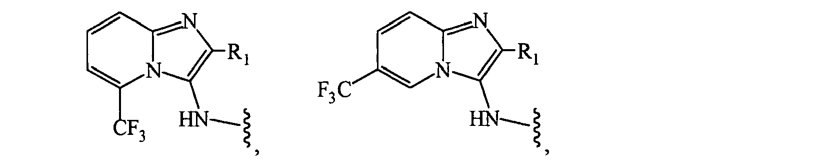 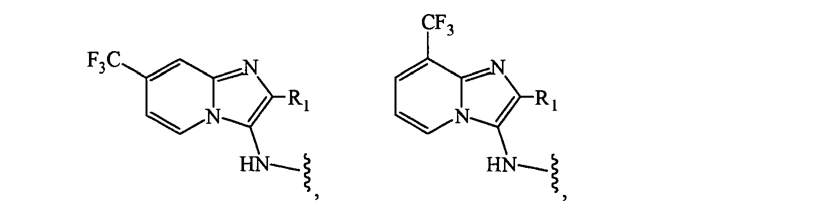 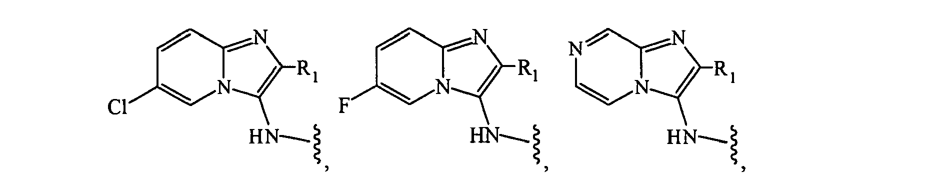 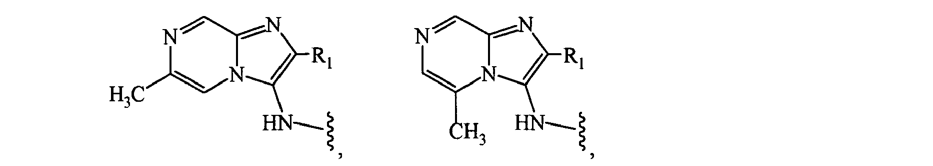 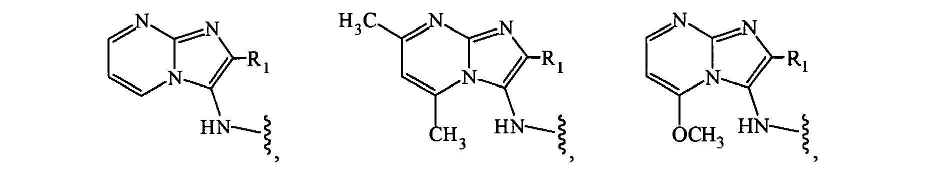 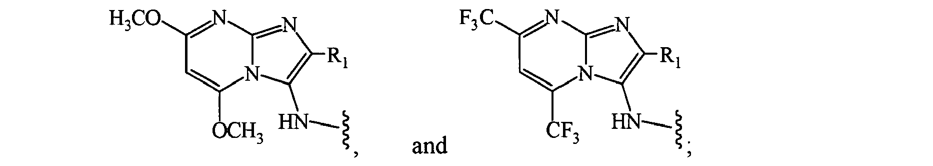
b) R₁ is selected from the group consisting of optionally substituted aryl, optionally substituted heteroaryl, optionally substituted carbocyclic ring, and optionally substituted heterocyclic ring;
c) n is 1 or 2; and,
d) at least one atom in the compound is a radioisotope;
and wherein, when a group of the compound of Formula I is described as optionally substituted, said group is unsubstituted, or substituted with one or more substituents independently selected from alkyl, alkenyl, alkynyl, cycloalkyl, cycloalkenyl, cycloalkynyl, aryl, heteroaryl, heteroalicyclyl, aralkyl, heteroaralkyl, (hetereoalicyclyl)alkyl, hydroxy, alkoxy, aryloxy, acyl, ester, mercapto, alkylthio, arylthio, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, and amino, including mono- and di-substituted amino groups.

Further, disclosed is a pharmaceutical composition comprising the compound of any one of claims 1-11, and a physiologically acceptable component such as a carrier, a diluent, a salt or an excipient, or a combination thereof.

Also disclosed is the compound of claim 5 for use in a method of PET imaging a first area of a tissue of a subject, the method comprising:
administering to the subject a pharmaceutical composition comprising a compound of Formula I as defined in claim 5;
measuring the signal emitted by the radioisotope from the first area of the tissue; and
comparing the amount of signal emitted from the first area of the tissue to an amount of signal emitted from a control sample.

### DETAILED DESCRIPTION OF THE INVENTION

CB2 receptor modulators (i.e., agonists, partial agonists, antagonists, or inverse agonists) have therapeutic utility for analgesia, acute and chronic pain, inflammatory pain, post-operative pain, neuropathic pain, muscle relaxation, immunosuppression, as anti-inflammatory agents, for allergies, glaucoma, bronchodilation, neuroprotection, osteoporosis and disorders of the skeletal system, cancer, neurodegenerative disorders including but not limited to Alzheimer's disease, Parkinson's disease (PD), and Huntington's disease, multiple sclerosis (MS), muscle spasticity, tremor, fibromyalgia, lupus, rheumatoid arthritis, myasthenia gravis, other autoimmune disorders, irritable bowel syndrome, interstitial cystitis, migraine, pruritis, excema, sebhorea, psoriasis, shingles, cerebral ischemia, cerebral apoplexy, craniocerebral trauma, stroke, spinal cord injury, liver cirrhosis, liver fibrosis, atherosclerosis, as an anti-tussive, asthma, nausea, emesis, gastric ulcers, and diarrhea.

In addition, compounds that bind with high potency and selectivity to CB2 receptors may be labeled with a radioactive element, or other detectable moiety, and be used as imaging agents to visualize and quantify CB2 receptors in many pathophysiological states. Such labeled CB2-selective compounds may be useful for early diagnosis of and progression of diseases such as Alzheimer's disease, Parkinson's disease, multiple sclerosis (MS), Huntington's disease, lupus, rheumatoid arthritis, myasthenia gravis, and fibromyalgia.

Thus, in one aspect, disclosed herein is a compound of Formula I: 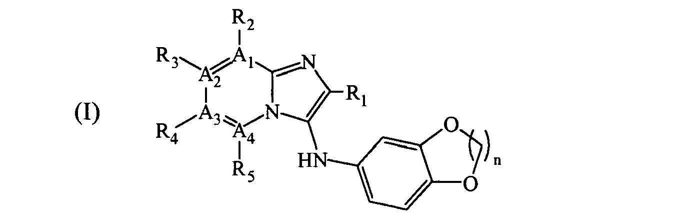 or a pharmaceutically acceptable salt thereof, wherein:
a) the 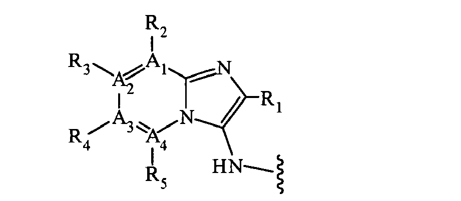 moiety is selected from the group consisting of 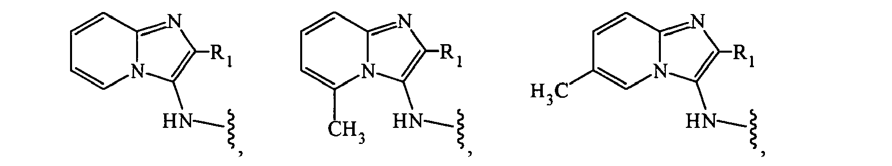 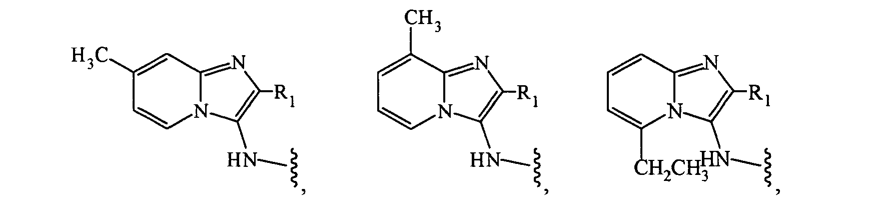 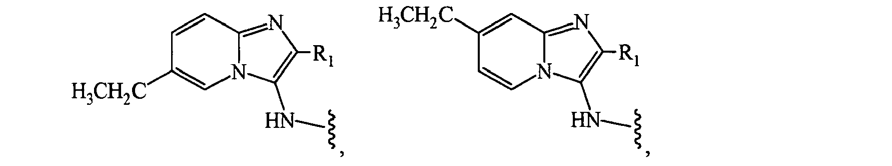 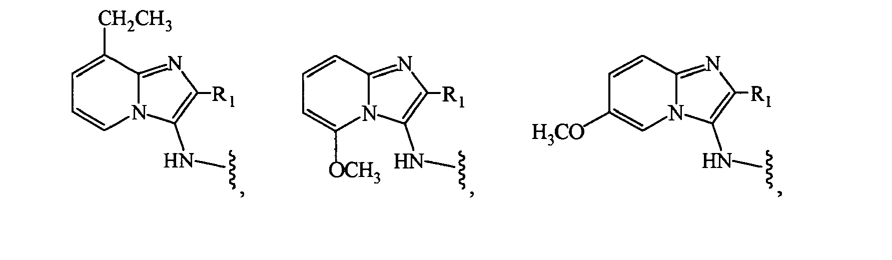 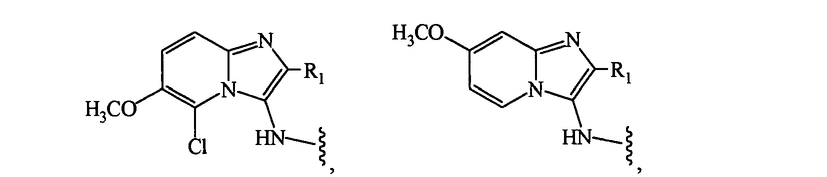 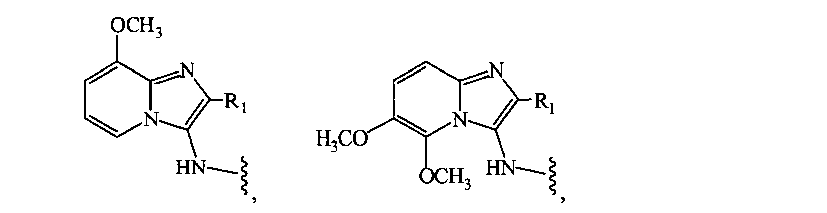 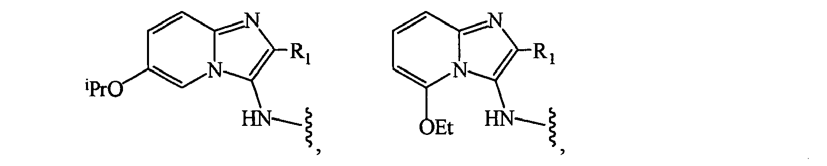 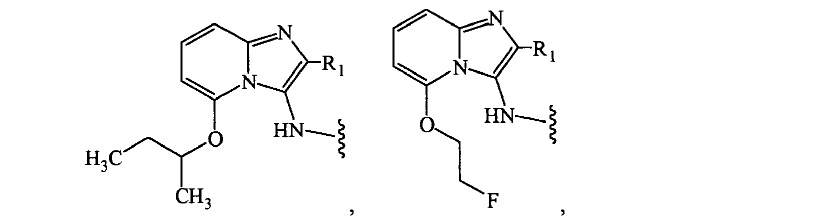 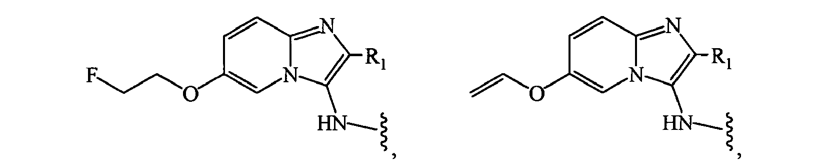 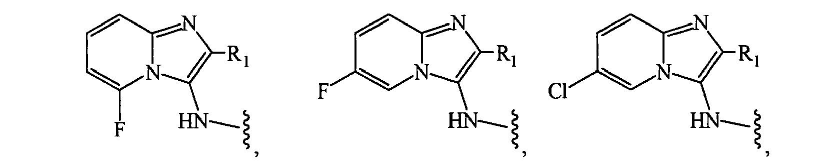 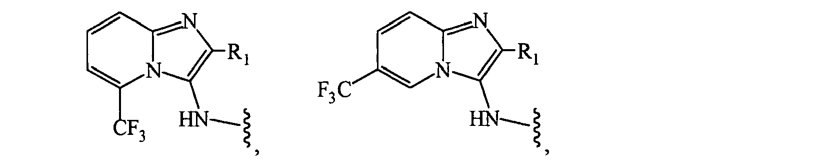 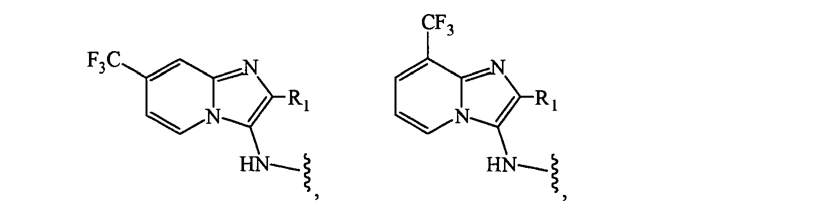 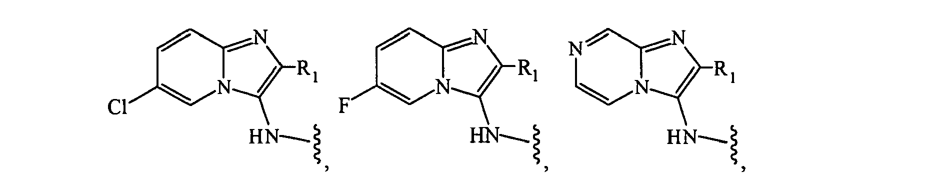 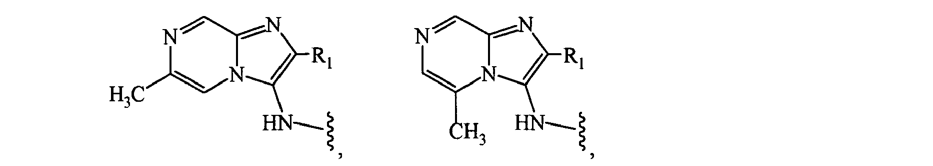 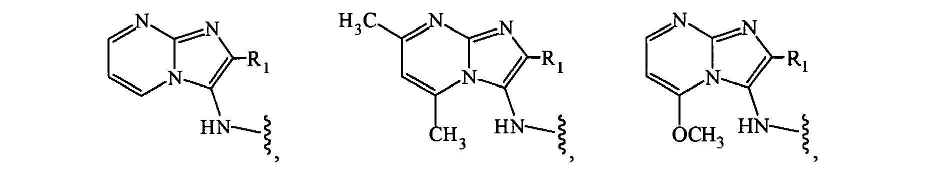 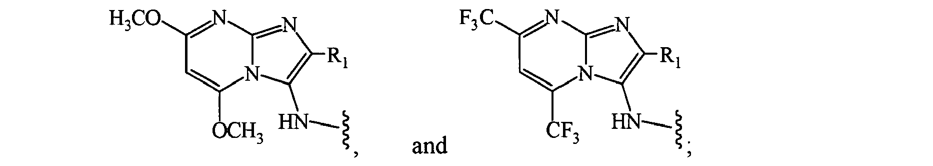
b) R₁ is selected from the group consisting of optionally substituted aryl, optionally substituted heteroaryl, optionally substituted carbocyclic ring, and optionally substituted heterocyclic ring;
c) n is 1 or 2; and,
d) at least one atom in the compound is a radioisotope;
and wherein, when a group of the compound of Formula I is described as optionally substituted, said group is unsubstituted, or substituted with one or more substituents independently selected from alkyl, alkenyl, alkynyl, cycloalkyl, cycloalkenyl, cycloalkynyl, aryl, heteroaryl, heteroalicyclyl, aralkyl, heteroaralkyl, (hetereoalicyclyl)alkyl, hydroxy, alkoxy, aryloxy, acyl, ester, mercapto, alkylthio, arylthio, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, and amino, including mono- and di-substituted amino groups.

The term "pharmaceutically acceptable salt" refers to a formulation of a compound that does not abrogate the biological activity and properties of the compound. Pharmaceutical salts can be obtained by reacting a compound of the invention with inorganic acids such as hydrochloric acid, hydrobromic acid, sulfuric acid, nitric acid, phosphoric acid, methanesulfonic acid, ethanesulfonic acid, p-toluenesulfonic acid, salicylic acid and the like. Pharmaceutical salts can also be obtained by reacting a compound of the invention with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, and sahs with amino acids such as arginine, lysine, and the like.

The term "ester" refers to a chemical moiety with formula -(R)ₙ-COOR^{'}, where R and R' are independently selected from the group consisting of alkyl, cycloalkyl, aryl, heteroaryl (bonded through a ring atom) and heteroalicyclic (bonded through a ring atom), and where n is 0 or 1.

An "amide" is a chemical moiety with formula -(R)ₙ-C(O)NHR' or -(R)ₙ-NHC(O)R', where R and R' are independently selected from the group consisting of alkyl, cycloalkyl aryl, heteroaryl (bonded through a ring atom) and heteroalicyclic (bonded through a ring atom), and where n is 0 or 1. An amide may be an amino acid or a peptide molecule attached to a molecule of the present invention, thereby forming a prodrug.

Any amine, hydroxy, or carboxyl side chain on the compounds of the present invention can be esterified or amidified. The procedures and specific groups used to achieve this end are known to those of skill in the art and can readily be found in reference sources such as Greene and Wuts, Protective Groups in Organic Synthesis, 3rd Ed., John Wiley & Sons, New York, N.Y., 1999, which is incorporated herein in its entirety.

Whenever a group of this invention is described as being "optionally substituted" that group may be unsubstituted or substituted with one or more of the substituents described for that group. Likewise, when a group is described as being "unsubstituted or substituted," if substituted, the substituent may be selected from the same group of substituents. Unless otherwise indicated, when a substituent is deemed to be "optionally subsituted," or "substituted" it is meant that the subsitutent is a group that may be substituted with one or more group(s) individually and independently selected from alkyl, alkenyl, alkynyl, cycloalkyl, cycloalkenyl, cycloalkynyl, aryl, heteroaryl, heteroalicyclyl, aralkyl, heteroaralkyl, (hetereoalicyclyl)alkyl, hydroxy, protected hydroxyl, alkoxy, aryloxy, acyl, ester, mercapto, alkylthio, arylthio, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, and amino, including mono- and di-substituted amino groups, and the protected derivatives thereof. The protecting groups that may form the protective derivatives of the above substituents are known to those of skill in the art and may be found in references Greene and Wuts, Protective Groups in Organic Synthesis, 3rd Ed., John Wiley & Sons, New York, NY, 1999, which is hereby incorporated by reference in its entirety.

As used herein, "Cₘ to Cₙ" or "Cₘ-Cₙ" in which "m" and "n" are integers refers to the number of carbon atoms in an alkyl, alkenyl, alkynyl and the rings of cycloalkyl and cycloalkenyl group. That is, the alkyl, alkenyl or alkynyl can contain from "m" to "n", inclusive, carbon atoms. If no "m" and "n" are designated with regard to an alkyl, alkenyl or alkynyl group herein, the broadest range described in these definitions is to be assumed. Thus "alkyl" alone means C₁-C₂₀ alkyl. A "C₁ to C₄ alkyl" group refers to all alkyl groups having from 1 to 4 carbons, that is, CH₃-, CH₃CH₂-, CH₃CH₂CH₂-, CH₃CH(CH₃)-, CH₃CH₂CH₂CH₂-, CH₃CH₂CH(CH₃)- and (CH₃)₃CH-, etc. With regard to cyclic compounds, "m" and "n" provide the number of possible carbon atoms in the ring.

As used herein, "alkyl" refers to a straight or branched chain fully saturated (no double or triple bonds) hydrocarbon (all carbon) group. An alkyl group of this invention may comprise from 1 - 20 carbon atoms, that is, "m" = 1 and "n" = 20, designated as a "C₁ to C₂₀ alkyl." In some embodiments, "m" = 1 and "n":= 12 (C₁ to C₁₂ alkyl). In other embodiments, that "m" = 1 and "n" = 6 (C₁ to C₆ alkyl). Examples of alkyl groups include, without limitation, methyl, ethyl, n-propyl, isopropyl, n-butyl, iso-butyl, sec-butyl, tert-butyl, amyl, tert-amyl, hexyl, heptyl, octyl, nonyl, decyl, undecyl and dodecyl.

An alkyl group of this invention may be substituted or unsubstituted. When substituted, the substituent group(s) is(are) one or more group(s) independently selected from cycloalkyl, aryl, heteroaryl, heteroalicyclyl, hydroxy, alkoxy, aryloxy, mercapto, alkylthio, arylthio, cyano, halo, oxo, carbonyl, thiocarbonyl, O-carbamyl, N-carbamyl, O-thiocarbamyl, N-thiocarbamyl, C-amido, N-amido, S-sulfonamido, N-sulfonamido, C-carboxy, O-carboxy, isocyanato, thiocyanato, isothiocyanato, nitro, silyl, trihalomethanesulfonyl, -NR^{a}R^{b}, protected hydroxyl, protected amino, protected carboxy and protected amido groups.

Examples of substituted alkyl groups include, without limitation, 2-oxo-prop-1-yl, 3-oxo-but-1-yl, cyanomethyl, nitromethyl, chloromethyl, hydroxymethyl, tetrahydropyranyloxymethyl, m-trityloxymethyl, propionyloxymethyl, aminomethyl, carboxymethyl, allyloxycarbonylmethyl, allyloxycarbonylaminomethyl, methoxymethyl, ethoxymethyl, t-butoxymethyl, acetoxymethyl, chloromethyl, bromomethyl, iodomethyl, trifluoromethyl, 6-hydroxyhexyl, 2,4-dichlorobutyl, 2-aminopropyl, 1-chloroethyl, 2-chloroethyl, 1-bromoethyl, 2-chloroethyl, 1-fluoroethyl, 2-fluoroethyl, 1-iodoethyl, 2-iodoethyl, 1-chloropropyl, 2-chloropropyl, 3-chloropropyl, 1-bromopropyl, 2-bromopropyl, 3-bromopropyl, 1-fluoropropyl, 2-fluoropropyl, 3-fluoropropyl, 1-iodopropyl, 2-iodopropyl, 3-iodopropyl, 2-aminoethyl, 1-aminoethyl, N-benzoyl-2-aminoethyl, N-acetyl-2-aminoethyl, N-benzoyl-1-aminoethyl and N-acetyl-1-aminoethyl.

As used herein, "alkenyl" refers to an alkyl group that contains in the straight or branched hydrocarbon chain one or more double bonds. Examples of alkenyl groups include, without limitation, vinyl (CH₂=CH-), allyl (CH₃CH=CH₂-), 1-propenyl, 2-propenyl, 1-butenyl, 2-butenyl; 1-pentenyl, 2-pentenyl, 3-pentenyl, 4-pentenyl, 3-methyl-1-butenyl, and the various isomers of hexenyl, heptenyl, octenyl, nonenyl, decenyl undecenyl and dodecenyl.

An alkenyl group of this invention may be unsubstituted or substituted. When substituted, the substituent(s) may be selected from the same groups disclosed above with regard to alkyl group substitution. Examples of substituted alkenyl groups include, without limitation, styrenyl, 3-chloro-propen-1-yl, 3-chloro-buten-1-yl, 3-methoxy-propen-2-yl, 3-phenyl-buten-2-yl and 1-cyano-buten-3-yl.

As used herein, "alkynyl" refers to an alkyl group that contains in the straight or branched hydrocarbon chain one or more triple bonds.

An alkynyl group of this invention may be unsubstituted or substituted. When substituted, the substituent(s) may be selected from the same groups disclosed above with regard to alkyl group substitution.

As used herein, "cycloalkyl" refers to a completely saturated (no double bonds) hydrocarbon ring. Cycloalkyl groups of this invention may range from C₃ to C₁₀, preferably at present from C₃ to C₇. Examples of cycloalkyl groups include, without limitation, cyclopropyl, cyclobutyl, cyclopentyl, cyclohexyl and cycloheptyl.

As used herein, "cycloalkenyl" refers to a cycloalkyl group that contains one or more double bonds in the ring although, if there is more than one, they cannot form a fully delocalized pi-electron system in the ring (otherwise the group would be "aryl," as defined herein). A cycloalkenyl of this invention may have from 5 to 10 carbon atoms in the ring, i.e., it may be C₅ to C₁₀, preferably at present C₅ to C₇. An cycloalkenyl group of this invention may unsubstituted or substituted. When substituted, the substituent(s) may be selected from the same groups disclosed above with regard to alkyl group substitution.

As used herein, "acyl" refers to an "RC(=O)O-" Examples of acyl groups include, without limitation, formyl, acetyl, propionyl, butyryl, pentanoyl, pivaloyl, hexanoyl, heptanoyl, octanoyl, nonanoyl, decanoyl, undecanoyl, dodecanoyl and benzoyl. Presently preferred acyl groups are acetyl and benzoyl.

An acyl group of this invention may be unsubstituted or substituted. When substituted, the substituent(s) may be selected from the same groups disclosed above with regard to alkyl group substitution. Example of substituted acyl groups include, without limitation, 4-phenylbutyroyl, 3-phenylbutyroyl, 3-phenylpropanoyl, 2-cyclohexanylacetyl, cyclohexanecarbonyl, 2-furanoyl and 3-dimethylaminobenzoyl.

The term "aromatic" refers to an aromatic group which has at least one ring having a conjugated pi electron system and includes both carbocyclic aryl (e.g., phenyl) and heterocyclic aryl groups (e.g., pyridine). The term includes monocyclic or fused-ring polycyclic (i.e., rings which share adjacent pairs of carbon atoms) groups. The term "carbocyclic" refers to a compound which contains one or more covalently closed ring structures, wherein the atoms forming the backbone of the ring are all carbon atoms. The term "heteroaromatic" or "heteroaryl" refers to an aromatic group, which contains at least one heterocyclic ring, which may be optionally substituted.

As used herein, "aryl" refers to a carbocyclic (all carbon) ring or two or more fused rings (rings that share two adjacent carbon atoms) that have a fully delocalized pi-electron system. Examples of aryl groups include, but are not limited to, benzene, and substituted benzene, such as toluene, aniline, xylene, and the like, naphthalene and substituted naphthalene, and azulene.

As used herein, "heteroaryl" refers to a ring or two or more fused rings that contain(s) one or more heteroatoms selected from the group consisting of nitrogen, oxygen and sulfur and that have a fully delocalized pi-electron system. Examples of heteroaryl groups include, but are not limited to, furan, thiophene, pyrrole, pyrroline, pyrrolidine, oxazole, thiazole, imidazole, imidazoline, imidazolidine, pyrazole, pyrazoline, pyrazolidine, isoxazole, isothiazole, triazole, thiadiazole, pyran, pyridine, piperidine, morpholine, thiomorpholine, pyridazine, pyrimidine, pyrazine, piperazine, triazine.

As used herein, "heteroalicyclic," "heteroalicyclyl," or "heterocyclic" refers to a ring or one or more fused rings having in the ring system one or more heteroatoms independently selected from nitrogen, oxygen and sulfur. The rings may also contain one or more double bonds provided that they do not create a fully delocalized pi-electron system in the rings. Heteroalicyclyl groups of this invention may be unsubstituted or substituted. When substituted, the substituent(s) may be one or more groups independently selected from the group consisting of, without limitation, halogen, hydroxy, protected hydroxy, cyano, nitro, alkyl, alkoxy, acyl, acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-alkylcarboxamide, protected N-alkylcarboxamide, N,N-dialkylcarboxamide, trifluoromethyl, N-alkylsulfonylamino and N-(phenylsulfonyl)amino. Presently preferred heteroalicyclyl groups include, without limitation, morpholino, piperidinyl, piperazinyl, 2-amino-imidazoyl, tetrahydrofurano, pyrrolo, tetrahydrothiophenyl, hexylmethyleneimino and heptylmethyleneimino.

As used herein, "arylalkyl" or "aralkyl," which are used synonymously and interchangeably, refer to an aryl group covalently bonded to an alkyl group, as defined herein. A "phenylalkyl" is a species of an aralkyl group, and refers to a phenyl ring covalently bonded to an alkyl group as defined herein. Examples, without limitation, of phenylalkyl groups include, without limitation, benzyl, 2-phenylethyl, 1-phenylpropyl, 4-phenylhexyl, 3-phenylamyl and 3-phenyl-2-methylpropyl. Presently preferred phenylalkyl groups are those wherein the phenyl group is covalently bonded to one of the presently preferred alkyl groups. A phenyl alkyl group of this invention may be unsubstituted or substituted. Examples of substituted phenylalkyl groups include, without limitation, 2-phenyl-1-chloroethyl, 2-(4-methoxyphenyl)ethyl, 4-(2,6-dihydroxy phenyl)hexyl, 2-(5-cyano-3-methoxyphenyl)pentyl, 3-(2,6-dimethylphenyl)propyl, 4-chloro-3-aminobenzyl, 6-(4-methoxyphenyl)-3-carboxy(n-hexyl), 5-(4-aminomethylphenyl)-3-(aminomethyl)pentyl and 5-phenyl-3-oxo-pent-1-yl.

As used herein, "heteroarylalkyl" or "heteroaralkyl," which are used synonymously and interchangeably, and "heteroalicyclylalkyl" refer to a heteroaryl or a heteroalicyclyl group, respectively, covalently bonded to an alkyl group, as defined herein. Examples of such groups include, without limitation, 2-pyridylethyl, 3-pyridylpropyl, 4-furylhexyl, 3-piperazylamyl and 3-morpholinylbutyl. Presently preferred heteroarylalkyl and heteroalicyclylalkyl groups are those in which a presently preferred heteroaryl or heteroalicyclyl group is covalently bonded to a presently preferred alkyl group as disclosed herein.

As used herein, "phenyl" refers to a 6-member aryl group. A phenyl group may be unsubstituted or substituted. When substituted the substituent(s) is/are one or more, preferably one or two, group(s) independently selected from the group consisting of halogen, hydroxy, protected hydroxy, cyano, nitro, alkyl, alkoxy, acyl, acyloxy, carboxy, protected carboxy, carboxymethyl, protected carboxymethyl, hydroxymethyl, protected hydroxymethyl, -NR^{a}R^{b} wherein R^{a} and R^{b} are as defined above but in addition R^{a} may be an amino protecting group as defined herein, carboxamide, protected carboxamide, N-alkylcarboxamide, protected N-alkylcarboxamide, N,N-dialkylcarboxamide, trifluoromethyl, N-alkylsulfonylamino, N-(phenylsulfonyl)amino and phenyl (resulting in the formation of a biphenyl group).

Examples of substituted phenyl groups include, without limitation, 2, 3 or 4-chlorophenyl, 2,6-dichlorophenyl, 2,5-dichlorophenyl, 3,4-dichlorophenyl, 2, 3 or 4-bromophenyl, 3,4-dibromophenyl, 3-chloro-4-fluorophenyl, 2, 3 and 4-fluorophenyl, 2, 3 or 4-hydroxyphenyl, 2,4-dihydroxyphenyl, the protected-hydroxy derivatives thereof, 2, 3 or 4-nitrophenyl; 2, 3 or 4-cyanophenyl; 2, 3 or 4-methylphenyl, 2,4-dimethylphenyl, 2, 3 or 4-(iso-propyl)phenyl, 2, 3 or 4-ethylphenyl, 2, 3 or 4-(n-propyl)phenyl, 2,6-dimethoxyphenyl, 2, 3 or 4-methoxyphenyl, 2, 3 or 4-ethoxyphenyl, 2, 3 or 4-(isopropoxy)phenyl, 2, 3 or 4-(t-butoxy)phenyl, 3-ethoxy-4-methoxyphenyl; 2, 3 or 4-trifluoromethylphenyl; 2, 3 or 4-carboxyphenyl or 2,4-di(protected carboxy)phenyl; 2, 3, or 4-(protected hydroxymethyl)phenyl or 3,4-di(hydroxymethyl)phenyl; 2, 3 or 4-(aminomethyl)phenyl or 2,4-(protected aminomethyl)phenyl; and 2, 3 or 4-(N-(methylsulfonylamino))phenyl.

As used herein, "phenylalkoxy" refers to a "phenylalkyl-O-" group with "phenyl" and "alkyl" as defined herein. A phenylalkoxy group of this invention may be substituted or unsubstituted on the phenyl ring, in the alkyl group or both. Examples of phenylalkoxy groups include, without limitation, 2-(4-hydroxyphenyl)ethoxy, 4-(4-methoxyphenyl)butoxy, (2R)-3-phenyl-2-amino-propoxy, (2S)-3-phenyl-2-amino-propoxy, 2-indanoxy, 6-phenyl-1-hexanoxy, cinnamyloxy, 2-phenyl-1-propoxy and 2,2-dimethyl-3-phenyl-1-propoxy.

As used herein, "halo" and "halogen" refer to the fluoro, chloro, bromo or iodo atoms. Presently preferred halogens are chloro and fluoro.

As used herein, "amino protecting group" refers to a group commonly employed to keep (i.e., to "block" or "protect") an amino group from reacting with a reagent while it reacts with an intended target functional group of a molecule.

As used herein, a "protected carboxamide" refers to a carboxamide in which the nitrogen is substituted with an amino protecting group.

Examples of amino protecting groups include, without limitation, formyl ("For"), trityl, phthalimido, trichloroacetyl, chloroacetyl, bromoacetyl, iodoacetyl groups, t-butoxycarbonyl ("Boc"), 2-(4-biphenylyl)propyl-2-oxycarbonyl ("Bpoc"), 2-phenylpropyl-2-oxycarbonyl ("Poc"), 2-(4-xenyl)isopropoxycarbonyl, 1,1-diphenylethyl-1-oxycarbonyl, 1,1-diphenylpropyl-1-oxycarbonyl, 2-(3,5-dimethoxyphenyl)propyl-2-oxycarbonyl ("Ddz"), 2-(p-toluyl)propyl-2-oxycarbonyl, cyclopentanyloxycarbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfonyl)-ethoxycarbonyl, 2-(methylsulfonyl)ethoxycarbonyl, 2-(triphenylphosphino)-ethoxycarbonyl, 9-fluorenylmethoxycarbonyl ("Fmoc"), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyl-oxycarbonyl, 2,2,2-trichloroethoxycarbonyl, 2-ethynyl-2-propoxycarbonyl, cyclopropylmethoxycarbonyl, isobornyloxycarbonyl, 1-piperidyloxycarbonyl, benzyloxycarbonyl ("Cbz"), 4-phenylbenzyloxycarbonyl, 2-methylbenzyloxy-carbonyl, -2,4,5,-tetramethylbenzyloxycarbonyl ("Tmz"), 4-methoxybenzyloxy- carbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyl-oxycarbonyl, 4-bromobenzyloxycarbonyl, 3-bromobenzyloxycarbonyl, 4-nitrobenzyloxy-carbonyl, 4-cyanobenzyloxycarbonyl, 4-(decyloxy) benzyloxycarbonyl, benzoylmethylsulfonyl, dithiasuccinoyl ("Dts"),2-(nitro)phenylsulfenyl ("Nps"), and diphenyl-phosphine oxide. The species of amino-protecting group employed is not critical so long as the derivatized amino group is stable to the conditions of the subsequent reaction(s) and can be removed at the appropriate point without disrupting the remainder of the molecule. Presently preferred amino-protecting groups are Boc, Cbz and Fmoc. Descriptions of these and other amino-protecting groups may be found in T. W. Greene and P. G. M. Wuts, "Protective Groups in Organic Synthesis," 2nd ed., John Wiley and Sons, New York, N.Y., 1991, Chapter 7, M. Bodanzsky, "Principles of Peptide Synthesis," 1st and 2nd revised ed., Springer-Verlag, New York, N.Y., 1984 and 1993, and Stewart and Young, "Solid Phase Peptide Synthesis," 2nd ed., Pierce Chemical Co., Rockford, Ill., 1984.

As used herein, the term "carboxy protecting group" refers to a labile ester commonly used to block or protect a carboxylic acid while reactions are carried out on other functional groups on the compound. Examples of carboxy protecting groups include, without limitation, t-butyl, 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxytrityl, 4,4',4"-trimethoxytrityl, 2-phenylpropyl, trimethylsilyl, t-butyldimethylsilyl, phenacyl, 2,2,2-trichloroethyl, -(trimethylsilyl)ethyl, -(di(n-butyl)methylsilyl)ethyl, p-toluenesulfonylethyl, 4-nitrobenzylsulfonylethyl, allyl, cinnamyl, and 1-(trimethylsilylmethyl)-propenyl. The ester employed is not critical so long as it is stable to the conditions of subsequent reaction(s) and can be removed at the appropriate point without disrupting the remainder of the molecule. Further examples of carboxy-protecting groups are found in E. Haslam, "Protective Groups in Organic Chemistry," J. G. W. McOmie, Ed., Plenum Press, New York, N.Y., 1973, Chapter 5, and T. W. Greene and P. G. M. Wuts, "Protective Groups in Organic Synthesis," 2nd ed., John Wiley and Sons, New York, N.Y., 1991, Chapter 5.

As used herein, a "hydroxyl protecting group" refers to a readily cleavable group that replaces the hydrogen of the hydroxyl group, such as, without limitation, tetrahydropyranyl, 2-methoxypropyl, 1-ethoxyethyl, methoxymethyl, 2-methoxyethoxymethyl, methylthiomethyl, t-butyl, t-amyl, trityl, 4-methoxytrityl, 4,4'-dimethoxytrityl, 4,4',4"-trimethoxytrityl, benzyl, allyl, trimethylsilyl, (t-butyl)dimethylsilyl, and 2,2,2-trichloroethoxycarbonyl. The species of hydroxyl protecting groups is not critical so long as the derivatized hydroxyl group is stable to the conditions of subsequent reaction(s) and can be removed at the appropriate point without disrupting the remainder of the molecule. Further examples of hydroxy-protecting groups are described by C. B. Reese and E. Haslam, "Protective Groups in Organic Chemistry," J. G. W. McOmie, Ed., Plenum Press, New York, N.Y., 1973, Chapters 3 and 4, respectively, and T. W. Greene and P. G. M. Wuts, "Protective Groups in Organic Synthesis," 2nd ed., John Wiley and Sons, New York, N.Y., 1991, Chapters 2 and 3.

As used herein, "alkylthio" refers to an "alkyl-S-" group, with alkyl as defined above. Examples of alkylthio group include, without limitation, methylthio, ethylthio, n-propylthio, isopropylthio, n-butylthio and t-butylthio.

As used herein, "alkylsulfinyl" refers to an "alkyl-SO-" group, with alkyl as defined above. Examples of alkylsulfinyl groups include, without limitation, methylsulfinyl, ethylsulfinyl, n-propylsulfinyl, isopropylsulfinyl, n-butylsulfinyl and sec-butylsulfinyl.

As used herein, "alkylsulfonyl" refers to an "alkyl-SO₂-" group. Examples of alkylsulfonyl groups include, without limitation, methylsulfonyl, ethylsulfonyl, n-propylsulfonyl, isopropylsulfonyl, n-butylsulfonyl, and t-butylsulfonyl.

As used herein, "phenylthio," "phenylsulfinyl," and "phenylsulfonyl" refer to a "phenyl-S-," "phenyl-SO-," and "phenyl-SO₂-" group, phenyl as defined herein.

As used herein, "alkylaminocarbonyl" refers to an "alkylNHC(=O)-" group, with alkyl as defined herein. Examples of alkylaminocarbonyl groups include, without limitation, methylaminocarbonyl, ethylaminocarbonyl, propylaminocarbonyl and butylaminocarbonyl. Examples of substituted alkylaminocarbonyl include,without limitation, methoxymethyl-aminocarbonyl, 2-chloroethylaminocarbonyl, 2-oxopropylaminocarbonyl and 4-phenylbutylaminocarbonyl.

As used herein, "alkoxycarbonyl" refers to an "alkyl-OC(=O)-" group, with alkyl as defined above.

As used herein, "phenylaminocarbonyl" refers to a "phenyl-NHC(=O)-" group, with phenyl as defined above. Examples of substituted phenylaminocarbonyl groups include, without limitation, 2-chlorophenyl-aminocarbonyl, 3-chlorophenylaminocarbonyl, 2-nitorphenylaminocarbonyl, 4-biphenylaminocarbonyl, and 4-methoxyphenylaminocarbonyl.

As used herein, "alkylaminothiocarbonyl" refers to an "alkyl-NHC(=O)-" group, with alkyl as defined above. Examples of alkylaminothiocarbonyl groups include, without limitation, methylaminothiocarbonyl, ethylaminothiocarbonyl, propylaminothiocarbonyl and butylaminothiocarbonyl.

Examples of alkyl-substituted alkylaminothiocarbonyl groups include, without limitation, methoxymethylaminothiocarbonyl, 2-chloroethylaminothiocarbonyl, 2-oxopropylaminothiocarbonyl and 4-phenylbutylaminothiocarbonyl.

As used herein, "phenylaminothiocarbonyl" refers to a "phenyl-NHC(=S)-" group, with phenyl as defined above. Examples of phenylaminothiocarbonyl groups include, without limitation, 2-chlorophenylaminothiocarbonyl, 3-chlorophenyl-aminothiocarbonyl, 2-nitrophenylaminothiocarbonyl, 4-biphenylaminothiocarbonyl and 4-methoxyphenylaminothiocarbonyl.

As used herein, "carbamoyl" refers to an "-NCO-" group.

As used herein, "hydroxyl" refers to an "-OH" group.

As used herein, "cyano" refers to a "-C=N" group.

As used herein, "nitro" refers to an "-NO₂" group.

An "O-carboxy" group refers to a "RC(=O)O-" group with R as defined above.

A "C-carboxy" group refers to a "-C(=O)OR" group with R as defined above.

An "acetyl" group refers to a CH₃C(=O)- group.

A "trihalomethanesulfonyl" group refers to an "X₃CSO₂-" group wherein X is a halogen.

An "isocyanato" group refers to an "-NCO" group.

A "thiocyanato" group refers to a "-CNS" group.

An "isothiocyanato" group refers to an "-NCS" group.

A "sulfinyl" group refers to an "-S(=O)-R" group with R as defined above.

An "S-sulfonamido" group refers to a "-SO₂NR" group with R as defined above.

An "N-sulfonamido" group refers to a "RSO₂NH-" group with R as defined above.

A "trihalomethanesulfonamido" group refers to an "X₃CSO₂NR-" group with X as halogen and R as defined above.

An "O-carbamyl" group refers to a "-OC(=O)-NR" group with R as defined above.

An "N-carbamyl" group refers to an "ROC(=O)NH-" group with R as defined above.

An "O-thiocarbamyl" group refers to a "-OC(=S)-NR" group with R as defined above.

"N-thiocarbamyl" group refers to an "ROC(=S)NH-" group with R as defined above.

A "C-amido" group refers to a "-C(=O)-NR^{a}R^{b} group with R^{a} and R^{b} as defined above.

An "N-amido" group refers to a RC(=O)NH- group with R as defined above.

The term "haloalkyl" refers to an alkyl group where one or more of the hydrogen atoms are replaced by halogen. Such groups include but are not limited to , chloromethyl, fluoromethyl, difluoromethyl, trifluoromethyl and 1-chloro-2-fluoromethyl, 2-fluoroisobutyl.

The term "perhaloalkyl" refers to an alkyl group in which all the hydrogen atoms are replaced by halogen atoms.

As used herein, an "ester" refers to a "-C(O)OR^{a}" group with R^{a} as defined herein.

As used herein, an "amide" refers to a "-C(O)NR^{a}R^{b}" group with R^{a} and R^{b} as defined herein.

Where the numbers of substituents are not specified (e.g. haloalkyl) there may be one or more substituents presents. For example "haloalkyl" may include one or more of the same or differents halogens. As another example "C₁-C₃ alkoxy phenyl" may include one or more of the same of different alkoxygroups containing one, two or three atoms.

Any unsubstituted or monosubstituted amine group on a compound herein can be converted to an amide, any hydroxyl group can be converted to an ester and any carboxyl group can be converted to either an amide or ester using techniques well-known to those skilled in the art (see, for example, Greene and Wuts, Protective Groups in Organic Synthesis, 3rd Ed., John Wiley & Sons, New York, NY, 1999). Compounds containing any such converted hydroxyl, amino and/or carboxylic acid groups are within the scope of this invention.

As used herein, an "ether" refers to a "-C-O-C-" group wherein either or both carbons may independently be part of an alkyl, alkenyl, alkynyl, aryl, heteroaryl or heteroalicyclyl group.

As used herein, a "halogenated ether" refers to an ether in which the groups to either side of the oxygen are both alkyl substituted with halogen.

As used herein, "amino acid" refers to any one of the twenty naturally-occurring L-amino acids, to their non-natural D-enantiomers, to non-naturally occurring amino acids such as, without limitation, norleucine ("Nle"), norvaline ("Nva"), L- or D-naphthalanine, ornithine ("Orn"), homoarginine (homoArg) and to other amino acids well-known in the peptide art such as those described in M. Bodanzsky, "Principles of Peptide Synthesis," 1st and 2nd revised ed., Springer-Verlag, New York, N.Y., 1984 and 1993, and Stewart and Young, "Solid Phase Peptide Synthesis," 2nd ed., Pierce Chemical Co., Rockford, Ill.

When two substituents taken together along with the carbon atoms to which they are attached form a five- or six-membered optionally substituted carbocyclic ring or optionally substituted heterocyclic ring, or form a six-membered optionally substituted aryl, optionally substituted heteroaryl, it is meant that the following structure: 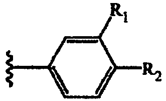 can be representative of, for example, the following structures: 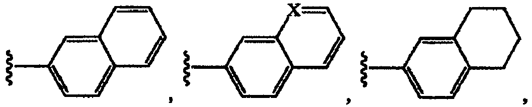 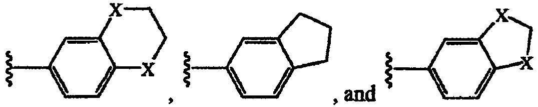 where X is a heteroatom.

Throughout the present disclosure, when a particular compound comprises a chiral center, the scope of the present disclosure also includes compositions comprising the racemic mixture of the two enantiomers, as well as compositions comprising each enantiomer individually substantially free of the other enantiomer. Thus, for example, contemplated herein is a composition comprising the S enantiomer substantially free of the R enantiomer, or a composition comprising the R enantiomer substantially free of the S enantiomer. By "substantially free" it is meant that the composition comprises less than 10%, or less than 8%, or less than 5%, or less than 3%, or less than 1% of the minor enantiomer. If the particular compound comprises more than one chiral center, the scope of the present disclosure also includes compositions comprising a mixture of the various diastereomers, as well as compositions comprising each diastereomer substantially free of the other diastereomers. The recitation of a compound, without reference to any of its particular diastereomers, includes compositions comprising all four diastereomers, compositions comprising the racemic mixture of R,R and S,S isomers, compositions comprising the racemic mixture of R,S and S,R isomers, compositions comprising the R,R enantiomer substantially free of the other diastereomers, compositions comprising the S,S enantiomer substantially free of the other diastereomers, compositions comprising the R,S enantiomer substantially free of the other diastereomers, and compositions comprising the S,R enantiomer substantially free of the other diastereomers.

The compound of Formula I is labelled with a radioisotope that may be detected using in vitro or in vivo techniques, which are discussed in more detail below. In some embodiments, at least one atom in the compound of Formula I is a radioisotope. The radioisotope may be an isotope of hydrogen, carbon, oxygen, nitrogen, or halogen. Those of skill in the art recognize an isotope of hydrogen, i.e., tritium (³H), certain isotopes of carbon, e.g., ¹¹C, certain isotopes of iodine, e.g., ¹²³I, certain isotopes of fluorine, e.g., ¹⁸F, certain isotopes of nitrogen, e.g.,¹³N, and certain isotopes of oxygen, e.g., ¹⁵O, are radioactive and once incorporated into a compound, their presence can be detected using known methods in the art, for example in vivo imaging techniques such as positron emission tomography (PET) or single photon emission computed tomography (SPECT).

The isotopically-labeled compounds of Formula I have one or more atoms replaced by an atom having an atomic mass or mass number different from the atomic mass or mass number of the most abundant isotope found in nature. Examples of isotopes that can be incorporated into compounds of Formula I include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively.

Certain isotopically-labeled compounds of the present invention (e.g., those labeled with ³H and ¹⁴C) are useful in compound and/or substrate tissue distribution assays. Tritiated (i.e., ³H) and carbon-14 (i.e., ¹⁴C) isotopes are particularly preferred for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements) and hence may be preferred in some circumstances. Positron emitting isotopes such as ¹⁵O, ¹³N, ¹¹C, and ¹⁸F are useful for positron emission tomography (PET) studies to examine substrate receptor occupancy. Isotopically labeled compounds of the present invention can generally be prepared by following procedures analogous to those disclosed in the Schemes and/or in the Examples herein below, by substituting an isotopically labeled reagent for a non-isotopically labeled reagent.

In some embodiments of the compound of Formula I, R₁ is an optionally substituted heteroaryL The heteroaryl may be selected from the group consisting of furan, thiophene, phthalazinone, pyrrole, oxazole, thiazole, imidazole, pyrazole, isoxazole, isothiazole, triazole, thiadiazole, pyran, pyridine, pyridazine, pyrimidine, pyrazine and triazine.. In some embodiments, the heteroaryl is pyridyl or thiophenyl.

In some embodiments of the compound of Formula I, R₁ is an optionally substituted aryl, which can be phenyl. In some of these embodiments, R₁ is 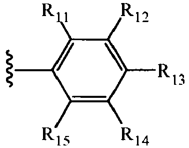 where
R₁₁, R₁₂, R₁₃, R₁₄, and R₁₅ is each independently selected from the group consisting of hydrogen, alkyl, halo, perhaloalkyl, hydroxy, alkoxy, and mercaptoalkyl, or R₁₁ and R₁₂ taken together along with the carbon atoms to which they are attached, or R₁₂ and R₁₃ taken together along with the carbon atoms to which they are attached, or R₁₃ and R₁₄ taken together along with the carbon atoms to which they are attached, or R₁₄ and R₁₅ taken together along with the carbon atoms to which they are attached form a five- or six-membered optionally substituted carbocyclic ring or optionally substituted heterocyclic ring, or form a six-membered optionally substituted aryl, optionally substituted heteroaryl.

In some embodiments, the alkyl is selected from the group consisting of methyl, ethyl, n-propyl, isopropyl, n-butyl, sec-butyl, tert-butyl, and methylencyclopropyl.. In further embodiments, the alkoxy is selected from the group consisting of methoxy, ethoxy, n-propoxy, isopropoxy, n-butoxy, sec-butoxy, and tert-butoxy. In additional embodiments, the halo is selected from the group consisting of fluoro, chloro, bromo, and iodo.

In some embodiments, R₁ is 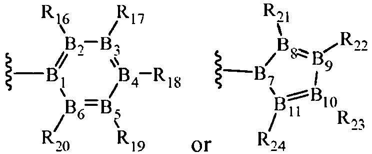
a) B₁, B₂, B₃, B₄, B₅, and B₆ is each independently selected from the group consisting of carbon, sulfur, oxygen, and nitrogen;
b) B₇, B₈, B₉, B₁₀, and B₁₁ is each independently selected from the group consisting of carbon, sulfur, oxygen, and nitrogen;
c) R₁₆, R₁₇, R₁₈, R₁₉, and R₂₀ is each independently selected from the group consisting of hydrogen, alkyl, alkenyl, alkynyl, cycloalkyl, cycloalkenyl, optionally substituted₋ aryl, optionally substituted heteroaryl, optionally substituted heteroalicyclyl, halogen, sulfenyl, sulfinyl, sulfonyl, haloalkyl, haloalkoxy, perhaloalkyl, CN, C(=Z)R', C(=Z)OR', C(=Z)NR'R", -C(R')=NR', -NR'R", -N=CR'R", N(R')C(=Z)R', N(R')C(=Z)NR'R", -S(O)NR'R", -S(O)₂NR'R", N(R')S(=O)R', N(R')S(=O)₂R', -OR', -SR', and OC(=Z)R',
   wherein R' and R" are each independently selected from the group consisting of hydrogen, alkyl, cycloalkyl, optionally substituted aryl, optionally substituted heteroaryl, and optionally substituted heteroalicyclyl, and Z is oxygen or sulfur,
   or R₁₆ and R₁₇ taken together along with the carbon atoms to which they are
   attached, or R₁₇ and R₁₈ taken together along with the carbon atoms to which they are attached, or R₁₈ and R₁₉ taken together along with the carbon atoms to which they are attached, or R₁₉ and R₂₀ taken together along with the carbon atoms to which they are attached form a five- or six-membered optionally substituted carbocyclic ring or optionally substituted heterocyclic ring, or form a six-membered optionally substituted aryl, optionally substituted heteroaryl;
   provided that,
   R₁₆ does not exist when B₂ is not carbon,
   R₁₇ does not exist when B₃ is not carbon,
   R₁₈ does not exist when B₄ is not carbon,
   R₁₉ does not exist when B₅ is not carbon, and
   R₂₀ does not exist when B₆ is not carbon; and
d) R₂₁, R₂₂, R₂₃, and R₂₄ is each independently selected from the group consisting of hydrogen, alkyl, alkenyl, alkynyl, cycloalkyl, cycloalkenyl, optionally substituted aryl, optionally substituted heteroaryl, optionally substituted heteroalicyclyl, halogen, sulfenyl, sulfinyl, sulfonyl, haloalkyl, haloalkoxy, perhaloalkyl, CN, C(=Z)R', C(=Z)OR', C(=Z)NR'R", -C(R')=NR', -NR'R", -N=CR'R", N(R')C(=Z)R', N(R')C(=Z)NR'R", -S(O)NR'R", -S(O)₂NR'R", N(R')S(=O)R', N(R')S(=O)₂R', -OR', -SR', and OC(=Z)R',
   wherein R' and R" are each independently selected from the group consisting of hydrogen, alkyl, cycloalkyl, optionally substituted aryl, optionally substituted heteroaryl, and optionally substituted heteroalicyclyl, and Z is oxygen or sulfur,
   or R₂₁ and R₂₂ taken together along with the carbon atoms to which they are attached, or R₂₂ and R₂₃ taken together along with the carbon atoms to which they are attached, or R₂₃ and R₂₄ taken together along with the carbon atoms to which they are attached form a five- or six-membered optionally substituted carbocyclic ring or optionally substituted heterocyclic ring, or form a six-membered optionally substituted aryl, optionally substituted heteroaryl;
   provided that,
   R₂₁ does not exist when B₈ is not carbon,
   R₂₂ does not exist when B₉ is not carbon,
   R₂₃ does not exist when B₁₀ is not carbon, and
   R₂₄ does not exist when B₁₁ is not carbon.

In some embodiments, at least three of B₁, B₂, B_{3,} B₄, B₅, and B₆ are carbon. In other embodiments, at least two of B₁, B₂, B₃, B₄, B₅, and B₆ are carbon. In further embodiments, at least one of B₁, B₂, B₃, B₄, B₅, and B₆ is carbon. In some embodiments, at least three of B₇, B₈, B₉, B₁₀, and B₁₁ are carbon. In other embodiments, at least two of B₇, B₈, B₉, B₁₀, and B₁₁ are carbon. In additional embodiments, at least one of B₇, B₈, B₉, B₁₀, and B₁₁ is carbon.

In some of the above embodiments, R₁ is selected from the group consisting of: 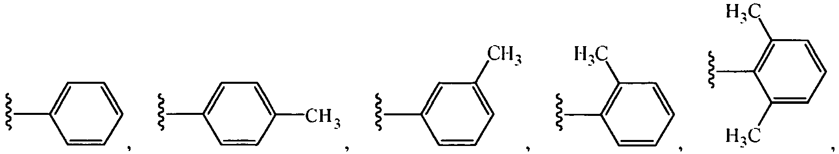 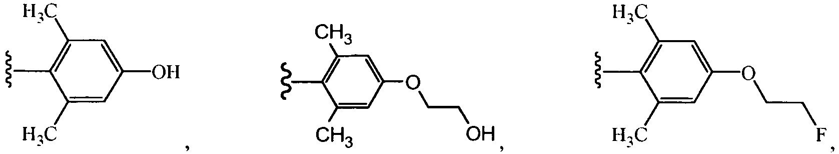 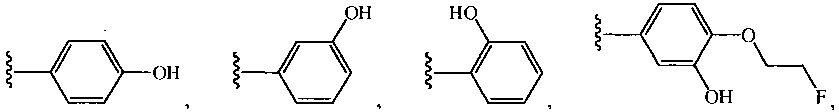 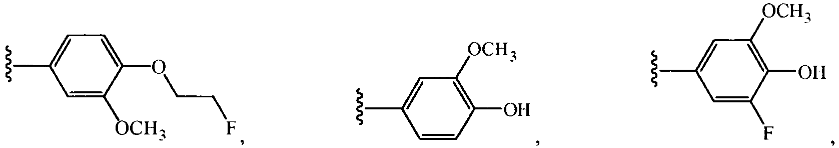 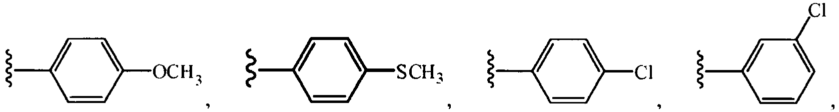 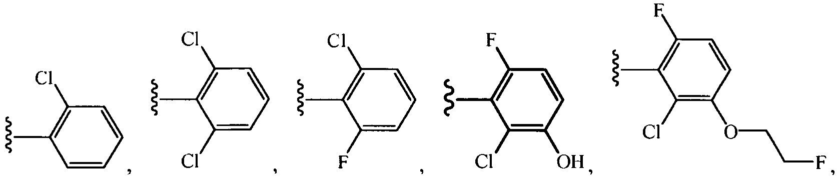 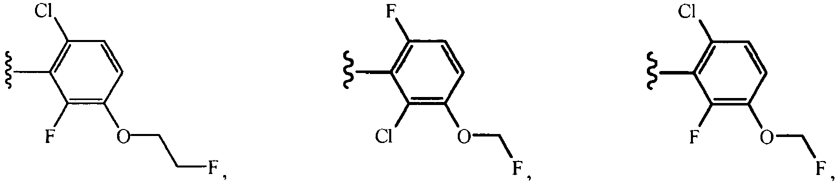 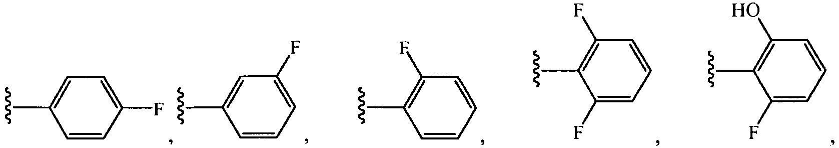 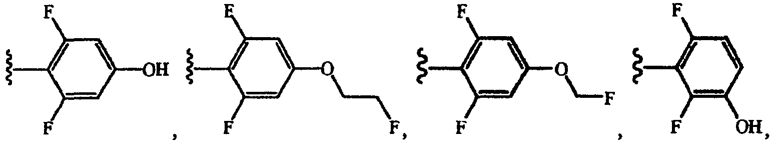 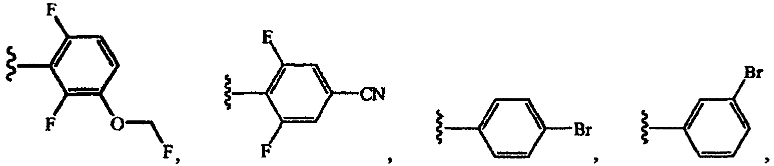 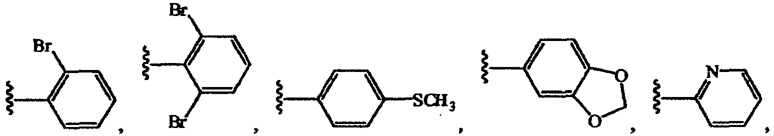 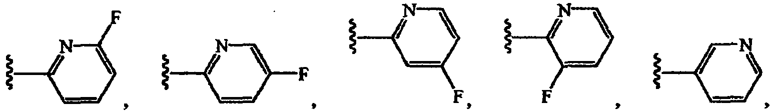 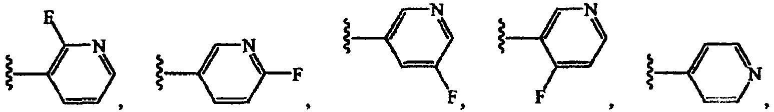 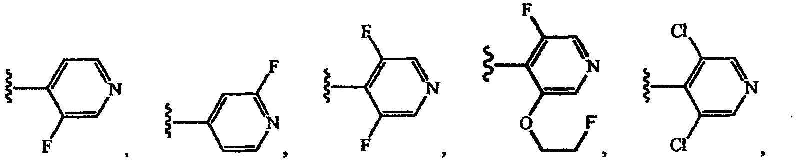 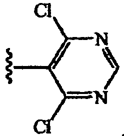 and

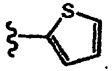

In the compound of Formula I, the 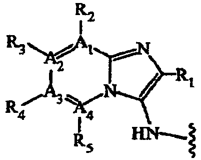 moiety is selected from the group consisting of 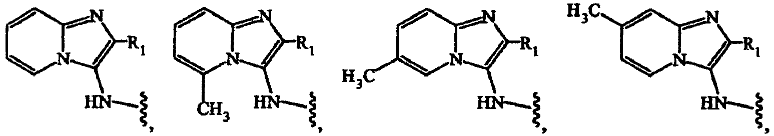 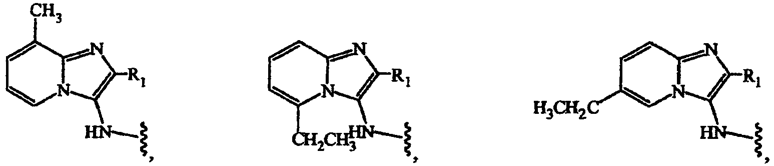 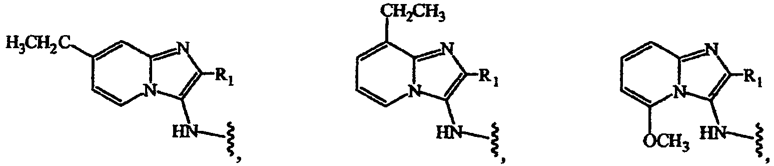 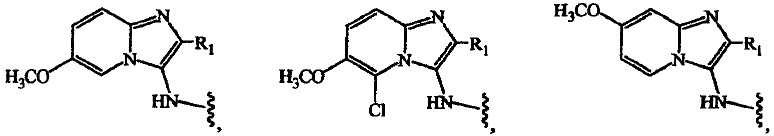 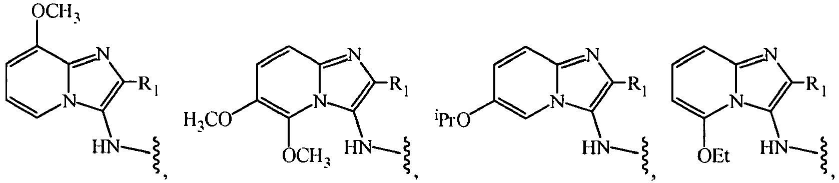 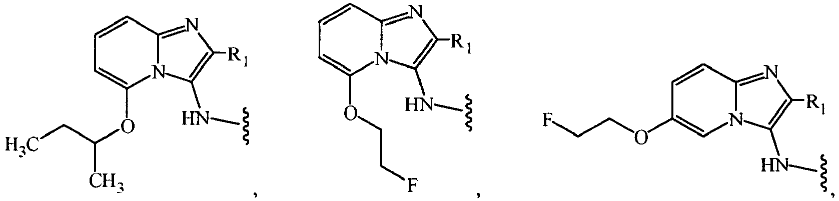 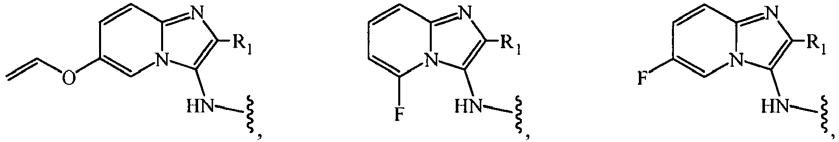 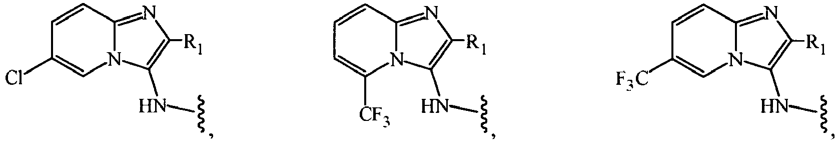 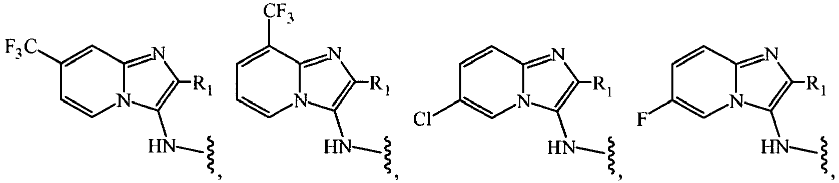 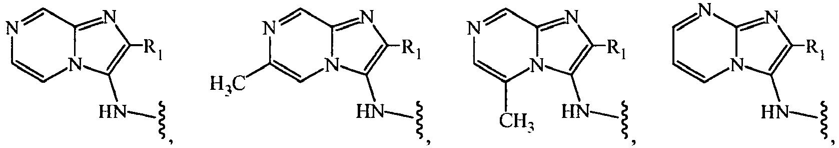 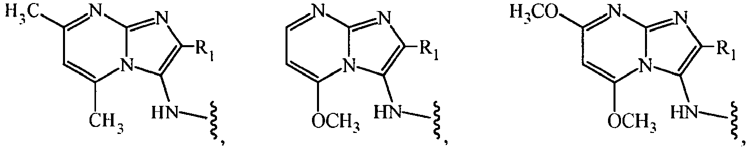 and

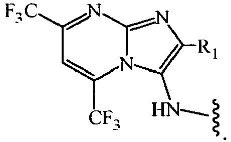

In another aspect, disclosed herein is a compound selected from the group consisting of radiolabelled versions of:

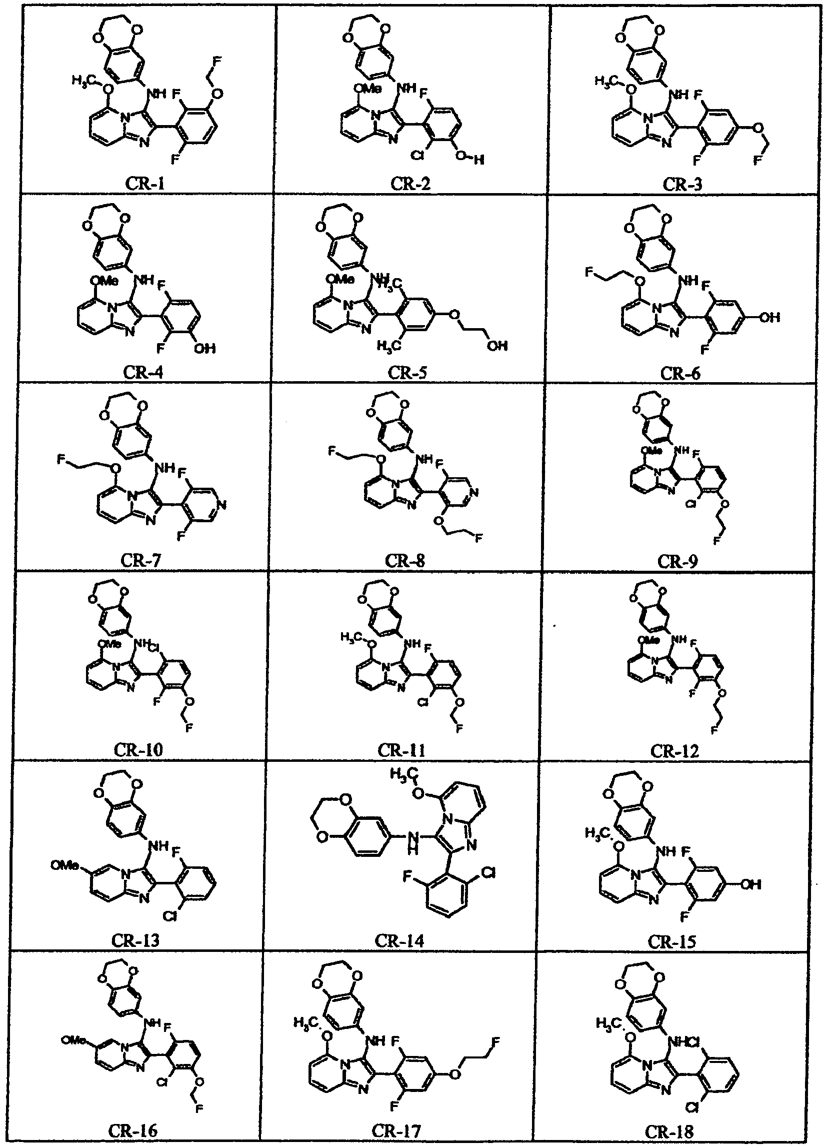

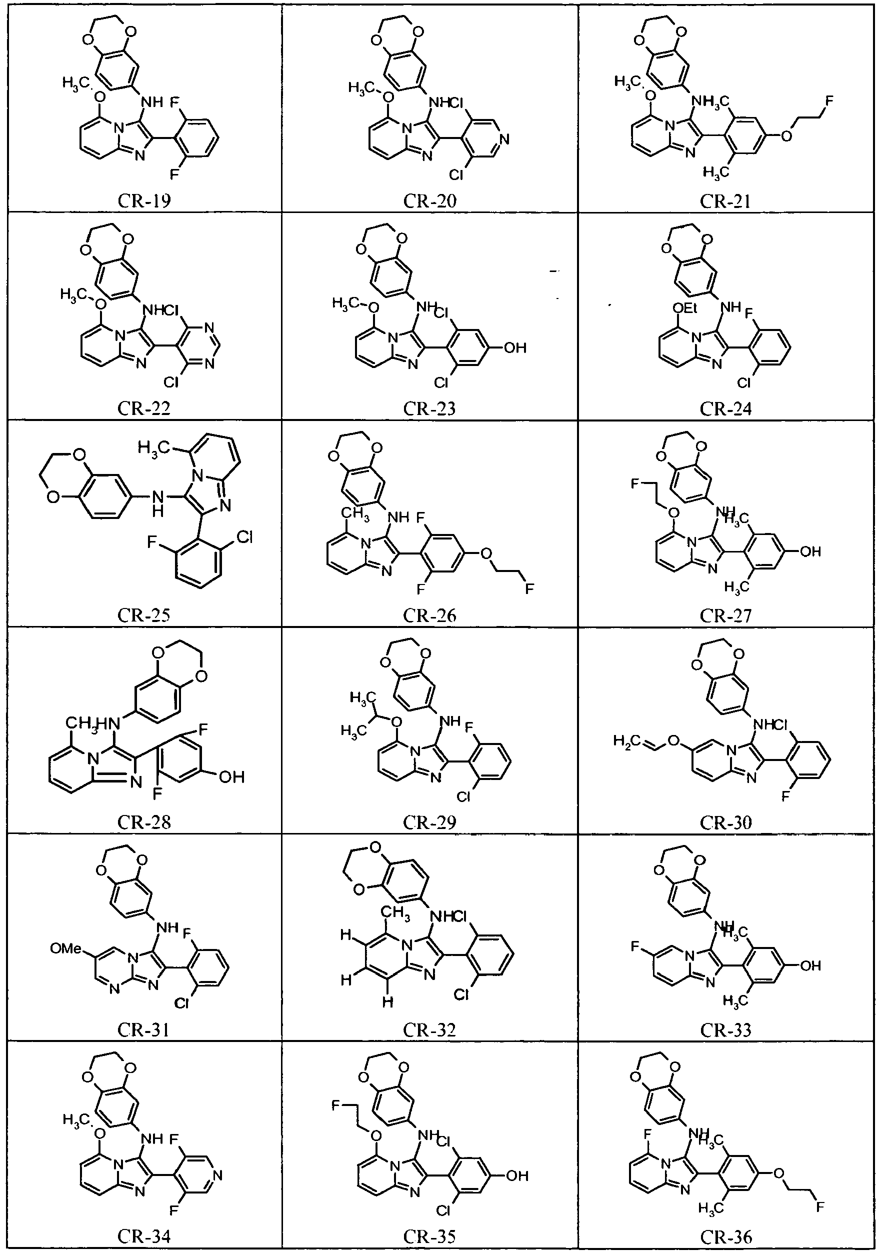

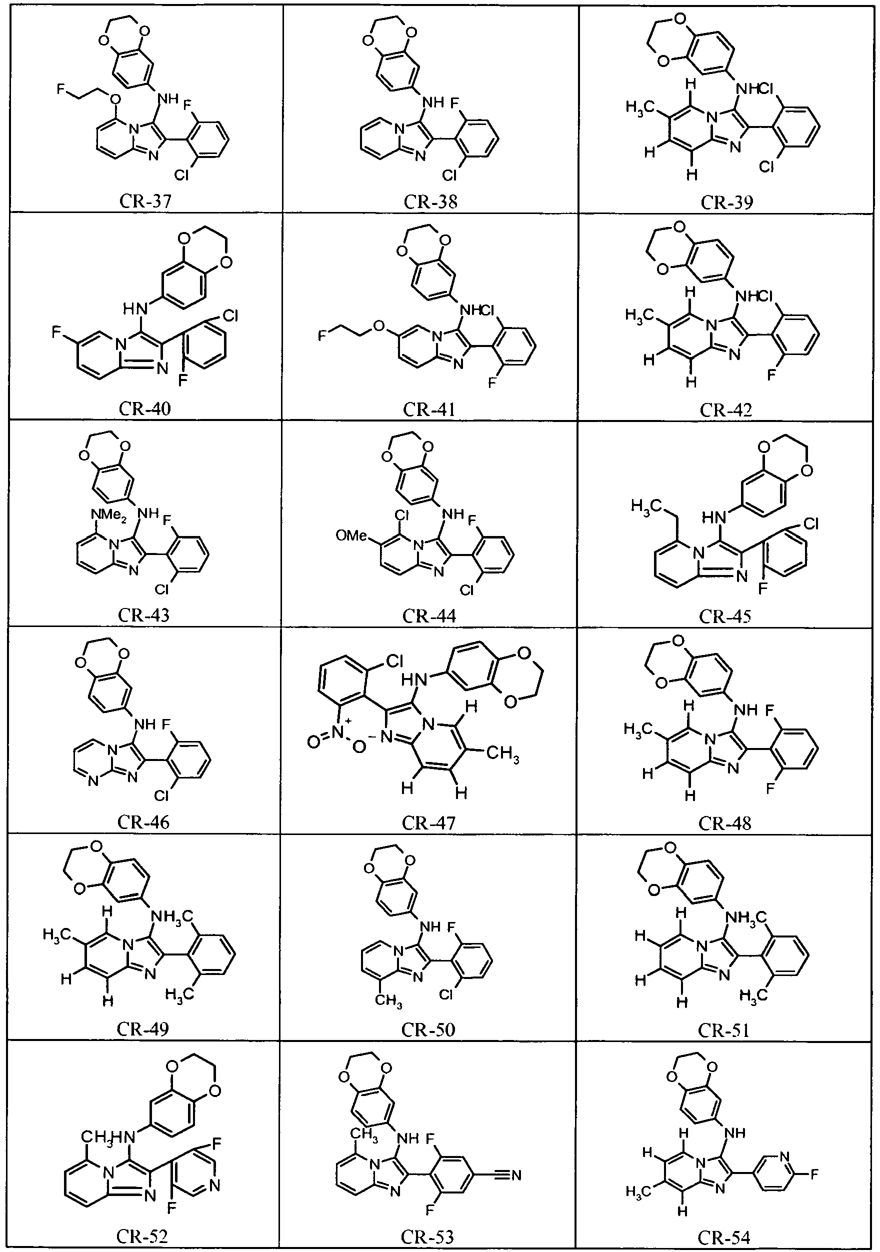

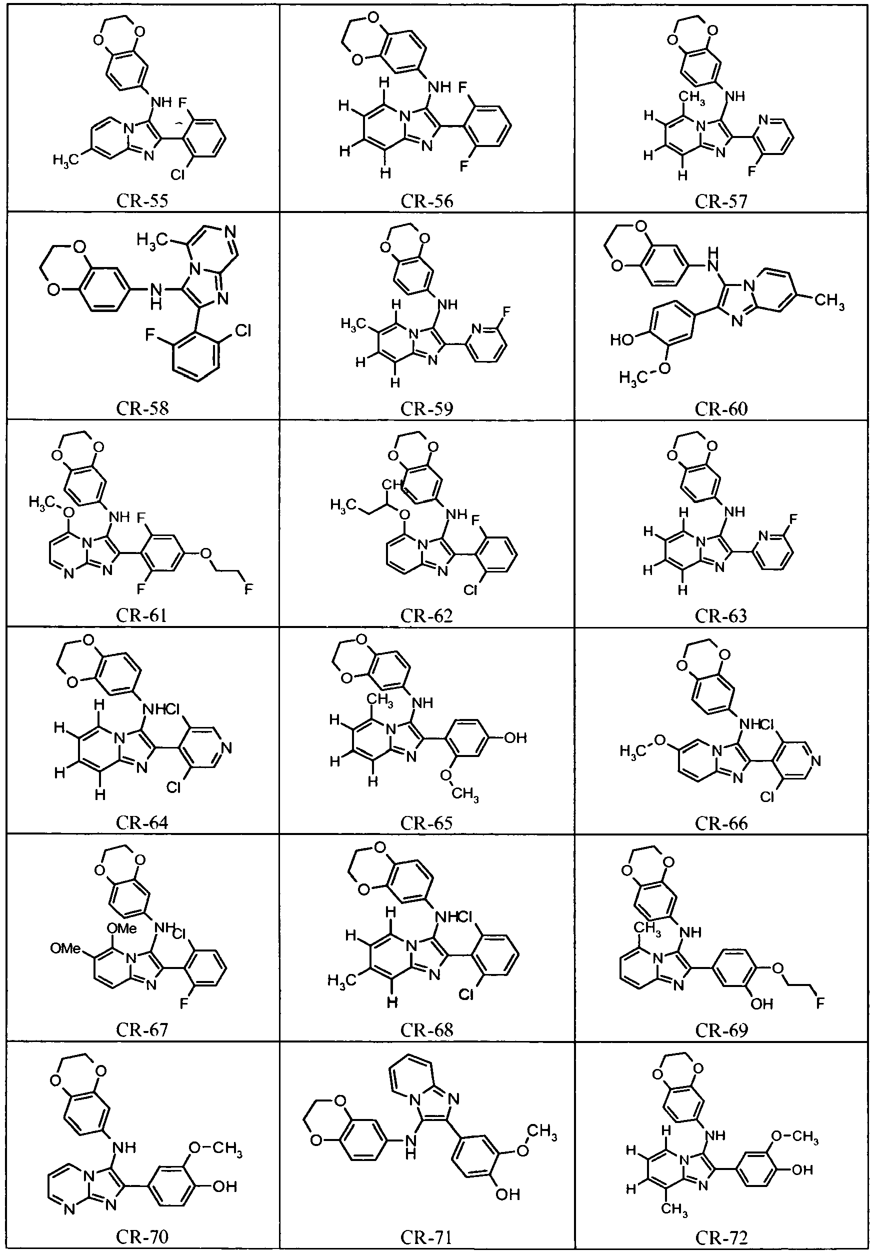

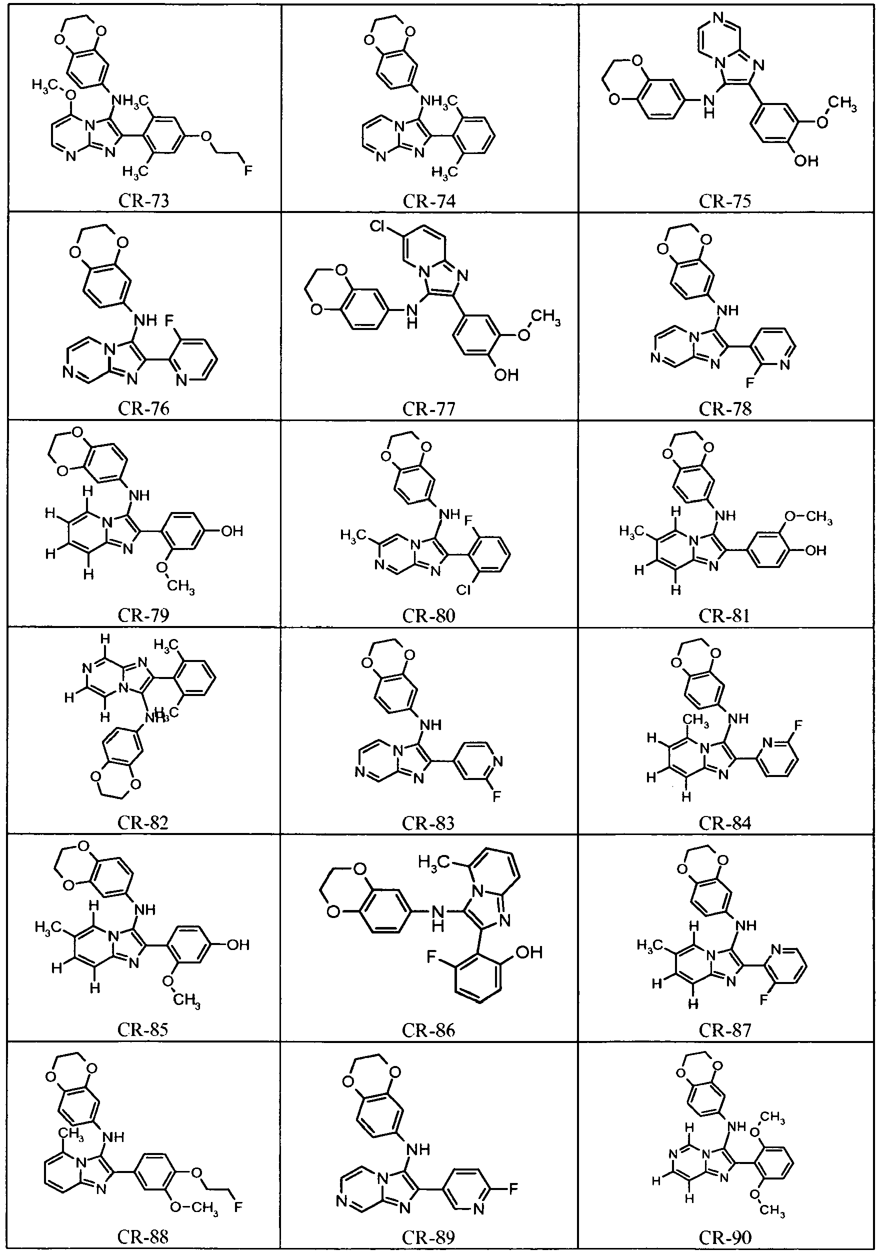

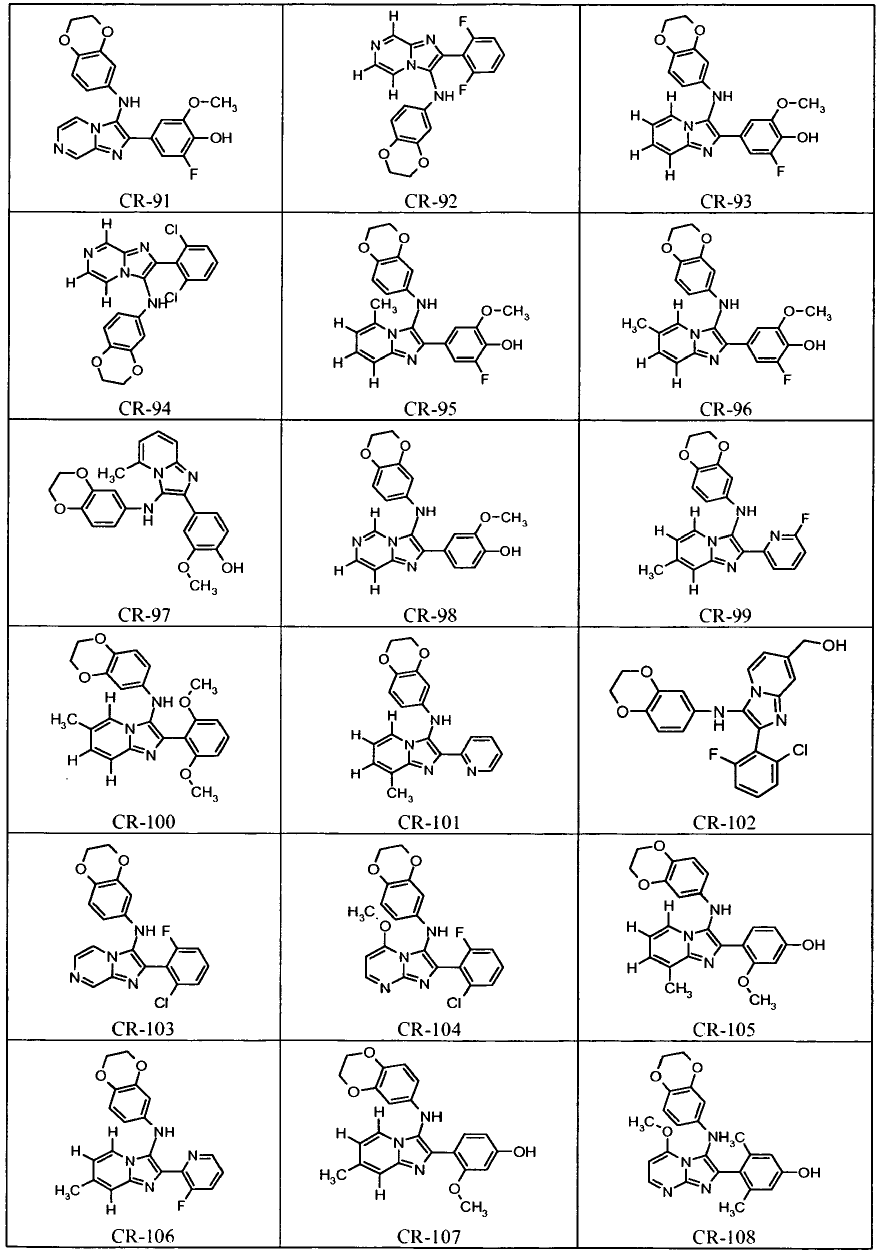

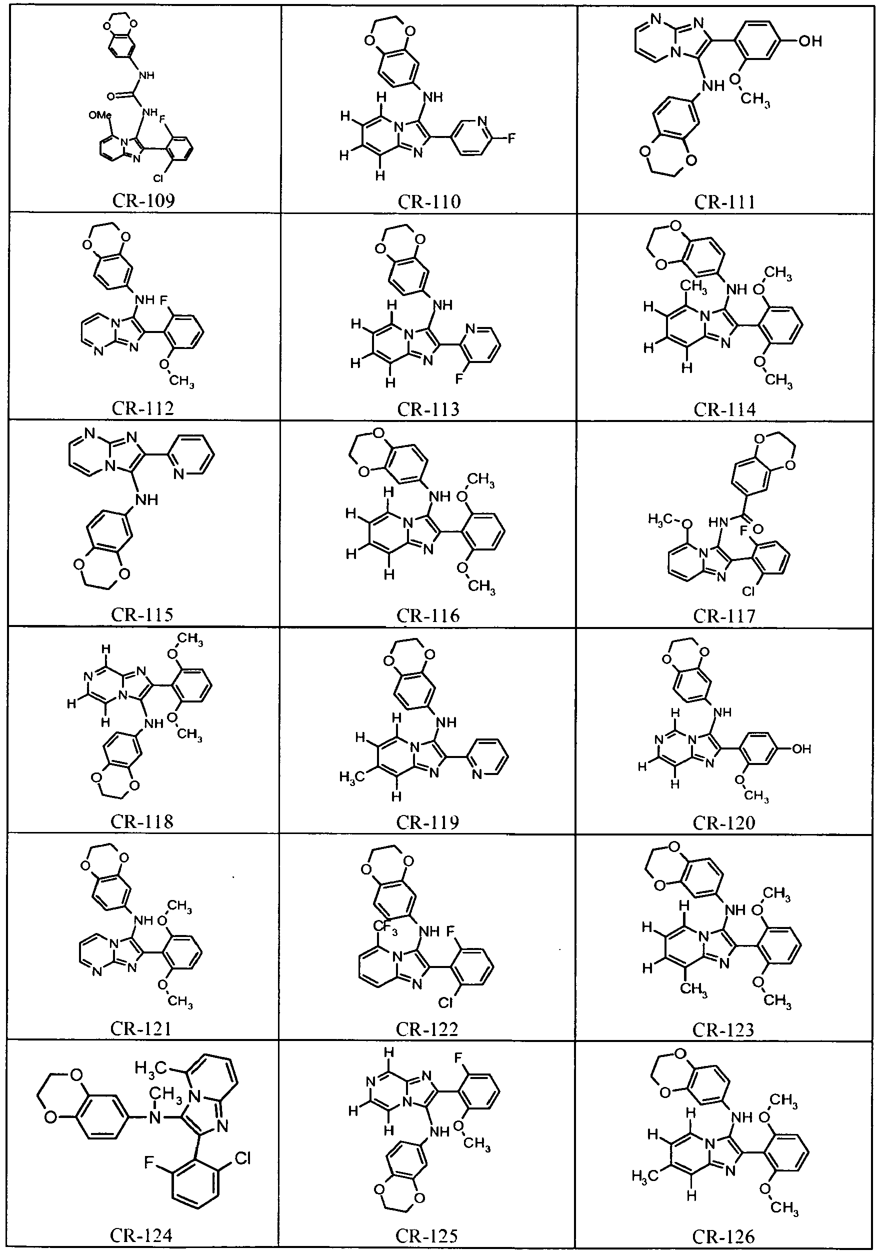

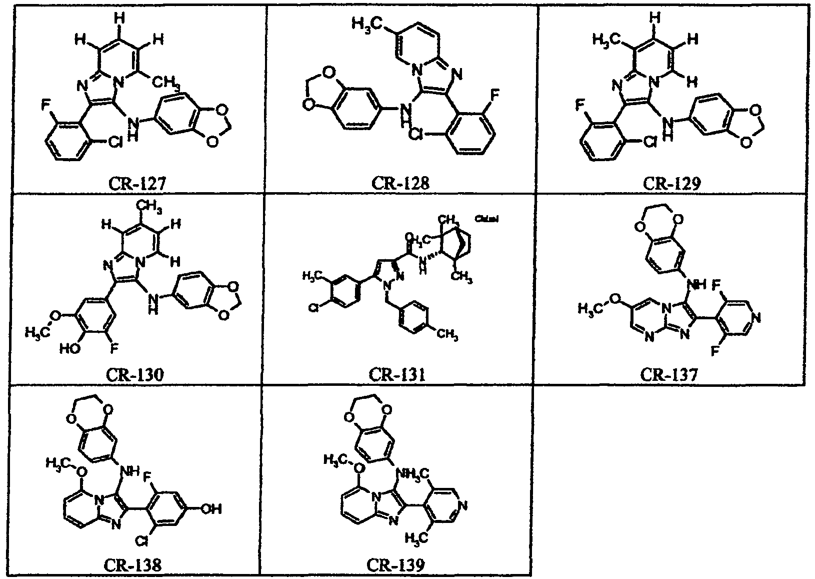

Certain compounds disclosed herein are radiolabeled in vivo imaging agents of Formula I useful, *inter alia,* for imaging cannabinoid CB2 receptors in the central nervous system (CNS) to diagnose CNS abnormalities. Preferred radiolabelled forms of the compounds of Formula I are radioisotope versions of some of the compounds of Formula I as described above. In some embodiments, in vivo imaging agents are compounds of Formula I described above comprising a halogen atom where the halogen atom is a radiohalogen. In some embodiments, such compounds comprise ¹²³I and are particularly suitable for SPECT imaging. In other embodiments, the compounds comprise ¹¹C or ¹⁸F and are particularly suitable for PET imaging. Examples of some ¹⁸F-labelled compounds are provided below:

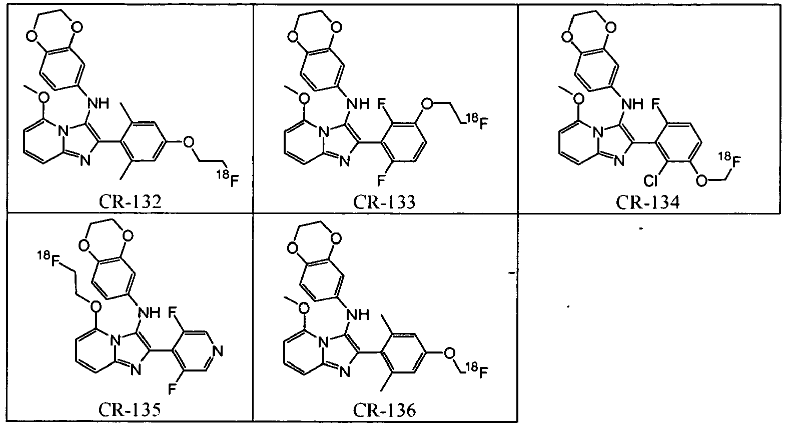

The radiolabeled forms of compounds of Formula I are useful as radioligands to determine the binding of compounds to the cannabinoid CB2 receptor. They are also useful as labeled parent compounds to determine the metabolism of the compound in animals.

The compounds disclosed herein can be synthesized using well-known synthetic organic chemistry techniques. For example, imidazo[1,2-*a*]pyridines disclosed herein can be synthesized using the general reaction scheme set forth in Scheme 1: 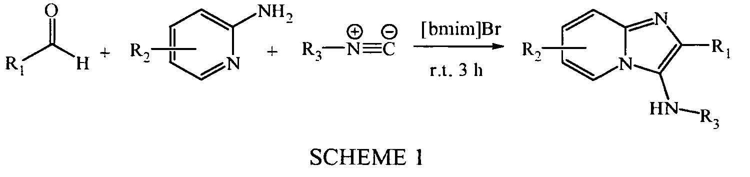 where bmim is 1-butyl3-methylimidazolium bromide, and R₁, R₂ and R₃ can be chosen to mach the desired compound disclosed herein. Full experimental detail is found in Shaabani, A.; Soleimani, E.; Maleki, A. "Ionic liquid promoted one-pot synthesis of 3-amino imidazo[1,2-a]pyridines," Tetrahedron Lett. 2006, 47, 3031-3034. Alternative synthetic methodology is disclosed in Sharma, A.; Behera, G.B. "Condensation of 2-Substituted N-Phenacylium Bromide with p-Dimethylaminobenzaldehyde & p-Nitrosodimethylaniline," Indian J. Chem. 1976, 14B, 551-552.

Imidazo[1,2-*a*]pyrimidines disclosed herein can be synthesized using the general reaction scheme set forth in Scheme 2:

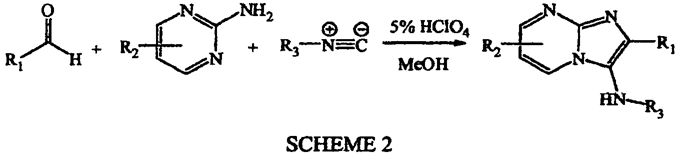

Full experimental detail is found in Bienaymé, H; Bouzid, K. "A New Heterocyclic Multicomponent Reaction for the Combinatorial Synthesis of Fuesed 3-Aminoimidazoles," Angew. Chem. Int. Ed. 1998, 37(16), 2234-2237.

Compounds of Formula I of the present invention may conveniently be prepared by reaction of a precursor compound with a suitable source of the desired radiosotope. A "precursor compound" comprises an unlabelled derivative of the labeled compound, designed so that chemical reaction with a convenient chemical form of the radiosotope occurs site-specifically; can be conducted in the minimum number of steps (ideally a single step); and without the need for significant purification (ideally no further purification), to give the desired labeled compound. Such precursor compounds are synthetic and can conveniently be obtained in good chemical purity. The precursor compound may optionally comprise a protecting group for certain functional groups of the precursor compound. By the term "protecting group" is meant a group which inhibits or suppresses undesirable chemical reactions, but which is designed to be sufficiently reactive that it may be cleaved from the functional group in question under mild enough conditions that do not modify the rest of the molecule. After deprotection, the desired labeled compound is obtained. Protecting groups are well known to those skilled in the art and are described in 'Protective Groups in Organic Synthesis', Theorodora W. Greene and Peter G. M. Wuts, (Third Edition, John Wiley & Sons, 1999). Radiohalogens are preferred radiosotopes of the present invention, with radioiodine and radiofluorine being most preferred.

Where the radiosotope is radioiodine, preferred precursor compounds are those which comprise a derivative which either undergoes electrophilic or nucleophilic iodination or undergoes condensation with a labelled aldehyde or ketone. Examples of the first category are:
(a) organometallic derivatives such as a trialkylstannane (e.g., trimethylstannyl or tributylstannyl), or a trialkylsilane (e.g., trimethylsilyl) or an organoboron compound (e.g., boronate esters or organotrifluoroborates);
(b) a non-radioactive alkyl bromide for halogen exchange or alkyl tosylate, mesylate or triflate for nucleophilic iodination;
(c) aromatic rings activated towards nucleophilic iodination (e.g., aryl iodonium salt aryl diazonium, aryl trialkylammonium salts or nitroaryl derivatives);
d) aromatic rings activated towards electrophilic iodination (eg for iodination sites *ortho* to phenols, anilines).

The precursor compound preferably comprises: a non-radioactive halogen atom such as an aryl iodide or bromide (to permit radioiodine exchange); an organometallic group (e.g. trialkyltin, trialkylsilyl or organoboron compound); or an organic group such as triazenes or a good leaving group for nucleophilic substitution such as an iodonium salt. Preferably for radioiodination, the precursor compound comprises an organometallic group, most preferably trialkyltin. Precursor compounds and methods of introducing radioiodine into organic molecules are described by Bolton [J. Lab. Comp. Radiopharm., 45, 485-528 (2002)]. Suitable boronate ester organoboron compounds and their preparation are described by Kabalka et al [Nucl. Med. Biol., 29, 841-843 (2002) and 30, 369-373(2003)]. Suitable organotrifluoroborates and their preparation are described by Kabalka et al [Nucl. Med. Biol., 31, 935-938 (2004)].

Radiofluorination may be carried out via direct labelling using the reaction of ¹⁸F-fluoride with a suitable chemical group in the precursor compound having a good leaving group, such as an alkyl bromide, alkyl mesylate or alkyl tosylate. ¹⁸F can also be introduced by alkylation of N-haloacetyl groups with a ¹⁸F(CH₂)₃OH reactant, to give -NH(CO)CH₂O(CH₂)₃¹⁸F derivatives. For aryl systems, ¹⁸F-fluoride nucleophilic displacement reactions from an aryl diazonium salt, aryl nitro compound or an aryl quaternary ammonium salt are suitable routes to aryl- ¹⁸F derivatives. A ¹⁸F-labelled compound of the invention may be obtained by formation of ¹⁸F fluorodialkylamines and subsequent amide formation when the ¹⁸F fluorodialkylamine is reacted with a precursor containing, e.g. acid chloride, P(O)Ph₃ or an activated ester. Further details of synthetic routes to ¹⁸F-labelled derivatives are described by Botton, J. Lab. Comp. Radiopharm., 45, 485-528 (2002).
The compounds of Formula I are imaging agents that can be used in either in vitro or in vivo imaging techniques. In vivo imaging techniques are non-invasive diagnostic techniques that generally involve administering a compound comprising a detectable moiety that can be detected externally to the subject. Generally, these methods comprise administering to a subject a compound of Formula I of the present invention, dissolved or dispersed in a suitable pharmaceutical carrier or diluent. The compound of Formula I selectively binds to cannabinoid CB2 receptors, thus permitting the imaging of the receptors and the ability to, *inter alia,* evaluate the chemistry of the particular tissue, the effectiveness of drugs, and organ functions. In vivo imaging techniques suitable for practicing the methods disclosed herein include, but are not limited to, single photon emission computed tomography (SPECT) and positron emission tomography (PET).

In some embodiments, the imaging is conducted as part of an in vitro assay. In these embodiments, the radiolabeled compound of Formula I is administered to a tissue, cell, cell lysate, or a mixture comprising the CB2 receptor, in vitro. The binding of the compound to the particular tissue or cell or the effectiveness of drugs on modulating the activity of the CB2 receptor can then be determined in vitro using in vitro assays well-known in the art. An example of such assay is described below in Example 2. Other examples include contacting a biopsy obtained from a subject with a compound of Formula I to determine whether such biopsy contains CB2 receptors, which may be indicative of a disorder such as multiple sclerosis.

In certain diseases, CB2 receptor expression in a subject is upregulated. In some cases, CB2 receptor expression is upregulated only in particular region of a tissue, for example, a lesion on a tissue, such as brain or lymph nodes. When the compounds disclosed herein are administered to a subject, the compounds disclosed herein bind to CB2 receptors preferentially. Where the compound comprises a detectable moiety suitable for in vivo imaging the location and extent of binding of the compound can be determined. Therefore, if the extent of binding, i.e., the local concentration of the compounds of Formula I, in a subject is measured against a background, areas that show greater binding, i.e., have higher concentrations of the compounds of Formula I, coincide with areas having cells that express CB2 to a greater extent, i.e., the diseased area.

Concentration or extent of binding can be measured using well-known in vivo imaging techniques in the art, such as PET or SPECT scanning (for an overview see "Textbook of In Vivo Imaging in Vertabrates" 2007; published by John Wiley & Sons: Ntziachristos et al, Eds.). In these techniques, a composition comprising a suitably radiolabelled compound is administered to a subject. The radiolabelled compound is typically one that is selective for a particular receptor. In the context of the present disclosure, the composition being administered to the subject comprises a radiolabelled compound of Formula I, which is selective for CB2. In PET imaging, the detectable moiety is a positron emitter wherein the signals emitted are positrons. Gamma radiation, caused by the collision of a positron decayed from the positron emitter with an electron in the subject's body, is detected by a PET scanner. The scanner can localize the source of radiation along a straight line of coincidence (also called formally the "line of response" or LOR). By drawing a number of LORs, the source of radiation, which is the area of a tissue expressing CB2, can be pinpointed. In SPECT imaging, the detectable moiety is a gamma emitter wherein the signals emitted are gamma rays. A gamma camera detects these gamma rays, enabling reconstruction of an image of where the gamma rays originated. Because CB2 is expressed in many different tissues, and not necessarily a diseased tissue, the amount of detected radiation is compared with a background amount of radiation. The background radiation may be radiation detected from different parts of the subject's body or different parts of the same tissue. If radiation differential between the area of interest and the background is greater than a particular threshold, then it can be concluded that the area of interest expresses CB2 to a greater extent than other areas of the body, and that the area of interest may be diseased. In some cases, a database of radiation obtained from healthy subjects is obtained and an average amount of radiation for a healthy subject is calculated. The extent of radiation detected from a subject is compared to this external control to determine whether the subject shows greater than normal CB2 expression in the particular tissue.

Thus, in another aspect, disclosed herein is the compound of Formula I for use in a method of in vivo imaging a first area of a tissue of a subject by, the method comprising:
administering to the subject a pharmaceutical composition comprising a compound of Formula I, wherein the compound comprises a radioisotope;
measuring the signal emitted by the radioisotope from the first area of the tissue; and
comparing the amount of signal emitted from the first area of the tissue to an amount of signal emitted from a control sample.

Preferred methods of in vivo imaging are SPECT and PET, with PET being most preferred.

In some embodiments, the control sample is internal to the subject, which can include a similar tissue or a second area of the same tissue. In other embodiments, the control sample is external to the subject, which may include a database of emissions collected from several subjects.

In some embodiments, the first area of the tissue is a part of the central nervous system (CNS), the nervous system, the immune system, the gastrointestinal tract, the lung, the skin, the liver, the cardiovascular system, or the muscular system. The in vivo imaging method may be used in a method of diagnosing a disorder in a subject, the method comprising:
administering to the subject a pharmaceutical composition comprising a compound of Formula I, wherein the compound comprises a radioisotope;
measuring signal emitted by the radioisotope from a first area of a tissue of the subject;
measuring signal emitted by the radioisotope from a second area of a tissue of the subject;
comparing the signal emitted by the radioisotope from the first area of the tissue to signal emitted by the radioisotope from the second area of the tissue; and
determining whether the signal emitted by the radioisotopefrom the first area of the tissue is greater than the signal emitted by the radioisotopefrom the second area of the tissue.

In some embodiments, the disorder is selected from the group consisting of acute and chronic pain, inflammatory pain, post-operative pain, neuropathic pain, muscle relaxation, a disease or disorder requiring immunosuppression, inflammation, allergies, glaucoma, bronchodilation, neuroprotection, osteoporosis and disorders of the skeletal system, cancer, neurodegenerative disorders, Alzheimer's disease, Parkinson's disease (PD), Huntington's disease, multiple sclerosis (MS), muscle spasticity, tremor, fibromyalgia, lupus, rheumatoid arthritis, myasthenia gravis, autoimmune disorders, irritable bowel syndrome, interstitial cystitis, migraine, pruritis, excema, sebhorea, psoriasis, shingles, cerebral ischemia, cerebral apoplexy, craniocerebral trauma, stroke, spinal cord injury, liver cirrhosis, liver fibrosis, atherosclerosis, as an anti-tussive, asthma, nausea, emesis, gastric ulcers, and diarrhea.

In some embodiments, the disorder is selected from the group consisting of multiple sclerosis, rheumatoid arthritis, arthritis, systemic lupus erythematosus (SLE), myasthenia gravis, diabetes mellitus type I, hepatitis, psoriasis, stroke, migraine, cluster headaches, chronic degenerative diseases, Parkinson's disease, Alzheimer's disease, amyotrophic lateral sclerosis, Huntington's chorea, prison-associate neurodegeneration, peripheral pain, visceral pain, neuropathic pain, inflammatory pain, referred pain, arrhythmia, hypertension, myocardial ischemia, muscle spasm, tremor, malignant brain tumors, skin tumors, lung adenocarcinoma, glioma, and thyroid epithelioma.

In some embodiments, the disorder is an immune related disorder selected from the group consisting of tissue rejection in organ transplants, malabsorption syndromes, celiac, pulmonary diseases, asthma, Sjögren's syndrome, inflammatory bowel disease, and rheumatic diseases.
Preferably, the in vivo imaging method is a method of CB2 imaging by positron emission tomography (PET) or single photon emission computed tomography (SPECT), comprising: a) administering to a subject an amount of a radiolabeled compound of Formula I; and (b) measuring the distribution of the radiolabeled compound in the subject by PET or SPECT.

Certain compounds of the invention may be useful in obtaining an autoradiograph image of a tissue. This aspect, therefore, relates to a method of determining a distribution of CB2 receptors in a tissue comprising administering a radiolabeled compound of Formula I to the tissue and obtaining an image of the tissue.

An autoradiograph is an image produced on an x-ray film or nuclear emulsion by the pattern of decay emissions (e.g., beta particles or gamma rays) from a distribution of a radioactive substance. This technique can be used to determine the tissue localization of a radioactive substance bound to a CB2 receptor. The film or emulsion is apposed to the labeled tissue section to obtain the autoradiograph (also called an autoradiogram).

The use of radiolabeled ligands to determine the tissue distributions of receptors is termed either in vivo or in vitro receptor autoradiography if the ligand is administered into the circulation (with subsequent tissue removal and sectioning) or applied to the tissue sections, respectively.

Pharmaceutical compositions comprising a compound of Formula I are another aspect of the present invention.

The term "subject" refers to an animal, preferably a mammal, and most preferably a human, who is the object of treatment, observation or experiment. The mammal may be selected from the group consisting of mice, rats, rabbits, guinea pigs, dogs, cats, sheep, goats, cows, primates, such as monkeys, chimpanzees, and apes, and humans.

In one aspect, the present invention relates to a pharmaceutical composition comprising a compound of Formula I, and a physiologically acceptable component such as a carrier, a diluent, a salt or an excipient, or a combination thereof.

The term "pharmaceutical composition" refers to a mixture of a compound disclosed herein with other chemical components, such as diluents or carriers. The pharmaceutical composition facilitates administration of the compound to a subject. Multiple techniques of administering a compound exist in the art including, but not limited to, oral, injection, aerosol, parenteral, and topical administration. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like.

The term "carrier" defines a chemical compound that facilitates the incorporation of a compound into cells or tissues. For example dimethyl sulfoxide (DMSO) is a commonly utilized carrier as it facilitates the uptake of many organic compounds into the cells or tissues of a subject.

The term "diluent" defines chemical compounds diluted in water that will dissolve the compound of interest as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound.

The term "physiologically acceptable" defines a carrier or diluent that does not abrogate the biological activity and properties of the compound.

The pharmaceutical compositions described herein can be administered to a subject per se, or in pharmaceutical compositions where they are mixed with other active ingredients, as in combination therapy, or suitable carriers or excipient(s). Techniques for formulation and administration of the compounds of the instant application may be found in "Remington's Pharmaceutical Sciences," Mack Publishing Co., Easton, PA, 18th edition, 1990. For in vivo imaging techniques, the preferred route of administration is intravenous.

Alternatively, one may administer the drug in a targeted drug delivery system, for example, in a liposome coated with a tissue-specific antibody. The liposomes will be targeted to and taken up selectively by the organ.

Pharmaceutical compositions for use in accordance with the present disclosure thus may be formulated in a conventional manner using one or more physiologically acceptable carriers comprising excipients and auxiliaries, which facilitate processing of the active compounds into preparations, which can be used pharmaceutically. Proper formulation is dependent upon the route of administration chosen. Any of the well-known techniques, carriers, and excipients may be used as suitable and as understood in the art; e.g., in Remington's Pharmaceutical Sciences, above.

For injection, the agents disclosed herein may be formulated in aqueous solutions, preferably in physiologically compatible buffers such as Hank's solution, Ringer's solution, or physiological saline buffer. For transmucosal administration, penetrants appropriate to the barrier to be permeated are used in the formulation. Such penetrants are generally known in the art.
Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances, which increase the viscosity of the suspension, such as sodium carboxymethyl cellulose, sorbitol, or dextran. Optionally, the suspension may also contain suitable stabilizers or agents, which increase the solubility of the compounds to allow for the preparation of highly, concentrated solutions.

A pharmaceutical carrier for the hydrophobic compounds disclosed herein is a cosolvent system comprising benzyl alcohol, a nonpolar surfactant, a water-miscible organic polymer, and an aqueous phase. A common cosolvent system used is the VPD co-solvent system, which is a solution of 3% w/v benzyl alcohol, 8% w/v of the nonpolar surfactant Polysorbate 80™, and 65% w/v polyethylene glycol 300, made up to volume in absolute ethanol. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of POLYSORBATE 80™; the fraction size of polyethylene glycol may be varied; other biocompatible polymers may replace polyethylene glycol, e.g., polyvinyl pyrrolidone; and other sugars or polysaccharides may be used.

Alternatively, other delivery systems for hydrophobic pharmaceutical compounds may be employed. Liposomes and emulsions are well known examples of delivery vehicles or carriers for hydrophobic drugs. Certain organic solvents such as dimethylsulfoxide also may be employed, although usually at the cost of greater toxicity.

Many of the compounds used in the pharmaceutical combinations disclosed herein may be provided as salts with pharmaceutically compatible counterions. Pharmaceutically compatible salts may be formed with many acids, including but not limited to hydrochloric, sulfuric, acetic, lactic, tartaric, malic, succinic, etc. Salts tend to be more soluble in aqueous or other protonic solvents than are the corresponding free acids or base forms.

### EXAMPLES

The following examples are provided as an illustration of the present invention, but should in no way be considered as limiting the scope of invention.

### Example 1. Synthesis of the Compounds

### Analytical methods

### Analytical HPLC/MS, Ammonium Acetate (AP)

*System*: Waters/Micromass ZQ2000 LC/MS system consisting of a ZQ single quadropole mass spectrometer equipped with an electrospray ionization interface, and a Waters Alliance HT with a 2795 Separation Module and 996 Photodiode Array Detector.

*Column*: Reversed phase column (Waters Xterra® MS C₁₈ 3.5µm, 30x4.6mm ID) with a guard column cartridge system.

*Mobile* Phase: A: 10mM aqueous Ammonium Acetate; B: 10mM aqueous Ammonium Acetate Acetonitrile/Water (95:5).

*Program*: 10 min. gradient program starting at 30%B (initial hold for 0.5 min.), over 5 min. to 100%B, hold for 1.5 min., over 0.5 min. to 30%B, hold for 2.5 min. The flow rate was I mL/min.

### Preparative HPLC/MS, Ammonium Acetate (PP)

*System*: Waters/Micromass LC/ZMD Autopurification system consisting of a ZMD single quadropole mass spectrometer equipped with an electrospray ionization interface, and a Waters 600E Gradient Pump with in-line degassing, 2700 Sample Manager and 996 Photodiode Array Detector.

*Column*: Reversed phase column (Waters Xterra® Prep MS C₁₈ 5µm, 19x100mm).

*Mobile Phase*: A: 10mM aqueous Ammonium Acetate; B: 10mM aqueous Ammonium Acetate Acetonitrile/Water (95:5).

*Program*: 12 min. gradient program starting at 30%B (initial hold for 2.5 min.), over 8.5 min. to 100%B, over 0.5 min. to 30%B, hold for 0.5 min. The flow rate was 17 mL/min.

### Building block synthesis

### ALDEHYDES

### General procedure GP1

### 4-(2-fluoroethoxy)-2,6-dimethylbenzaldehyde

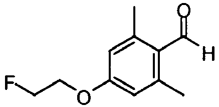

To a solution of 2-fluoroethanol (0.55 mL, 9.4 mmol) and *N,N-*diisopropylethylamine (3.2 mL, 18.7 mmol) in CH₂Cl₂ (35 mL) at -78°C was added Tf₂O (1.44 mL, 8.7 mmol) dropwise. The mixture was stirred for 1.5h. A solution of 4-hydroxy-2,6-dimethylbenzaldehyde (1.02 g, 6.8 mmol) in CH₂Cl₂ (4 mL) + DMF (2 mL) was added dropwise. After stirring for 1h the cooling bath was removed and the mixture stirred at room temperature overnight. The mixture was then diluted with diethyl ether (200 mL) and this mixture was washed with H₂O (2x 40mL), I M HCl (40 mL), 1 M NaOH (40 mL) and brine and then dried over Na₂SO₄. After evaporation to dryness the title compound was obtained as a yellow oil that solidified on standing (1.01g, 76%). ¹H NMR (400 MHz, CDCl₃) δ 10.48 (s, 1H), 6.62 (s, 2H), 4.82-4.80 (m, 1H), 4.70-4.68 (m, 1H), 4.30-4.28 (m, 1H), 4.23-4.21 (m, 1H), 2.60. ¹³C NMR (100 MHz, CDCl₃) δ 191.5, 161.4, 144.4, 126.4, 115.3, (82.4 + 80.7, d, *J* = 170 Hz), (67.0+66.8, d, *J* = 20 Hz), 21.0.

### 2,6-difluoro-3-(2-fluoroethoxy)benzaldehyde

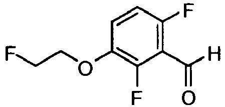

Prepared according to GP1 by using 2-fluoroethanol (0.34 mL, 5.8 mmol), 2,6-difluoro-3-hydroxybenzaldehyde (700 mg, 4.43 mmol), *N,N-*diisopropylethylamine (1.89 mL, 11.1 mmol) and Tf₂O (0.92 mL, 5.5 mmol) in CH₂Cl₂ (30 mL) yielding the title compound as a yellow oil (647 mg, 72%). ¹H NMR (400 MHz, CDCl₃) δ 10.35 (m, 1H), 7.27-7.21 (m, 1H), 6.94-6.89 (m, 1H), 4.82-4.80 (m, 1H), 4.70-4.68 (m, 1H), 4.34-4.32 (m, 1H), 4.28-4.26 (m, 1H).

### 2-chloro-4-fluoro-1-(2-fluoroethoxy)benzene

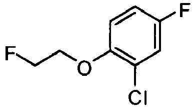

Prepared according to GP1 by using 2-fluoroethanol (0.85 mL, 14.5 mmol), 2-chloro-4-fluorophenol (1.67 g, 11.4 mmol), *N,N-*diisopropylethylamine (5.00 mL, 29.2 mmol) and Tf₂O (2.40 mL, 14.5 mmol) in CH₂Cl₂ (60 mL) with the exception that the phenol was added in neat CH₂Cl₂ (3 mL). This yielded the title compound as a orange oil (2.22 g, 100%). ¹H NMR (400 MHz, CDCl₃) δ 7.16-7.12 (m, 1H), 6.94-6.91 (m, 2H), 4.84-4.81 (m, 1H), 4.72-4.70 (m, 1H), 4.29-4.27 (m, 1H), 4.23-4.20 (m, 1H).

### 4-(2-fluoroethoxy)-3-methoxybenzaldehyde

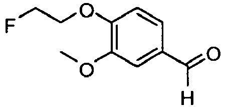

Prepared according to GP1 by using 2-fluoroethanol (628.0 mg, 9.28 mmol), *N,N-*diisopropylethylamine (3.56 mL, 2.1 mmol), Tf₂O (1.51 mL, 9.1 mmol) in CH₂Cl₂ (35 mL) and vanillin (1.06 g, 7.0 mmol) in CH₂Cl₂:DMF 2:1 (6 mL) to give the title compound (1.41 g, 100%). LCMS *m*/*z* 199 [M+H]⁺, purity (UV/MS) 99/84.

### 2,6-difluoro-4-(2-fluoroethoxy)benzaldehyde

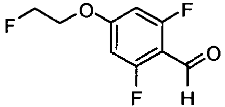

Prepared according to GP1 by using 2-fluoroethanol (628.0 mg, 9.28 mmol), *N,N-*diisopropylethylamine (3.56 mL, 2.1 mmol), Tf₂O (1.51 mL, 9.1 mmol) and 2,6-difluoro-4-hydroxybenzaldehyde (1.10 g, 7.0 mmol) in CH₂Cl₂ (35 mL) to give the title compound (1.2 g, 84%). ¹H NMR (CDCl₃, 400 MHz) 8 10.20 (s, 1H, CHO), 6.54-6.51 (m, 2H, ArH), 4.83-4.81 (m, 1H, CH₂), 4.72-4.70 (m, 1H, CH₂), 4.31-4.30 (m, 1H, CH₂), 4.24-4.22 (m, 1H, CH₂). LCMS *m*/*z* 205 [M+H]⁺, purity (UV/MS) 99/45.

### 4-(2-fluoroethoxy)-3-hydroxybenzaldehyde

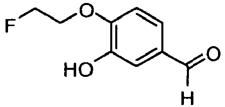

Prepared according to GP1 by using 2-fluoroethanol (628.0 mg, 9.28 mmol), *N,N*-diisopropylethylamine (3.56 mL, 2.1 mmol), Tf₂O (1.51 mL, 9.1 mmol) in CH₂Cl₂ (35 mL) and 3,4-dihydroxybenzaldehyde (967.0 mg, 7.0 mmol) in CH₂Cl₂:DMF 2:1 (6 mL). The workup followed the original procedure but the extraction with NaOH (1M) was acidified with HCl(aq) and extracted with EtOH to afford the title compound (1.1 g, 86%). ¹H NMR (CDCl₃, 400 MHz) δ 9.76 (s, 1H, CHO), 7.42-7.39 (m, 1H, ArH), 7.33-7.32 (m, 1H, ArH), 7.11-7.09 (m, 1H, ArH), 4.85-4.84 (m, 1H, CH₂), 4.74-4.72 (m, 1H, CH₂), 4.53 (br s, 1H, OH), 4.41-4.39 (m, 1H, CH₂), 4.34-4.32 (m, 1H, CH₂). LCMS *m*/*z* 185 [M+H]⁺, purity (UV/MS) 56/53.

### 4-(2-hydroxyethoxy)-2,6-dimethylbenzaldehyde

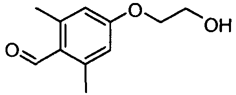

A dry flask was charged with 4-hydroxy-2,6-dimethylbenzaldehyde (2.04 g, 13.6 mmol), ethylenecarbonate (1.58 g, 17.9 mmol) and K₂CO₃ (2.81 g, 20.3 mmol) in DMF (30 mL) and heated to 80 °C overnight. The mixture was poured into diethyl ether (400 mL) and the organic layer was washed with H₂O (2x 100 mL), 2M HCl (50 mL) and 2M NaOH (50 mL). The organic layer was then washed with brine followed by drying over Na₂SO₄ to give the title compound as a yellow oil that solidified on standing (1.58g, 60%). ¹H NMR (400 MHz, CDCl₃) δ 10.48 (s, 1H), 6.61 (s, 2H), 4.14-4.12 (m, 2H), 3.98-3.96 (m, 2H), 2.60 (s, 6H).

### General procedure GP2

### 2-chloro-4-fluoro-1-(fluoromethoxy)benzene

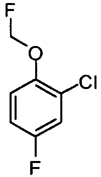

A MW reaction vessel was charged with 2-chloro-4-fluorophenol (1.03 g, 7.05 mmol), K₂CO₃ (1.31 g, 9.48 mmol) and CH₃CN (4 mL). The mixture was cooled to 0 °C before addition of bromofluoromethane (0.50 mL, 7.80 mmol). The vessel was capped and heated to 120 °C for 30 min. After cooling to room temperature the mixture was diluted with diethyl ether (200 mL). The organic layer was washed with H₂O, 2M NaOH, brine and then dried over Na₂SO₄ and evaporated to dryness to give the pure title compound as a colorless liquid that solidified on standing. Yield 1.12 g (89%). ¹H NMR (400 MHz, CDCl₃) δ 7.20-7.15 (m, 2H), 6.99-6.94 (m, 1H), 5.74 (s, 1H), 5.61 (s, 1H).

### 4-chloro-2-fluoro-1-(fluoromethoxy)benzene

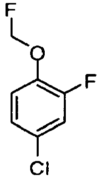

Prepared according to GP2 by using 4-chloro-2-fluorophenol (1.71 g, 11.7 mmol), K₂CO₃ (1.93 g, 14.0 mmol) and bromofluoromethane (1.00 mL, 15.6 mmol) yielding the title compound (1.94g, 93%). ¹H NMR (400 MHz, CDCl₃) δ 7.17-7.07 (m, 3H), 5.74 (s, 1H), 5.61 (s, 1H).

### General procedure GP3

### 2-chloro-6-fluoro-3-(fluoromethoxy)benzaldehyde

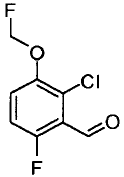

A dry flask was charged with 2-chloro-4-fluoro-1-(fluoromethoxy)benzene (1.12 g, 6.27 mmol) in THF (20 mL) and cooled to -65 °C (int). A solution of *n*-BuLi (2.0 M, 3.50 mL, 7.00 mmol) was added dropwise keeping the internal temperature below -50 °C. The mixture was stirred between -50 °C and - 65 °C for 30 min and then cooled to -78 °C. DMF (1.00 mL, 12.9 mmol) was added in one portion and the mixture was stirred at -78 °C for 15 min before being allowed to reach room temperature. The mixture was poured into ice-water which was slightly acidified with 2M HCl. The aquous layer was extracted with diethyl ether (3x 50 mL). The combined organic layers were washed with 2M HCl, H₂O, brine and dried over Na₂SO₄. Evaporation gave the title compound as yellow crystals (1.28 g, 99%). ¹H NMR (400 MHz, CDCl₃) δ 10.45 (s, 1H), 7.44-7.40 (m, 1H), 7.12-7.08 (m, 1H), 5.78 (m, sH), 5.64 (s, 1H).

### 2-chloro-6-fluoro-3-(2-fluoroethoxy)benzaldehyde

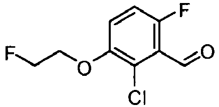

Prepared according to GP3 by using 2-chloro-4-fluoro-1-(2-fluoroethoxy)benzene (2.22 g, 11.5 mmol), n-BuLi (2.0 M, 6.34 mL, 12.7 mmol) and DMF (1.79 mL, 23.1 mmol) in THF (35 mL) yielding the title compound (2.49 g, 98%). ¹H NMR (400 MHz, CDCl₃) δ 10.45 (s, 1H), 7.19-7.15 (m, 1H), 7.08-7.03 (m, 1H), 4.86-4.84 (m, 1H), 4.74-4.72 (m, 1H), 4.33-4.31 (m, 1H), 4.26-4.24 (m, 1H).

### 6-chloro-2-fluoro-3-(fluoromethoxy)benzaldehyde

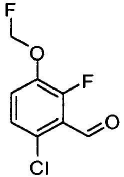

Prepared according to GP3 by using 4-chloro-2-fluoro-1-(fluoromethoxy)benzene (1.94 g, 10.8 mmol), *n*-BuLi (2.2 M, 5.50 mL, 12.1 mmol) and DMF (2.00 mL, 25.8 mmol) in THF (30 mL) yielding the title compound (2.21 g, 99%). ¹H NMR (400 MHz, CDCl₃) δ 10.43 (s, 1H), 7.39-7.35 (m, 1H), 7.26-7.22 (m, 1H), 5.77 (s, 1H), 5.63 (s, 1H).

### 2-Chloro-6-fluoro-4-hydroxybenzaldehyde

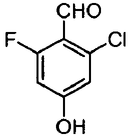

A round bottom flask fitted with internal thermometer and refluxcondenser was charged with 3-chloro-5-fluorophenol (20 mmol, 2.92 g), calcium hydroxide (86 mmol, 6.36 g) and sodium carbonate (69 mmol, 7.31 g) in water (50 mL). Chloroform (40 mmol, 4.77g) was added and the reaction heated to 80 °C, at this temperature the reaction became exothermic and the heating was stopped, after 30 min the heating was resumed and the reaction heated to 120 °C for 2 h. After cooling to rt the reaction mixture was acidified with conc. HCl (17 mL), then filtered through celite and extracted with diethyl ether. Dried over Na₂SO₄ and concentrated *in vacuo.* Part of the obtained dark red oil precipitated, the precipitate was filtered off and purified by flash chromatography (eluent: 50% EtOAc in heptane) yielding the title compound (522 mg, 15%). ¹H NMR (400 MHz, CDCl₃) δ 10.31 (s, 1H), 6.77 (d, *J*= 1.3, 1H). 6.57 (d, *J* = 11.8, 1H), 6.27 (s, 1H), 2.05 (s, OH), 1.63 (s, 3H), 1.25 (s, 5H).

### 2-Chloro-6-fluoro-3-methoxybenzaldehyde

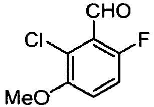

In a round bottom flask fitted with an argon inlet 2-chloro-4-fluoro-1-methoxybenzene (10 mmol, 1.6 g) was taken up in dry THF (20 mL) and cooled to - 65 °C. n-BuLi (11.2 mmol, 2.5 M, 4.5 mL) was added drop wise while keeping the temperature below -55 °C, the reaction was left at -65 °C for 30 min, then cooled to - 78 °C before adding DMF in one portion. Left on the cooling bath for 30 min, then allowed to warm up to rt poured onto ice waterand extracted with ether. The combined organic phase was dried over Na₂SO4 and then concentrated *in vacuo.* The title compound was obtained as a white solid (1.71 g, 90%). ¹H NMR (400 MHz, CDCl₃) cS 10.46 (s, 1H), 7.16 - 7.01 (m, 3H), 3.95 (d, *J*= 1.5, 3H).

### 2-Chloro-6-fluoro-3-hydroxybenzaldehyde

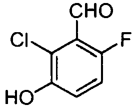

2-Chloro-6-fluoro-3-methoxybenzaldehyde (9 mmol, 1.71 g) was taken up in CH₂Cl₂ and cooled to 0 °C on an ice bath. Boron tribromide (18.6 mmol, 1M, 18.6 mL) was added drop wise over 15 min. The reaction mixture was allowed to warm to rt over night, then quenched with ice water and extracted with CH₂Cl₂. Dried over Na₂SO₄ and concentrated *in vacuo.* The title compound was obtained as an oil (1.4 g, 90%). ¹H NMR (400 MHz, CDCl₃) δ 10.41 (s, 1H), 7.38 - 7.14 (m, 1H), 7.07 (t,*J*=9.4, 1H), 5.75 (s, 1H).

### 4-(t-Butyldimethylsilyloxy)-2,6-dimethylbenzaldehyde

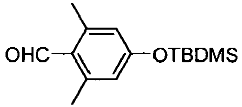

In a round bottom flask fitted with an argon inlet 4-hydroxy-2,6-dimethylbenzaldehyde (9.0 mmol, 1.2 g) was taken up in THF (75 mL). TEA (18 mmol, 1.81 g) was added followed by *t*-butylchlorodimethylsilane (12 mmol, 1.81 g). The reaction mixture was left at rt for 2h, then concentrated onto celite. Purified by flash chromatography (eluent: 20 % EtOAc in heptane) yielding the title compound (2.1 g, 88%). ¹H NMR (400 MHz, CDCl₃) δ 10.30 (s, 1H), 6.35 (s, 2H), 2.40 (d, *J*= 5.9, 7H), 0.81 (s, 9H), 0.05 (s, 6H).

### AMINES

### 5-methoxypyrimidin-2-amine

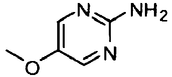

A MW reaction vessel was charged with 2-chloro-5-methoxypyrimidine (0.817 g, 5.65 mmol) and 25% NH₃(aq). The vessel was capped and heated to 150 °C for 3h. The mixture was evaporated to dryness. The resulting material was dissolved in CH₂Cl₂:MeOH (1:1) and adsorbed onto silica. Purification by flash CC (eluent: 50-100 % EtOAc in heptane) gave the title compound as colorless crystals (386 mg, 55%). ¹H NMR (400 MHz, dmso-_{d6}) δ 8.02 (s, 1H), 6.06 (br s, 1H), 3.71 (s, 3H).

### ISOCYANIDES

### General procedure GP4

### 6-(isocyanomethyl)-2,3-dihydrobenzo[b][1,4]dioxine

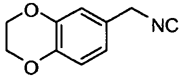

To a MW vial equipped with a stirring bar was added (2,3-dihydrobenzo[b][1,4]dioxin-6-yl)methanamine (5 mmol, 825 mg) and ethyl formate (1 mL). The vial was capped and heated to 120 °C for 15 min. The crude was checked by GCMS, and then concentrated *in vacuo.* The crude was taken up in CH₂Cl₂ and Burgess reagent (7.5 mmol, 1.79 g) was added before recapping the vial. The mixture was heated in a MW reactor at 100 °C for 10 sec and then filtered through a plug of silica (eluent: heptane/CH₂Cl₂ 3:1 to CH₂Cl₂ 100%) yielding the title compound (430 mg, 50%). ¹H NMR (400 MHz, CDCl₃) δ 6.89-6.85 (m, 2H), 6.82-6.77 (m, 1H), 4.51 (s, 2H), 4.28-4.22 (m, 4H). ¹³C NMR (100 MHz, CDCl₃) δ 159.7, 143.8, 143.6, 125.5, 119.7, 117.6, 115.8, 64.3, 64.2, 44.9.

### OTHER BBs

### 2-(2-chloro-6-fluorophenyl)-5-methoxyimidazo[1,2-a]pyridin-3-amine

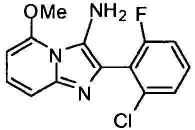

In a MW vial equipped with a magnetic stirring bar 6-methoxypyridin-2-amine (1.0 mmol, 124 mg), 2-fluoro-6-methoxybenzaldehyde (1.2 mmol, 190 mg) and polymer-bound scandium(III) bis(trifluoromethanesulfonate) (0.05 mmol, 14 mg) was dissolved in methanol (5 mL). Trimethylsilylcyanide (3 mmol, 297 mg) was added, the vial was capped and heated in a MW reactor at 140 °C for 20 min. The vial was decapped and sulfuric acid (conc., 5 drops) was added, the reaction mixture was left stirring at room temperature for 5 min, and then passed through a SCX cartridge. The crude product was purified by flash CC (eluent: 0-50 % EtOAc in heptane) yielding the title compound (106 mg, 36%). LCMS *m*/*z* 292 [M+H]⁺, purity (UV/MS) 93/78.

### t-Butyl 6-fluoropyridin-2-ylcarbamate + N,N-di-boc-6-fluoropyridine-2-amine

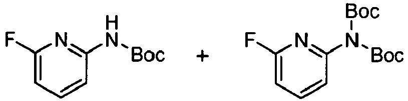

6-fluoropyridin-2-amine (5.00 g, 44.6 mmol) and Boc₂O (11.7 g, 53.5 mmol) was dissolved in THF (100 mL) and cooled to 0 °C under N₂. NaHMDS (26.8 mL, 2M in THF) was added dropwise and the reaction mixture was allowed to warm to rt overnight. The solvent was evaporated and the residue was separated between EtOAc and brine. The organic phase was washed with brine, dried and concentrated. Flash chromatography (silica, 0-20% heptane / EtOAc) gave 2.14 g of *t-*butyl 6-fluoropyridin-2-ylcarbamate and 5.73 g of *N,N-*di-Boc-6-fluoropyridine-2-amine. Combined yield 63%. *t*-Butyl 6-fluoropyridin-2-ylcarbamate: ¹H NMR (400 MHz, CDCl₃) δ 7.86 - 7.65 (m, 2H), 7.27 (s, 1H), 6.63 - 6.46 (m, 1H), 1.51 (s, 9H). *N,N*-di-Boc-6-Fluoropyridine-2-amine: ¹H NMR (400 MHz, CDCl₃) δ 7.80 (td, *J* = 0.7 Hz, 8.2 Hz, 1H), 7.21 - 7.12 (m, 1H), 6.83 (dd, *J* = 2.8 Hz, 8.1 Hz, 1H), 1.44 (s, 18H).

### 2-(6-Aminopyridin-2-yloxy)ethanol

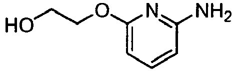

*t*-Butyl 6-fluoropyridin-2-ylcarbamate (10.0 mmol) or *N,N*-di-boc-6-fluoropyridine-2-amine (6.0 mmol) was dissolved in dry THF (10 mL) and ethylene glycol (6 mL) in a MW vial. NaH (3 eq, ~55w% in oil) was carefully added in portions through a stream a nitrogen. After effervescence had ceased the vial was capped and heated in the MW (140 °C, 30 min). Brine and EtOAc was added to the reaction mixture, shaken and separated and the organic phase was washed with brine, dried and concentrated. The resulting two-phase oily mixture was separated between heptane and methanol and the methanolic phase was concentrated and subjected to flash chromatography (silica, 10-80% heptane / EtOAc) to give 52-58% of the desired product.

In one preparation using *N,N*-di-Boc-6-fluoropyridine-2-amine (6.0 mmol) and using MW heating (120 °C, 30 min) it was possible to isolate 14% of *N-*Boc-2-(6-aminopyridin-2-yloxy)ethanol and 36% of the title compound. 2-(6-Aminopyridin-2-yloxy)ethanol: ¹H NMR (400 MHz, CDCl₃) δ 7.35 (dd, *J*= 7.9 Hz, 1H), 6.11 (d, *J* = 7.9 Hz, 1H), 6.07 (d, *J*= 7.8 Hz, 1H), 4.38 - 4.30 (m, 2H), 4.04 (s, 2H), 3.93 - 3.83 (m, 3H). ¹³C NMR (100 MHz, CDCl3) δ 163.4, 157.0, 140.9, 100.4, 99.3, 68.5, 62.6. *N*-Boc-2-(6-aminopyridin-2-yloxy)ethanol: ¹H NMR (400 MHz, CDCl₃) δ 7.51 (dd, *J* = 7.9 Hz, 1H), 7.45 (d, *J =* 7.9 Hz, 1H), 7.00 (s, 1H), 6.42 (d, *J=* 8.0 Hz, 1H), 4.42 - 4.28 (m, 2H), 3.97 - 3.85 (m, 2H), 1.51 (s, 9H).

### 2-(3,5-Difluoropyridin-4-yl)-N-(2,3-dihydrobenzo[b][1,4]dioxin-6-yl)-5-fluoroimidazo[1,2-a]pyridin-3-amine

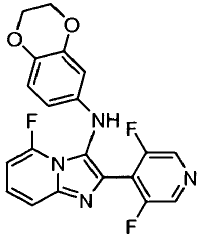

Prepared according to GP5 using 6-fluoropyridin-2-amine (7.5 mmol, 840 mg), 3,5-difluoroisonicotinaldehyde (9 mmol, 1290 mg) and 6-isocyano-2,3-dihydrobenzo[b]-[1,4]dioxine (9 mmol, 1449 mg). The compound was purified by flash chromatography (eluent: 20-50% EtOAc in heptane) to give the title compound (1.22 g, 34%). ¹H NMR (400 MHz, CDCl₃) δ 8.41 (s, 1H), 7.48 (dd, *J* = 0.8 and 9.1 Hz, 1H), 7.31 - 7.17 (m, 1H), 6.72 - 6.58 (m, 1H), 6.45 - 6.37 (m, 1H), 6.03 (dt, *J*= 2.4 and 2.9 Hz, 1H), 5.30 (s, 1H), 4.23 - 3.98 (m, 3H). LCMS *m*/*z* 399 [M+H]⁺, purity (UV/MS) 92/75.

### 2-(2-(3,5-difluoropyridin-4-yl)-3-(2,3-dihydrobenzo[b][1,4]dioxin-6-ylamino)imidazo[1,2-a]pyridin-5-yloxy)ethanol

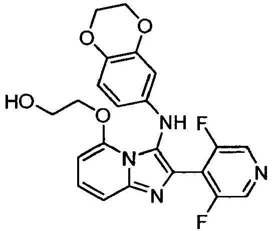

2-(3,5-difluoropyridin-4-yl)-N-(2,3-dihydrobenzo[b][1,4]dioxin-6-yl)-5-fluoroimidazo[1,2-a]pyridin-3-amine (638 mg, 1.60 mmol) was dissolved in THF (10 mL) and ethylene glycol (107 µL, 1.2 eq) and cooled to 0 °C under N₂ before NaH (92.3 mg, 1.92 mmol, ~50w% in oil) was added. Stirred 10 min at 0 °C the overnight at 60 °C. The reaction mixture was separated between brine and EtOAc, dried and concentrated. The crude mixture was purified flash chromatography (silica, 0-5% MeOH / DCM) to give 427 mg of material which by LCMS and ¹H-NMR was shown to be a ~1:1 mixture of product and a dimer. This mixture was used directly in the next step. LCMS *m*/*z* 441 [M+H]⁺, purity (UV/MS) 49/52.

### 3-Chloro-4-(3-(2,3-dihydrobenzo[b][1,4]dioxin-6-ylamino)-5-fluoroimidazo[1,2-a]pyridin-2-yl)-5-fluorophenol

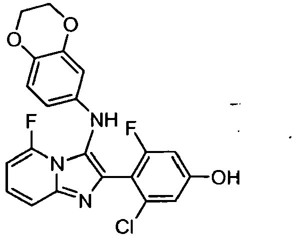

Prepared according to GP5 using 6-fluoropyridin-2-amine (0. 5 mmol, 56 mg), 2-chloro-6-fluoro-4-hydroxybenzaldehyde (0.60 mmol, 104 mg) and 6-isocyano-2,3-dihydrobenzo[b][1,4]dioxine (0.60 mmol, 97 mg). Purified by flash chromatography (eluent: 50% EtOAc in heptane) yielding the title compound (86 mg, 40%). ¹H NMR (400 MHz, CD₃OD) δ 7.40 - 7.28 (m, 2H), 6.74 (dd, *J* = 1.4, 2.3, 1H), 6.60 - 6.48 (m, 3H), 6.06 - 5.96 (m, 2H), 4.16 - 4.05 (m, 4H). LCMS *m*/*z* 430 [M+H]⁺, purity (UV/MS) 87/45.

### 2-(2-(4-(t-Butyldimethylsilyloxy)-2,6-dimethylphenyl)-3-(2,3-dihydrobenzo[b][1,4]dioxin-6-ylamino)imidazo[1,2-a]pyridin-5-yloxy)ethanol

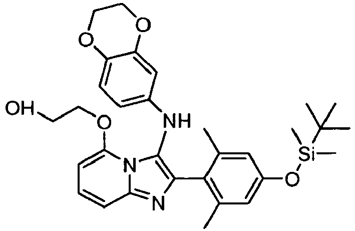

Prepared according to GP5 using 2-(6-aminopyridin-2-yloxy)ethanol (2.5 mmol, 385 mg), 4-(*t*-butyldimethylsilyloxy)-2,6-dimethylbenzaldehyde (3.0 mmol, 792 mg) and 6-isocyano-2,3-dihydrobenzo[b][1,4]dioxine (3.0 mmol, 483 mg). Purified by flash chromatography (eluent: 5% MeOH in CH₂Cl₂) yielding the title compound (473 mg, 34%). ¹H NMR (400 MHz, CD₃OD) δ 7.16 (t, *J* = 7.9, 1H), 6.38 (d, *J* = 8.6, 1H), 6.35 (s, 2H), 6.18 (d, *J* = 8.0, 1H), 5.92 (d, *J* = 8.0, 1H), 5.86 - 5.73 (m, 3 H), 4.02 - 3.96 (m, 2H), 3.95 - 3.86 (m, 6H), 1.91 (s, 6H), 0.80 (s, 9H), 0.04 (s, 6H).

### 2-(2-(4-(t-Butyldimethylsilyloxy)-2,6-dimethylphenyl)-3-(2,3-dihydrobenzo[b][1,4]dioxin-6-ylamino)imidazo[1,2-a]pyridin-5-yloxy)ethyl 4-methylbenzenesulfonate

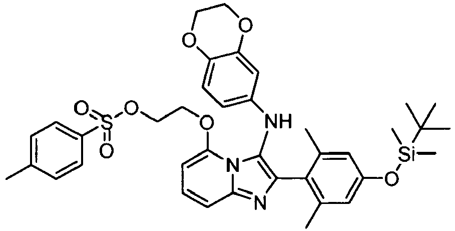

2-(2-(4-(*t*-Butyldimethylsilyloxy)-2,6-dimethylphenyl)-3-(2,3-dihydrobenzo[b][1,4]dioxin-6-ylamino)imidazo[1,2-a]pyridin-5-yloxy)ethanol (0.5 mmol, 280 mg) was taken up in DCM (10 mL), TEA (2.5 mmol, 252 mg) and tosyl chloride (0.6 mmol, 114 mg) was added and the reaction mixture left for 48h at rt. The crude was concentrated onto celite and purified by flash chromatography (eluent: 0-50% EtOAc in heptane) to yield the title compound (130 mg, 36%). ¹H NMR (400 MHz, CD₃OD) δ 7.59 (d, *J* = 7.5, 1H), 7.40 (d, *J* = 7.4, 1H), 7.15 (d, *J* = 8.0, 2H), 7.05 - 6.96 (m, 1H), 6.87 (t, *J* = 8.2, 1H), 6.34 (s, 3H), 5.87 - 5.60 (m, 3H), 4.09 - 3.73 (m, 8H), 1.95 (s, 6H), 0.79 (s, 9H), 0.02 (s, 6H). LCMS *m*/*z* 716 [M+H]⁺, purity (UV/MS) 95/61.

### 2-(2-(4-(t-Butyldimethylsilyloxy)-2,6-difluorophenyl)-3-(2,3-dihydrobenzo[b][1,4]dioxin-6-ylamino)imidazo[1,2-a]pyridin-5-yloxy)ethanol

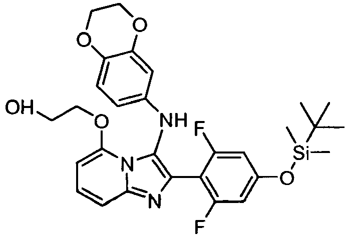

Prepared according to GP5 using 2-(6-aminopyridin-2-yloxy)ethanol (2.5 mmol, 385 mg), 4-(*t*-butyldimethylsilyloxy)-2,6-difluorobenzaldehyde (3.0 mmol, 816 mg) and 6-isocyano-2,3-dihydrobenzo[b][1,4]dioxine (3.0 mmol, 483 mg). Purified by flash chromatography (eluent: 0-50% EtOAc in heptane) yielding the title compound (309 mg, 22%). ¹H NMR (400 MHz, CD₃OD) δ 7.01 (dd, *J =* 0.8, 9.0, 1H), 6.89 (dd, *J =* 7.4, 9.0, 1H), 6.50 - 6.41 (m, 1H), 6.27 - 6.16 (m, 2H), 5.86 (dd, *J* = 2.5, 7.1, 2H), 5.71 (d, *J =* 7.4, 1 H), 5.23 (s, 1H), 4.00 - 3.87 (m, 4H), 3.87 - 3.79 (m, 2H), 3.50 - 3.36 (m, 2H), 0.75 (s, 9H), 0.03 (s, 6H). ¹³C NMR (100 MHz, CDCl₃) δ 162.8, 162.7, 160.4, 160.2, 157.5, 150.9, 145.6, 144.4, 142.6, 136.8, 131.9, 126.4, 121.5, 117.9, 110.8, 106.4, 104.3, 104.0, 102.3, 89.7, 71.4, 64.9, 64.3, 60.8, 25.7, 18.4, -4.2. LCMS *m*/*z* 570 [M+H]⁺, purity (UV/MS) 82/60.

### 2-(2-(4-(t-Butyldimethylsilyloxy)-2,6-difluorophenyl)-3-(2,3-dihydrobenzo[b][1,4]dioxin-6-ylamino)imidazo[1,2-a]pyridin-5-yloxy)ethyl 4-methylbenzenesulfonate

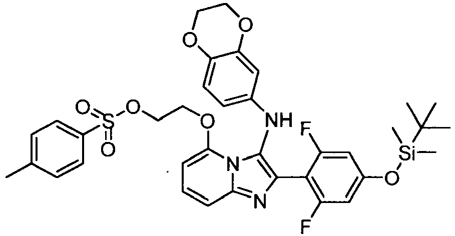

2-(2-(4-(*t*-Butyldimethylsilyloxy)-2,6-difluorophenyl)-3-(2,3-dihydrobenzo[b][1,4]dioxin-6-ylamino)imidazo[1,2-a]pyridin-5-yloxy)ethanol (0.54 mmol, 309 mg) was taken up in DCM (10 mL), TEA (2.7 mmol, 273 mg) and tosyl chloride (0.87 mmol, 165 mg) was added and the reaction mixture left for 48h at rt. The crude was concentrated onto celite and purified by flash chromatography (eluent: 50% EtOAc in heptane) to yield the title compound (209 mg, 54%). ¹H NMR (400 MHz, CD₃OD) δ 7.55 (d, *J* = 8.1, 2H), 7.06 (d, *J* = 8.1, 2H), 6.99 (d, *J* = 9.0, 1H), 6.89 - 6.77 (m, 1H), 6.35 - 6.28 (m, 1H), 6.27 - 6.16 (m, 2H), 5.75 - 5.67 (m, 2H), 5.64 (d, *J* = 9.5, 1H), 5.14 (s, 1H), 4.02 - 3.77 (m, 8H), 2.17 (s, 3H), 0.76 (s, 9H), 0.04 (s, 6H).

### Final compound examples, by MCR

### General procedure GP5

### 2-(2-chloro-6-fluorophenyl)-N-(2,3-dihydrobenzo[b][1,4]dioxin-6-yl)-5-ethylimidazo|1,2-a]pyridin-3-amine

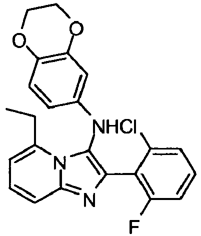

6-Ethylpyridin-2-amine (0.25 mmol, 30 mg), 2-fluoro-6-methoxybenzaldehyde (0.30 mmol, 47 mg) and 6-isocyano-2,3-dihydrobenzo[b][1,4]dioxine (0.30 mmol, 48 mg) were all weighed into a MW reaction vessel. 1,4-Dioxane (4 mL) and zinc chloride (0.02 mmol, 3 mg) were added and the vessel sealed. The reaction mixture was heated in a MW reactor at 140 °C for 20 min. The crude was run through a SCX ion exchange column and then adsorbed onto celite and purified by flash CC (eluent: 0-50 % EtOAc in heptane) to yield the title compound (98 mg, 93%). ¹H NMR (400 MHz, CDCl₃) δ 7.53 (d, *J* = 9.0 Hz, 1H), 7.26-7.21 (m, 2H), 7.17 (dd, *J* = 9.0, 6.9 Hz, 1H), 7.04-6.98 (m, 1H), 6.61-6.57 (m, 2H), 6.00-5.94 (m, 2H), 5.15 (s, 1H), 4.17-4.10 (m, 4H), 3.16 (q, *J* = 7.3 Hz, 2H), 1.29 (t, *J* = 7.3 Hz, 3H). ¹³C NMR (100 MHz, CDCl₃) δ (meaningful signals) 144.1, 142.0, 141.8, 130.1, 130.0, 125.2, 125.2, 125.0, 117.6, 116.2, 114.2, 114.0, 111.2, 106.6, 102.4, 64.5, 64.0, 24.3, 13.3. LCMS *m*/*z* 424 [M+H]⁺, purity (UV/MS) 93/51.

### 4-(3-(2,3-dihydrobenzo[b][1,4]dioxin-6-ylamino)-5-fluoroimidazo[1,2-a]pyridin-2-yl)-3,5-dimethylphenol

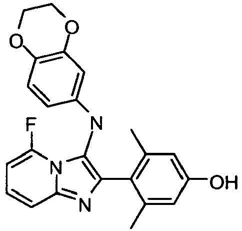

Prepared according to GP5 using 6-fluoropyridin-2-amine (1.0 mmol, 112 mg), 2,6-dimethyl-4-hydroxybenzaldehyde (1.2 mmol, 180 mg) and 6-isocyano-2,3-dihydrobenzo[b][1,4]dioxine (1.2 mmol, 193 mg). Purified by flash CC (eluent: 50 % EtOAc in heptane) yielding the title compound (198 mg, 49%). ¹H NMR (400 MHz, CD₃OD) δ 7.38-7.29 (m, 2H), 6.59-6.53 (m, 2H), 6.53-6.50 (m, 2H), 5.99 (dd, *J* = 8.6, 2.7 Hz, 1H), 5.94 (d, *J* = 2.7 Hz, 1H), 4.16-4.07 (m, 4H), 2.05 (s, 6H). ¹³C NMR (100 MHz, CD₃OD) δ (meaningful signals) 156.8, 143.9, 141.8, 140.3, 140.2, 139.2, 136.4, 126.2, 126.1, 123.1, 116.8, 113.6, 112.0, 106.3, 101.8, 93.3, 64.4, 63.8, 19.3. LCMS *m*/*z* 406 [M+H]⁺, purity (UV/MS) 94/67.

### 2-(2-chloro-6-fluorophenyl)-N-(2,3-dihydrobenzo[b][1,4]dioxin-6-yl)-6-fluoroimidazo[1,2-a]pyridin-3-amine

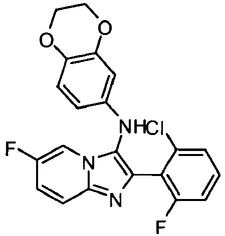

Prepared according to GP5 using 5-fluoropyridin-2-amine (0.50 mmol, 56 mg), 2-fluoro-6-methoxybenzaldehyde (0.60 mmol, 97 mg) and 6-isocyano-2,3-dihydrobenzo[b][1,4]dioxine (0.60 mmol, 98 mg). Purified by flash CC (eluent: 0-50 % EtOAc in heptane) yielding the title compound (110 mg, 53%). ¹H NMR (400 MHz, CDCl₃) δ 7.80-7.75 (m, 1H), 7.64 (ddd, *J* = 9.8, 4.9, 0.6 Hz, 1H), 7.31-7.25 (m, 2H), 7.19-7.12 (m, 1H), 7.09-7.02 (m, 1H), 6.70-6.64 (m, 1H), 6.10-6.03 (m, 2H), 5.42 (s, 1H), 4.21-4.13 (m, 4H). ¹³C NMR (100 MHz, CDCl₃) δ 163.3+160.8 (d, *J*= 250 Hz), 155.3+153.0 (d, *J* = 238 Hz), 145.1, 141.0, 139.0, 138.4, 136.3, 131.3, 131.2, 131.1, 126.3, 119.7, 119.7, 119.6, 118.7, 117.7, 115.2, 115.0, 110.8, 110.4, 108.1, 103.8, 65.4, 64.9. LCMS *m*/*z* 414 [M+H]⁺, purity (UV/MS) 98/70.

### 2-(3,5-difluoropyridin-4-yl)-N-(2,3-dihydrobenzo[b][1,4]dioxin-6-yl)-5-methylimidazo[1,2-a]pyridin-3-amine

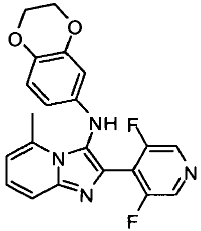

Prepared according to GP5 using 6-methylpyridin-2-amine (0.25 mmol, 27 mg), 3,5-difluoroisonicotinaldehyde (0.30 mmol, 43 mg) and 6-isocyano-2,3-dihydrobenzo[b][1,4]dioxine (0.30 mmol, 48 mg). Purified by flash CC (eluent: 0-100 % EtOAc in heptane) yielding the title compound (24 mg, 24%). ¹H NMR (400 MHz, CDCl₃) δ 8.43-8.35 (m, 2H), 7.52 (d, *J* = 9.1 Hz, 1H), 7.21-7.08 (m, 1H), 6.66-6.58 (m, 1H), 6.58-6.46 (m, 1H), 5.99-5.86 (m, 2H), 5.28 (s, 1H), 4.18-4.04 (m, 4H), 2.73 (s, 3H) ¹³C NMR (100 MHz, CDCl₃) δ 157.9+155.3 (d, *J* = 262 Hz), 144.2, 141.6, 137.0, 136.4, 134.6, 134.3, 125.9, 117.8, 116.3, 114.1, 113.1, 106.4, 105.5, 102.2, 64.5, 63.9, 19.0. LCMS *m*/*z* 395 [M+H]⁺, purity (UV/MS) 88/67.

### 4-(3-(2,3-dihydrobenzo[b][1,4]dioxin-6-ylamino)-5-methylimidazo[1,2-a]pyridin-2-yl)-3,5-difluorophenol

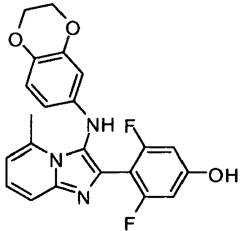

Prepared according to GP5 using 6-methylpyridin-2-amine (0.25 mmol, 27 mg), 2,6-difluoro-4-hydroxybenzaldehyde (0.30 mmol, 47 mg) and 6-isocyano-2,3-dihydrobenzo[b][1,4]dioxine (0.30 mmol, 48 mg). Purified by flash CC (eluent: 0-100 % EtOAc in heptane) yielding the title compound (61 mg, 60%). ¹H NMR (400 MHz, CDCl₃) δ 7.45 (d, *J* = 9.0 Hz, 1H), 7.10 (dd, *J* = 9.0, 6.9 Hz, 1H), 6.67-6.56 (m, 1H), 6.49 (d, *J* = 6.8 Hz, 1H), 6.42-6.31 (m, 2H), 6.03-5.87 (m, 2H), 5.25 (s, 1H), 4.63 (bs, 1H), 4.20-4.03 (m, 4H), 2.70 (s, 3H) ¹³C NMR (100 MHz, CDCl₃) δ 162.4+160.0 (d, *J* = 246 Hz), 162.3+159.9 (d, *J* = 246 Hz), 160.7+160.5+160.4 (t, *J* = 15 Hz), 144.1, 142.0, 138.6, 136.8, 136.5, 131.4, 125.9, 123.0, 117.7, 114.8, 113.9, 113.1, 106.7, 105.9, 102.4, 64.5, 63.9, 18.9. LCMS *m*/*z* 410 [M+H]⁺, purity (UV/MS) 93/45.

### 2-(2-chloro-6-fluorophenyl)-N-(2,3-dihydrobenzo[b][1,4]dioxin-6-yl)-6-methoxyimidazo[1,2-a]pyridin-3-amine

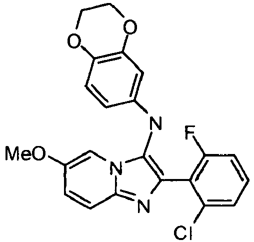

Prepared according to GP5 using 5-methoxypyridin-2-amine (0.25 mmol, 31 mg), 2,6-difluoro-4-hydroxybenzaldehyde (0.30 mmol, 47 mg) and 6-isocyano-2,3-dihydrobenzo[b][1,4]dioxine (0.30 mmol, 48 mg). Purified by flash CC (eluent: 0-50 % EtOAc in heptane) yielding the title compound (57 mg, 54%). ¹H NMR (400 MHz, CDCl₃) δ 7.40-7.24 (m, 3H), 7.14-7.08 (m, 2H), 6.52 (d, *J* = 8.6 Hz, 1H), 6.19 (d, *J* = 7.5 Hz, 1H), 6.03 (dd, *J* = 8.6, 2.7 Hz, 1H), 5.99 (d, *J* = 2.6 Hz, 1H), 5.34 (s, 1H), 4.13-4.04 (m, 4H), 3.80 (s, 3H) ¹³C NMR (100 MHz, CDCl₃) δ 166.5+164.0 (d, *J* = 250 Hz), 156.1, 148.7, 147.7, 146.7, 140.2, 139.6, 136.6, 134.3+134.2 (d, *J* = 9 Hz), 131.4, 129.8, 127.2, 125.4+125.2 (d, *J* = 20 Hz), 120.6, 117.7+117.4 (d, *J* = 23 Hz), 112.2, 110.6, 106.1, 93.0, 68.3, 67.8, 59.8. LCMS *m*/*z* 426 [M+H]⁺, purity (UV/MS) 96/94.

### 4-(3-(2,3-dihydrobenzo[b][1,4]dioxin-6-ylamino)-5-fluoroimidazo[1,2-a]pyridin-2-yl)-3,5-difluorophenol

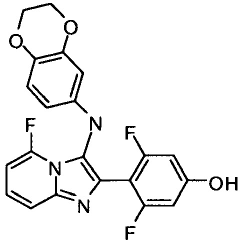

Prepared according to GP5 using 6-fluoropyridin-2-amine (1.0 mmol, 112 mg), 2,6-difluoro-4-hydroxybenzaldehyde (1.2 mmol, 190 mg) and 6-isocyano-2,3-dihydrobenzo[b][1,4]dioxine (1.2 mmol, 196 mg). Purified by flash CC (eluent: 50 % EtOAc in heptane) yielding the title compound (154 mg, 37%).

¹H NMR (400 MHz, CD₃OD) δ 7.45-7.23 (m, 2H), 6.63-6.48 (m, 2H), 6.48-6.38 (m, 2H), 5.99 (dd, *J* = 8.7, 2.6 Hz, 1H), 5.95 (d, *J* = 2.5 Hz, 1H), 4.13-4.05 (m, 4H). ¹³C NMR (100 MHz, CD₃OD) δ 164.3+161.8 (d, *J* = 247 Hz), 164.2+161.7 (d, *J* = 247 Hz), 161.6+161.4+161.3 (d, *J* = 15 Hz), 152.9+150.3 (d, *J* = 269 Hz), 146.2, 145.4, 142.9, 138.0, 132.4, 128.1, 123.9, 118.3, 113.6, 107.8, 103.3, 100.2+99.9 (d, *J* = 28 Hz), 95.0+94.8 (d, *J* = 17 Hz), 65.8, 65.3. LCMS *m*/*z* 414 [M+H]⁺, purity (UV/MS) 97/60.

### 2-(3,5-dichloropyridin-4-yl)-N-(2,3-dihydrobenzo[b][1,4]dioxin-6-yl)-6-methoxyimidazo[1,2-a]pyridin-3-amine

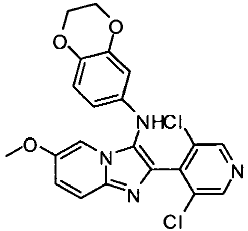

Prepared according to GP5 using 5-methoxypyridin-2-amine (0.25 mmol, 31 mg), 3,5-dichloro-4-pyridinecarboxaldehyde (0.30 mmol, 53 mg) and 6-isocyano-2,3-dihydrobenzo[b][1,4]dioxine (0.30 mmol, 48 mg). Purified by prep. TLC (eluent 5 % methanol in heptane) yielding the title compound (3 mg, 3%). ¹H NMR (400 MHz, CDCl₃) δ 8.46 (s, 1H), 7.49 (dd, *J* = 9.7, 0.7 Hz, 1H), 7.32 (dd, *J =* 2.3, 0.7 Hz, 1H), 7.01 (dd, *J* = 9.7, 2.4 Hz, 1H), 6.63-6.53 (m, 1H), 6.03-5.94 (m, 2H), 5.10 (s, 1H), 4.14-4.03 (m, 4H), 3.69 (s, 3H). LCMS *m*/*z* 414 [M+H]⁺, purity (UV/MS) 91/60.

### 3-(3-(2,3-dihydrobenzo[b][1,4]dioxin-6-ylamino)-5-methoxyimidazo[1,2-a]pyridin-2-yl)-2,4-difluorophenol

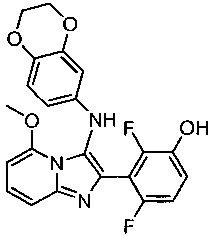

Prepared according to GP5 using 6-methoxypyridin-2-amine (2.5 mmol, 310mg), 2,6-difluoro-3-hydroxybenzaldehyde (3.0 mmol, 474 mg) and 6-isocyano-2,3-dihydro-benzo[b][1,4]dioxine (3.0 mmol, 483 mg). The title compound precipitated out of the crude reaction mixture, it was collected by filtration and subsequently washed with diethyl ether (243 mg, 23%). ¹H NMR (400 MHz, CD₃OD) δ 7.45 - 7.28 (m, 2H), 6.97 - 6.82 (m, 1H), 6.76 (t, *J* = 8.8, 1H), 6.53 (d, *J* = 8.7, 1H), 6.35 - 6.23 (m, 1H), 6.00 (dd, *J* = 2.6, 8.6, 1H), 5.94 (d, *J* = 2.6, 1H), 4.22 - 3.98 (m, 4H), 3.80 (s, 3H). ¹³C NMR (100 MHz, CD₃OD) δ (meaningful signals) 152.2, 145.1, 143.8, 142.3, 136.4, 123.9, 117.5, 116.7, 110.0, 109.8, 107.9, 106.7, 102.2, 64.4, 63.8, 56.2. LCMS *m*/*z* 426 [M+H]⁺, purity (UV/MS) 99/98.

### 2-Chloro-3-(3-(2,3-dihydrobenzo[b][1,4]dioxin-6-ylamino)-5-methoxyimidazo[1,2-a]pyridin-2-yl)-4-fluorophenol

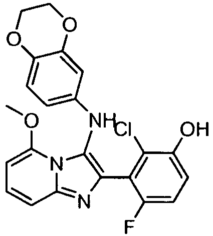

Prepared according to GP5 using 6-methoxypyridin-2-amine (1.7 mmol, 210 mg), 2-chloro-6-fluoro-3-hydroxybenzaldehyde (2.0 mmol, 348 mg) and 6-isocyano-2,3-dihydro-benzo[b][1,4]dioxine (2.0 mmol, 322 mg). The title compound precipitated out of the crude reaction mixture, it was collected by filtration and subsequently washed with methanol (232 mg, 30%). ¹H NMR (400 MHz, DMSO) δ 10.07 (s, 1H), 7.32 - 7.21 (m, 1H), 7.21 - 7.13 (m, 2H), 7.04 (t, *J* = 8.8, 1H), 7.00 - 6.92 (m, 1H), 6.51 (d, *J* = 8.6, 1H), 6.24 (d, *J* = 7.4, 1H), 5.99 (dd, *J* = 2.6, 8.7, 1H), 5.90 (d, *J* = 2.6, 1H), 4.17 - 3.94 (m, 4H), 3.77 (s, 3H). ¹³C NMR (100 MHz, CD₃OD) δ (meaningful signals) 153.5, 152.3, 149.6, 144.9, 143.8, 136.4, 116.7, 113.7, 113.5, 106.9, 102.4, 64.4, 63.8, 56.1. LCMS *m*/*z* 442 [M+H]⁺, purity (UV/MS) 97/50.

### General Procedure GP6

The amino-pyridine (0.53mmol), isonitrile (0.53mmol), aldehyde (0.53mmol) and ZnCl₂ (10%) were mixed in a MW reaction vessel and dissolved /suspended in 1,4-dioxane (4 mL). The vessel was capped and heated to 140 °C for 20 min. The solvent was evaporated and the crude product was purified by flash CC (2-5% MeOH in CH₂Cl₂). In some cases subsequent purification by pTLC (2-5% MeOH in CH₂Cl₂) was required.

### 2-(2-chloro-6-fluorophenyl)-N-(2,3-dihydrobenzo[b][1,4]dioxin-6-yl)imidazo[1,2-a]pyridin-3-amine

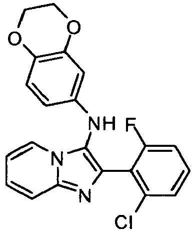

Prepared according to GP6 by using 2-Aminopyridine (30.0 mg, 0.32 mmol), 2-chloro-6-fluorobenzaldehyde (50.0 mg, 0.32 mmol), 2,3-dihydro-6-isocyano-1,4-benzodioxine (51.8 mg, 0.32 mmol), zinc chloride (4.4 mg, 0.03 mmol) in 1,4-dioxane (4.0 mL) to give the title compound (28.6mg, 23%). ¹H NMR (CDCl₃, 400 MHz) δ 7.92-7.91 (m, 1H, ArH), 7.80-7.78 (m, 1H, ArH), 7.27-7.26 (m, 3H, ArH), 7.10-7.06 (m, 1H, ArH), 6.90-6.89 (m, 1H, ArH), 6.66-6.63 (m, 1H, ArH), 6.06-6.05 (m, 2H, ArH), 5.54 (br s, 1H, NH), 4.16-4.11 (m, 4H, CH₂CH₂), 2.04 (s, 3H, CH₃). LCMS *m*/*z* 396 [M+H]⁺, purity (UV/MS) 100/98.

### 2-(2-chloro-6-fluorophenyl)-N-(2,3-dihydrobenzo[b][1,4]dioxin-6-yl)-7-methylimidazo[1,2-a]pyridin-3-amine

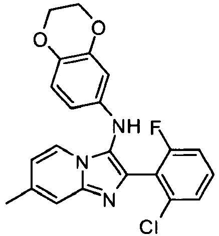

Prepared according to GP6 by using 2-amino-4-methylpyridine (34.6 mg, 0.32 mmol), 2-chloro-6-fluorobenzaldehyde (50.0 mg, 0.32 mmol), 2,3-dihydro-6-isocyano-1,4-benzodioxine (51.8 mg, 0.32 mmol), zinc chloride (4.4 mg, 0.03 mmol) in 1,4-dioxane (4.0 mL) to give the title compound (23.6mg, 18%). ¹H NMR (CDCl₃, 400 MHz) δ 7.54-7.53 (m, 1H, ArH), 7.05-7.03 (m, 3H, ArH), 6.80-6.82 (m, 1H, ArH), 6.46-6.42 (m, 2H, ArH), 5.85-5.84 (m, 2H, ArH), 5.19 (br s, 1H, NH), 3.94-3.93 (m, 4H, CH₂CH₂), 2.06 (s, 3H, CH₃). LCMS *m*/*z* 410 [M+H]⁺, purity (UV/MS) 97/93.

### 2-(2-chloro-6-fluorophenyl)-N-(2,3-dihydrobenzo[b][1,4]dioxin-6-yl)-8-methylimidazo[1,2-a]pyridin-3-amine

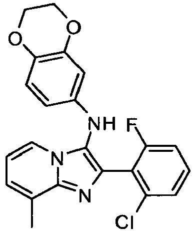

Prepared according to GP6 by using 2-amino-4-methylpyridine (34.6 mg, 0.32 mmol), 2-chloro-6-fluorobenzaldehyde (50.0 mg, 0.32 mmol), 2,3-dihydro-6-isocyano-1,4-benzodioxine (51.8 mg, 0.32 mmol), zinc chloride (4.4 mg, 0.03 mmol) in 1,4-dioxane (4.0 mL) to give the title compound (14.1mg, 11%). ¹H NMR (CDCl₃, 400 MHz) δ 7.78-7.76 (m, 1H, ArH), 7.28-7.25 (m, 2H, ArH), 7.10-7.04 (m, 2H, ArH), 6.78-6.77 (m, 1H, ArH), 6.65-6.63 (m, 1H, ArH), 6.07-6.06 (m, 2H, ArH), 5.43 (br s, 1H, NH), 4.15-4.11 (m, 4H, CH₂CH₂), 2.04 (s, 3H, CH₃). LCMS *m*/*z* 410 [M+H]⁺, purity (UV/MS) 99/84.

### 2-(2,6-difluoro-4-(2-fluoroethoxy)phenyl)-N-(2,3-dihydrobenzo[b][1,4]dioxin-6-yl)-5-methylimidazo[1,2-a]pyridin-3-amine

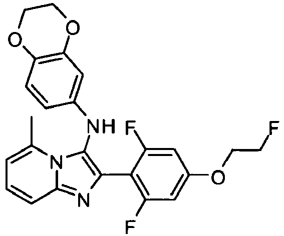

Prepared according to GP6 by using 2-amino-6-methylpyridine (58.0 mg; 0.53 mmol), 2,3-dihydro-6-isocyano-1,4-benzodioxine (86.0 mg, 0.53 mmol), 2,6-difluoro-4-(2-fluoroethoxy) benzaldehyde (108.0 mg, 0.53 mmol), zinc chloride (7.0 mg, 0.05 mmol) in 1,4-dioxane (8.0 mL) to give the title compound (7.5mg, 4%). ¹H NMR (CH₃OD, 400 MHz) δ 7.41-7.39 (m, 1H, ArH), 7.24-7.22 (m, 1H, ArH), 6.65-6.62 (m, 3H, ArH), 6.54-6.52 (m, 1H, ArH), 5.89-6.84 (m, 2H, ArH), 4.76-4.74 (m, 1H, CH₂), 4.64-4.62 (m, 1H, CH₂), 4.27-4.25 (m, 1H, CH₂), 4.20-4.18 (m, 1H, CH₂), 4.12-4.10 (m, 4H, CH₂CH₂), 2.04 (s, 3H, CH₃). LCMS *m*/*z* 456 [M+H]⁺, purity (UV/MS) 98/67.

### 5-(3-(2,3-dihydrobenzo[b][1,4]dioxin-6-ylamino)-5-methylimidazo[1,2-a]pyridin-2-yl)-2-(2-fluoroethoxy)phenol

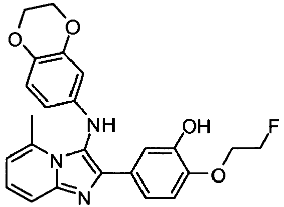

Prepared according to GP6 by using 2-amino-6-methylpyridine (58.0 mg, 0.53 mmol), 2,3-dihydro-6-isocyano-1,4-benzodioxine (86.0 mg, 0.53 mmol), 4-(2-fluoroethoxy)-3-hydroxybenzaldehyde (98.0 mg, 0.53 mmol), zinc chloride (7.0 mg, 0.05 mmol) in 1,4-dioxane (8.0 mL) to give the title compound (30.8mg, 14%). ¹H NMR (CH₃OD, 400 MHz) δ 7.50-7.49 (m, 1H, ArH), 7.42-7.36 (m, 2H, ArH), 7.20-7.16 (m, 1H, ArH), 6.92-6.90 (m, 1H, ArH), 6.64-6.62 (m, 1H, ArH), 6.58-6.56 (m, 1H, ArH), 5.97-5.95 (m, 1H, ArH), 5.91-5.90 (m, 1H, ArH), 4.76-4.74 (m, 1H, CH₂), 4.67-4.66 (m, 1H, CH₂), 4.29-4.27 (m, 1H, CH₂), 4.21-4.19 (m, 1H, CH₂), 4.13-4.07 (m, 4H, CH₂CH₂), 2.00 (s, 3H, CH₃). LCMS *m*/*z* 436 [M+H]⁺, purity (UV/MS) 100/78. LCMS *m*/*z* 403 [M+H]⁺, purity (UV/MS) 97/74.

### N-(2,3-dihydrobenzo[b][1,4]dioxin-6-yl)-2-(4-(2-fluoroethoxy)-3-methoxyphenyl)-5-methylimidazo[1,2-a]pyridin-3-amine

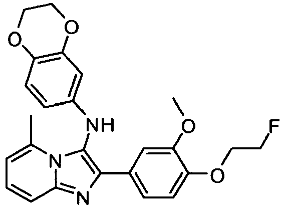

Prepared according to GP6 by using 2-amino-6-methylpyridine (58.0 mg, 0.53 mmol), 2,3-dihydro-6-isocyano-1,4-benzodioxine (86.0 mg, 0.53 mmol), 4-(2-fluoroethoxy)-3-methoxy benzaldehyde (98.0 mg, 0.53 mmol), zinc chloride (7.0 mg, 0.05 mmol) in 1,4-dioxane (8.0 mL) to give the title compound (58.8mg, 25%). ¹H NMR (CH₃OD, 400 MHz) δ 7.58-7.50 (m, 3H, ArH), 7.14-7.10 (m, 1H, ArH), 6.81-6.80 (m, 1H, ArH), 6.73-6.71 (m, 1H, ArH), 6.50-6.48 (m, 1H, ArH), 6.16-6.15 (m, 1H, ArH), 6.00 (br s, 1H, ArH), 4.82-4.81 (m, 1H, CH₂), 4.71-4.69 (m, 1H, CH₂), 4.27-4.26 (m, 1H, CH₂), 4.20-4.15 (m, 5H, CH₂), 3.75 (s, 3H, OCH₃), 2.75 (s, 3H, CH₃). LCMS *m*/*z* 450 [M+H]⁺, purity (UV/MS) 94/41.

### 2-(2-chloro-6-fluorophenyl)-N-(2,3-dihydrobenzo[b][1,4]dioxin-6-yl)-5-(trifluoromethyl)imidazo [1,2-a]pyridin-3-amine

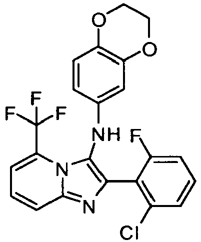

Prepared according to GP6 by using 2-Amino-6-(trifluoromethyl)pyridine (86.0 mg, 0.53 mmol), 2,3-dihydro-6-isocyano-1,4-benzodioxine (86.0 mg, 0.53 mmol), 2-chloro-6-fluorobenzaldehyde (84.0 mg, 0.53 mmol), zinc chloride (7.0 mg, 0.05 mmol) in 1,4-dioxane (5.0 mL) to give the title compound (4.7 mg, 2%). ¹H NMR (CH₃OD, 400 MHz) δ 7.76-7.75 (m, 2H, ArH), 7.36-7.32 (m, 2H, ArH), 7.08-7.04 (m, 2H, ArH), 6.54-6.52 (m, 1H, ArH), 6.06-6.05 (m, 1H, ArH), 6.01-5.99 (m, 1H, ArH), 4.15-3.95 (m, 4H, CH₂). LCMS *m*/*z* 464 [M+H]⁺, purity (UV/MS) 86/64.

### 4-(3-(2,3-dihydrobenzo[b][1,4]dioxin-6-ylamino)-5-methylimidazo[1,2-a]pyridin-2-yl)-3,5-difluorobenzonitrile

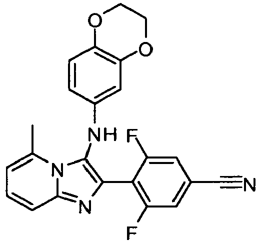

Prepared according to GP6 by using 2-amino-6-methylpyridine (57.0 mg, 0.53 mmol), 2,3-dihydro-6-isocyano-1,4-benzodioxine (86.0 mg, 0.53 mmol), 3,5-difluoro-4-formylbenzonitrile (89.0 mg, 0.53 mmol), zinc chloride (7.0 mg, 0.05 mmol) in 1,4-dioxane (5.0 mL) to give the title compound (4.2 mg, 2%). LCMS *m*/*z* 419 [M+H]⁺, purity (UV/MS) 99/87.

### 2-(2,6-difluoro-4-(2-fluoroethoxy)phenyl)-N-(2,3-dihydrobenzo[b][4,1]dioxin-6-yl)-5-methylimidazo[1,2-a]pyridin-3-amine

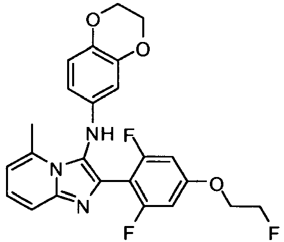

Prepared according to GP6 by using 2-amino-6-methylpyridine (58.0 mg, 0.53 mmol), 2,3-dihydro-6-isocyano-1,4-benzodioxine (86.0 mg, 0.53 mmol), 2,6-difluoro-4-(2-fluoroethoxy) benzaldehyde (58.0 mg, 0.53 mmol), zinc chloride (7.0 mg, 0.05 mmol) in 1,4-dioxane (8.0 mL) to give the title compound (169.0 mg, 70%). ¹H NMR (CH₃OD, 400 MHz) δ 7.65-7.62 (br s, 1H, NH), 7.33-7.29 (m, 1H, ArH), 6.73-6.72 (m, 1H, ArH), 6.59-6.57 (m, 2H, ArH), 6.55-6.52 (m, 1H, ArH), 5.89-5.85 (m, 3H, ArH), 4.76-4.74 (m, 1H, CH₂), 4.64-4.63 (m, 1H, CH₂), 4.24-4.23 (m, 1H, CH₂), 4.18-4.16 (m, 1H, CH₂), 4.12-4.07 (m, 4H, CH₂CH₂), 2.74 (s, 3H, CH₃). LCMS *m*/*z* 456 [M+H]⁺, purity (UV/MS) 100/80.

### 2-(2,6-difluoro-4-(2-fluoroethoxy)phenyl)-N-(2,3-dihydrobenzo[b][1,4]dioxin-6-yl)-5-methoxyimidazo[1,2-a]pyridin-3-amine

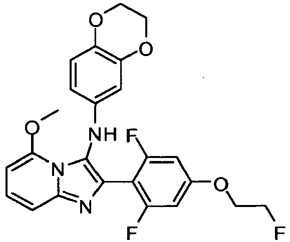

Prepared according to GP6 by using 6-methoxy-pyridin-2-ylamine (66.0 mg, 0.53 mmol), 2,3-dihydro-6-isocyano-1,4-benzodioxine (86.0 mg, 0.53 mmol), 2,6-difluoro-4-(2-fluoroethoxy) benzaldehyde (108.0 mg, 0.53 mmol), zinc chloride (7.0 mg, 0.05 mmol) in 1,4-dioxane (8.0 mL) to give the title compound (72.0 mg, 29%). ¹H NMR (CH₃OD, 400 MHz) δ 7.26-7.24 (m, 1H, ArH), 7.10-7.09 (m, 1H, ArH), 6.65-6.63 (m, 2H, ArH), 6.53-6.51 (m, 1H, ArH), 6.17-6.15 (m, 1H, ArH), 5.98-5.96 (m, 1H, ArH), 5.91-5.90 (m, 1H, ArH), 4.76-4.74 (m, 1H, CH₂), 4.64-4.62 (m, 1H, CH₂), 4.26-4.24 (m, 1H, CH₂), 4.19-4.17 (m, 1H, CH₂), 4.10-4.06 (m, 4H, CH₂CH₂), 3.78 (s, 3H, OCH₃). LCMS *m*/*z* 472 [M+H]⁺, purity (UV/MS) 90/83.

### 4-(3-(2,3-dihydrobenzo[b][1,4]dioxin-6-ylamino)-5-methoxyimidazo[1,2-a]pyridin-2-yl)-3,5-difluorophenol

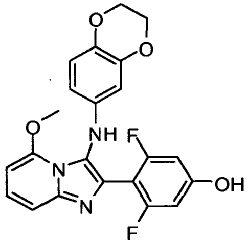

Prepared according to GP6 by using 6-methoxy-pyridin-2-ylamine (66.0 mg, 0.53 mmol), 2,3-dihydro-6-isocyano-1,4-benzodioxine (86.0 mg, 0.53 mmol), 2,6-difluoro-4-hydroxy benzaldehyde (84.0 mg, 0.53 mmol), zinc chloride (7.0 mg, 0.05 mmol) in 1,4-dioxane (8.0 mL) to give the title compound (72.0 mg, 29%). ¹H NMR (CH₃OD, 400 MHz) δ 7.30-7.20 (m, 1H, ArH), 7.12-7.07 (m, 1H, ArH), 6.54-6.50 (m, 1H, ArH), 6.44-6.38 (m, 2H, ArH), 6.20-6.16 (m, 1H, ArH), 6.00-5.96 (m, 1H, ArH), 5.93-5.90 (m, 1H, ArH), 4.10-4.07 (m, 4H, CH₂CH₂), 3.79 (s, 3H, OCH₃). LCMS *m*/*z* 426 [M+H]⁺, purity (UV/MS) 100/74.

### 2-(2,6-dichlorophenyl)-N-(2,3-dihydrobenzo[b][1,4]dioxin-6-yl)-5-methoxyimidazo[1,2-a]pyridin-3-amine

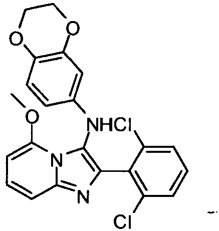

Prepared according to GP6 by using 6-methoxy-pyridin-2-ylamine (66.0 mg, 0.53 mmol), 2,3-dihydro-6-isocyano-1,4-benzodioxine (86.0 mg, 0.53 mmol), 2,6-dichlorobenzaldehyde (93.0 mg, 0.53 mmol), zinc chloride (7.0 mg, 0.05 mmol) in 1,4-dioxane (8.0 mL) to give the title compound (67.0 mg, 29%). ¹H NMR (CDCl₃, 400 MHz) δ 7.35-7.32 (m, 3H, ArH), 7.26-7.19 (m, 2H, ArH), 6.62-6.60 (m, 1H, ArH), 6.14-6.10 (m, 2H, ArH), 6.04-6.02 (m, 1H, ArH), 4.16-4.12 (m, 4H, CH₂CH₂), 3.78 (s, 3H, OCH₃). LCMS *m*/*z* 442 [M+H]⁺, purity (UV/MS) 96/80.

### 2-(2,6-difluorophenyl)-N-(2,3-dihydrobenzo[b][1,4]dioxin-6-yl)-5-methoxyimidazo[1,2-a]pyridin-3-amine

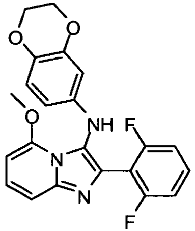

Prepared according to GP6 by using 6-methoxy-pyridin-2-ylamine (66.0 mg, 0.53 mmol), 2,3-dihydro-6-isocyano-1,4-benzodioxine (86.0 mg, 0.53 mmol), 2,6-difluorobenzaldehyde (76.0 mg, 0.53 mmol), zinc chloride (7.0 mg, 0.05 mmol) in 1,4-dioxane (8.0 mL) to give the title compound (18.6 mg, 9%). ¹H NMR (CDCl₃, 400 MHz) δ 7.42-7.30 (m, 2H, ArH), 6.99-6.95 (m, 2H, ArH), 6.64-6.61 (m, 1H, ArH), 6.38-6.36 (m, 1H, ArH), 6.21-6.18 (m, 1H, ArH), 6.05-6.03 (m, 2H, ArH), 4.16―4.12 (m, 4H, CH₂CH₂), 3.78 (s, 3H, OCH₃). LCMS *m*/*z* 410 [M+H]⁺, purity (UV/MS) 95/84.

### 2-(3,5-difluoropyridin-4-yl)-N-(2,3-dihydrobenzo[b][1,4]dioxin-6-yl)-5-methoxyimidazo[1,2-a]pyridin-3-amine

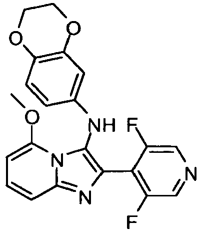

Prepared according to GP6 by using 6-methoxy-pyridin-2-ylamine (66.0 mg, 0.53 mmol), 2,3-dihydro-6-isocyano-1,4-benzodioxine (86.0 mg, 0.53 mmol), 3,5-difluoro-4-formyl pyridine (76.0 mg, 0.53 mmol), zinc chloride (7.0 mg, 0.05 mmol) in 1,4-dioxane (8.0 mL) to give the title compound (67.0 mg, 29%). ¹H NMR (CDCl₃, 400 MHz) δ 7.61-7.59 (m, 1H, ArH), 7.44-7.42 (m, 1H, ArH), 6.63-6.61 (m, 1H, ArH), 6.22-6.19 (m, 2H, ArH), 6.06-6.05 (m, 3H, ArH), 5.64-5.63 (m, 1H, ArH), 4.16-4.13 (m, 1H, CH₂CH₂), 3.88 (s, 3H, OCH₃). LCMS *m*/*z* 411 [M+H]⁺, purity (UV/MS) 87/60.

### N-(2,3-dihydrobenzo[b][1,4]dioxin-6-yl)-2-(4-(2-fluoroethoxy)-2,6-dimethylphenyl)-5-methoxyimidazo[1,2-a]pyridin-3-amine

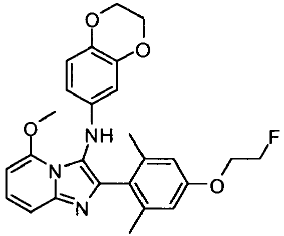

Prepared according to GP6 by using 6-methoxy-pyridin-2-ylamine (66.0 mg, 0.53 mmol), 2,3-dihydro-6-isocyano-1,4-benzodioxine (86.0 mg, 0.53 mmol), 4-(2-fluoroethoxy)-2,6-dimethylbenzaldehyde (104.0 mg, 0.53 mmol), zinc chloride (7.0 mg, 0.05 mmol) in 1,4-dioxane (8.0 mL) to give the title compound (72.0 mg, 29%). ¹H NMR (CH₃OD, 400 MHz) δ 7.53-7.47 (m, 1H, ArH), 6.62-6.60 (m, 3H, ArH), 6.32-6.30 (m, 1H, ArH), 6.05-6.01 (m, 3H, ArH), 5.44 (br s, 1H, NH), 4.79-4.77 (m, 1H, CH₂), 4.67-4.65 (m, 1H, CH₂), 4.26-4.24 (m, 1H, CH₂), 4.15-4.10 (m, 5H, CH₂(alkyl tail)+CH₂CH₂), 3.47 (s, 3H, OCH₃), 3.47 (s, 6H, 2xCH₃). LCMS *m*/*z* 472 [M+H]⁺, purity (UV/MS) 85/53.

### 2-(2,6-dichlorophenyl)-N-(2,3-dihydrobenzo[b][1,4]dioxin-6-yl)-5-methoxyimidazo[1,2-a]pyridin-3-amine

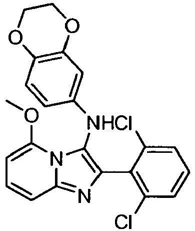

Prepared according to GP6 by using 6-Methoxy-pyridin-2-ylamine (66.0 mg, 0.53 mmol), 3,5-dichloro-4-pyridinecarboxaldehyde (94.0 mg, 0.53 mmol), 2,3-dihydro-6-isocyano-1,4-benzodioxine (86.0 mg, 0.53 mmol), zinc chloride (7.0 mg, 0.05 mmol) in 1,4-dioxane (8.0 mL) to give the title compound (57.7mg, 0.13 mmol, 24%). ¹H NMR (CDCl₃, 400 MHz) δ 8.54-8.53 (m, 2H, ArH), 7.31-7.30 (m, 1H, ArH), 7.14-7.13 (m, 1H, ArH), 6.52-6.51 (m, 1H, ArH), 6.21-6.22 (m, 1H, ArH), 6.10-6.03 (m, 3H, ArH), 5.47 (br s, 1H, NH), 4.10-4.06 (m, 4H, CH₂CH₂), 3.83 (s, 3H, OCH₃). LCMS *m*/*z* 396 [M+H]⁺, purity (UV/MS) 89/60.

### 2-(2,6-difluoro-3-(fluoromethoxy)phenyl)-N-(2,3-dihydrobenzo[b][1,4]dioxin-6-yl)-5-methoxyimidazo[1,2-a]pyridin-3-amine

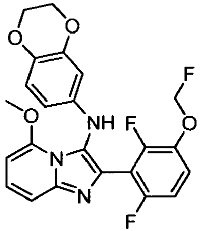

Prepared according to GP6 by using 6-Methoxy-pyridin-2-ylamine (200.0 mg, 1.61 mmol), 2,6-difluoro-3-(fluoromethoxy)benzaldehyde (306.0 mg, 1.61 mmol), 2,3-dihydro-6-isocyano-1,4-benzodioxine (260.0 mg, 1.61 mmol), zinc chloride (22.0 mg, 0.16 mmol) in 1,4-dioxane (8.0 mL) to give the title compound (106.8mg, 0.23 mmol, 15%). ¹H NMR (CDCl₃, 400 MHz) δ 7.30-7.25 (m, 2H, ArH), 7.13-7.10 (m, 1H, ArH), 6.97-6.96 (m, 1H, ArH), 6.53-6.51 (m, 1H, ArH), 6.19-6.17 (m, 1H, ArH), 6.00-5.97 (m, 1H, ArH), 5.93-5.92 (m, 1H, ArH), 5.75 (s, 1H, CH₂F), 5.61 (s, 1H, CH₂F), 4.11-4.06 (m, 4H, CH₂CH₂), 3.80 (s, 3H, OCH₃). LCMS *m*/*z* 396 [M+H]⁺, purity (UV/MS) 93/70.

### 2-(2,6-difluoro-4-(fluoromethoxy)phenyl-N-(2,3-dihydrobenzo[b][1,4]dioxin-6-yl)-5-methoxyimidazo[1,2-a]pyridin-3-amine

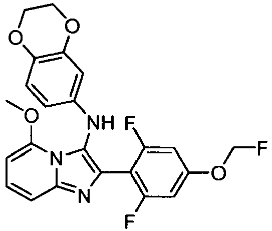

Prepared according to GP6 by using 6-Methoxy-pyridin-2-ylamine (100.0 mg, 0.81 mmol), 2,6-difluoro-4-(fluoromethoxy)benzaldehyde (153.0 mg, 0.80 mmol), 2,3-dihydro-6-isocyano-1,4-benzodioxine (130.0 mg, 0.81 mmol), zinc chloride (11.0 mg, 0.08 mmol) in 1,4-dioxane (4.0 mL) to give the title compound (69.6 mg, 0.15 mmol, 19%). ¹H NMR (CDCl₃, 400 MHz) δ 7.23-7.21 (m, 1H, ArH), 7.09-7.07 (m, 1H, ArH), 6.78-6.75 (m, 2H, ArH), 6.52-6.50 (m, 1H, ArH), 6.13-6.11 (m, 1H, ArH), 5.99-5.96 (m, 1H, ArH), 5.92-5.90 (m, 1H, ArH), 5.79 (s, 1H, CH₂F), 5.66 (s, 1H, CH₂F), 4.10-4.04 (m, 4H, CH₂CH₂), 3.33 (s, 3H, OCH₃). LCMS *m*/*z* 396 [M+H]⁺, purity (UV/MS) 95/85.

### General Procedure GP7

The amine (0.53mmol), isonitrile (0.53mmol), aldehyde (0.53mmol) and InCl₃ (10%) were suspended in dry toluene. The reaction mixture was shaken at 110°C for 72 h. The solvent was evaporated and the crude product was purified by flash CC (2-5% MeOH in CH₂Cl₂). In some cases subsequent purification by pTLC (2-5% MeOH in CH₂Cl₂) was required.

### 2-(2-chloro-6-fluorophenyl)-N-(2,3-dihydrobenzo[b][1,4]dioxin-6-yl)imidazo[1,2-a]pyrimidin-3-amine

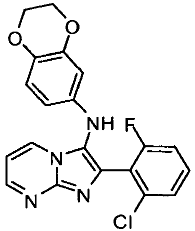

Prepared according to GP7 by using 2-aminopyrimidine (50.0 mg, 0.53 mmol), 2,3-dihydro-6-isocyano-1,4-benzodioxine (86.0 mg, 0.53 mmol), 2-chloro-6-fluorobenzaldehyde (84.0 mg, 0.53 mmol), indium chloride (10.0 mg, 0.04 mmol) in toluene (8.0 mL) to give the title compound (47.9 mg, 23%). ¹H NMR (CH₃OD, 400 MHz) δ 8.61-8.60 (m, 1H, ArH), 8.38-8.36 (m, 1H, ArH), 7.40-7.38 (m, 1H, ArH), 7.32-7.31 (m, 1H, ArH), 7.08-7.07 (m, 1H, ArH), 7.06-7.05 (m, 1H, ArH), 6.56-6.54 (m, 1H, ArH), 6.06-6.04 (m, 2H, ArH), 4.12-4.07 (m, 4H, CH₂CH₂). LCMS *m*/*z* 397 [M+H]⁺, purity (UV/MS) 95/71.

### 2-(2-chloro-6-fluorophenyl)-N-(2,3-dihydrobenzo[b][1,4]dioxin-6-yl)imidazo[1,2-a]pyrazin-3-amine

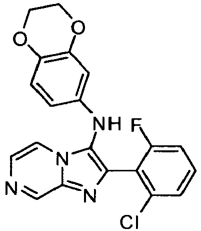

Prepared according to GP7 by using aminopyrazine (50.0 mg, 0.53 mmol), 2,3-dihydro-6-isocyano-1,4-benzodioxine (86.0 mg, 0.53 mmol), 2-chloro-6-fluorobenzaldehyde (84.0 mg, 0.53 mmol), indium chloride (10.0 mg, 0.04 mmol) in toluene (8.0 mL) to give the title compound (22.5 mg, 11%). ¹H NMR (CH₃OD, 400 MHz) δ 8.99-8.98 (s, 1H, ArH), 8.01-8.00 (m, 1H, ArH), 7.89-7.88 (m, 1H, ArH), 7.40-7.37 (m, 1H, ArH), 7.32-7.30 (m, 1H, ArH), 7.14-7.13 (m, 1H, ArH), 6.55-6.53 (m, 1H, ArH), 6.08-6.05 (m, 2H, ArH), 6.06-6.04 (m, 2H, ArH), 4.12-4.07 (m, 4H, CH₂CH₂). LCMS *m*/*z* 397 [M+H]⁺, purity (UV/MS) 100/43.

### 2-(2-chloro-6-fluorophenyl)-N-(2,3-dihydrobenzo[b][1,4]dioxin-6-yl)-6-methylimidazo[1,2-a]pyrazin-3-amine

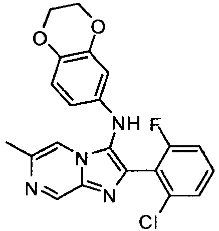

Prepared according to GP7 by using 2-amino-5-methyl pyrazine (58.0 mg, 0.53 mmol), 2,3-dihydro-6-isocyano-1,4-benzodioxine (86.0 mg, 0.53 mmol), 2-chloro-6-fluorobenzaldehyde (84.0 mg, 0.53 mmol), indium chloride (11.0 mg, 0.05 mmol) in toluene (8.0 mL) to give the title compound (129.5 mg, 60%). ¹H NMR (CDCl₃, 400 MHz) δ 9.02-9.01 (s, 1H, ArH), 7.59 (m, 1H, ArH), 7.29-7.26 (m, 2H, ArH), 7.05-7.03 (m, 1H, ArH), 6.66-6.64 (m, 1H, ArH), 6.04-6.02 (m, 2H, ArH), 5.58 (s, 1H, NH), 4.17-4.15 (m, 4H, CH₂CH₂), 2.48 (s, 3H, CH₃). LCMS *m*/*z* 411 [M+H]⁺, purity (UV/MS) 96/60.

### 4-(3-(2,3-dihydrobenzo[b][1,4]dioxin-6-ylamino)-5-methoxyimidazo[1,2-a]pyrimidin-2-yl)-3,5-dimethylphenol

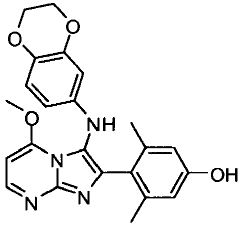

Prepared according to GP7 by using 4-methoxypyrimidin-2-amine (81.0 mg, 0.53 mmol), 2,3-dihydro-6-isocyano-1,4-benzodioxine (86.0 mg, 0.53 mmol), 2,6-dimethyl-4-hydroxy benzaldehyde (80.0 mg, 0.53 mmol), indium chloride (11.0 mg, 0.05 mmol) in toluene (8.0 mL) to give the title compound (11.8 mg, 5.3%). ¹H NMR (CDCl₃, 400 MHz) δ 7.85-7.84 (s, 1H, ArH), 6.65-6.62 (m, 1H, ArH), 6.43-6.39 (m, 3H, ArH), 6.04-6.01 (m, 2H, ArH), 5.39 (s, 1H, NH), 4.17-4.15 (m, 4H, CH₂CH₂), 4.04 (s, 3H, OCH₃), 1.87 (s, 3H, CH₃). LCMS *m*/*z* 419 [M+H]⁺, purity (UV/MS) 99/93.

### N-(2,3-dihydrobenzo[b][1,4]dioxin-6-yl)-2-(4-(2-fluoroethoxy)-2,6-dimethylphenyl)-5-methoxyimidazo[1,2-a]pyrimidin-3-amine

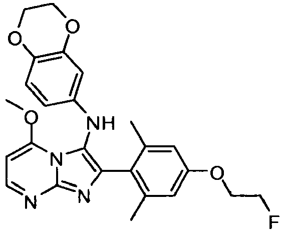

Prepared according to GP7 by using 4-methoxypyrimidin-2-amine (81.0 mg, 0.53 mmol), 2,3-dihydro-6-isocyano-1,4-benzodioxine (86.0 mg, 0.53 mmol), 4-(2-fluoroethoxy)-2,6-dimethylbenzaldehyde (104.0 mg, 0.53 mmol), indium chloride (11.0 mg, 0.05 mmol) in Toluene (8.0 mL) to give the title compound (27.2 mg, 11%). ¹H NMR (CDCl₃, 400 MHz) δ 7.84-7.82 (m, 1H, ArH), 6.59-6.55 (m, 3H, ArH), 6.31-6.30 (m, 1H, ArH), 5.99-5.98 (m, 2H, ArH), 5.8 (s, 1H, NH), 4.75-4.74 (m, 1H, CH₂), 4.63-4.62 (m, 1H, CH₂), 4.14-4.02 (m, 6H, OCH₂+CH₂CH₂), 4.02 (s, 3H, OCH₃), 2.09 (s, 6H, 2xCH₃). LCMS *m*/*z* 465 [M+H]⁺, purity (UV/MS) 98/85.

### 2-(2,6-difluoro-4-(2-fluoroethoxy)phenyl)-N-(2,3-dihydrobenzo[b][1,4]dioxin-6-yl)-5-methoxyimidazo[1,2-a]pyrimidin-3-amine

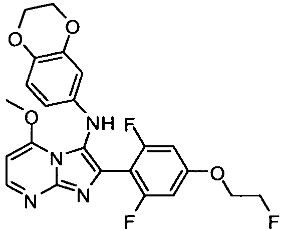

Prepared according to GP7 by using 4-methoxypyrimidin-2-amine (81.0 mg, 0.53 mmol), 2,3-dihydro-6-isocyano-1,4-benzodioxine (86.0 mg, 0.53 mmol), 2,6-difluoro-4-(2-fluoroethoxy)benzaldehyde (108.0 mg, 0.53 mmol), indium chloride (11.0 mg, 0.05 mmol) in toluene (8.0 mL) to give the title compound (34.6 mg, 14%). ¹H NMR (CDCl₃, 400 MHz) δ 7.90-7.89 (m, 1H, ArH), 6.64-6.61 (m, 1H, ArH), 6.50-6.48 (m, 2H, ArH), 6.38-6.36 (m, 1H, ArH), 6.07-6.06 (m, 2H, ArH), 5.61 (s, 1H, NH), 4.78-4.77 (m, 1H, CH₂), 4.67-4.65 (m, 1H, CH₂), 4.21-4.19 (m, 1H, CH₂), 4.14-4.13 (m, 5H, CH₂(alkyl tail)+CH₂CH₂), 4.02 (s, 3H, OCH₃). LCMS *m*/*z* 473 [M+H]⁺, purity (UV/MS) 95/50.

### 2-(2-chloro-6-fluorophenyl)-N-(2,3-dihydrobenzo[b][1,4]dioxin-6-yl)-5-methoxyimidazo[1,2-a]pyrimidin-3-amine

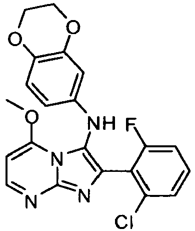

Prepared according to GP7 by using 4-methoxypyrimidin-2-amine (81.0 mg, 0.53 mmol), 2,3-dihydro-6-isocyano-1,4-benzodioxine (86.0 mg, 0.53 mmol), 2-chloro-6-fluorobenzaldehyde (84.0 mg, 0.53 mmol), indium chloride (11.0 mg, 0.05 mmol) in toluene (8.0 mL) to give the title compound (20.2 mg, 9%). ¹H NMR (CDCl₃, 400 MHz) δ 7.93-7.91 (m, 1H, ArH), 7.25-7.22 (m, 2H, ArH), 7.03-6.99 (m, 1H, ArH), 6.64-6.62 (m, 1H, ArH), 6.40-6.38 (m, 1H, ArH), 6.08-6.07 (m, 2H, ArH), 5.45 (s, 1H, NH), 4.16-4.12 (m, 4H, CH₂CH₂), 4.05 (s, 3H, OCH₃). LCMS *m*/*z* 427 [M+H]⁺, purity (UV/MS) 85/65.

### N-(2,3-dihydrobenzo[b][1,4]dioxin-6-yl)-2-(6-fluoropyridin-3-yl)imidazo[1,2-a]pyrazin-3-amine

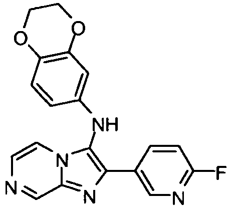

Prepared according to GP7 by using aminopyrazine (50.0 mg, 0.53 mmol), 2,3-dihydro-6-isocyano-1,4-benzodioxine (86.0 mg, 0.53 mmol), 2-fluoro-5-formylpyridine (66.0 mg, 0.53 mmol), indium chloride (11.0 mg, 0.05 mmol) in toluene (8.0 mL) to give the title compound (7.5 mg, 4%). LCMS *m*/*z* 364 [M+H]⁺, purity (UV/MS) 89/80.

### 4-(3-(2,3-dihydrobenzo[b][1,4]dioxin-6-ylamino)imidazo[1,2-a]pyrazin-2-yl)-2-fluoro-6-methoxyphenol

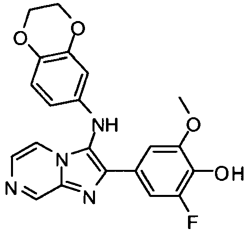

Prepared according to GP7 by using aminopyrazine (50.0 mg, 0.53 mmol), 2,3-dihydro-6-isocyano-1,4-benzodioxine (86.0 mg, 0.53 mmol), 3-fluoro-4-hydroxy-5-methoxybenzaldehyde (90.0 mg, 0.53 mmol), indium chloride (11.0 mg, 0.05 mmol) in toluene (8.0 mL) to give the title compound (0.5 mg, 0.2%). LCMS *m*/*z* 364 [M+H]⁺, purity (UV/MS) 100/90.

### N-(2,3-dihydrobenzo[b][1,4]dioxin-6-yl)-2-(3-fluoropyridin-2-yl)imidazo[1,2-a]pyrazin-3-amine

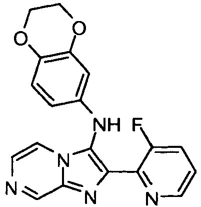

Prepared according to GP7 by using aminopyrazine (50.0 mg, 0.53 mmol), 2,3-dihydro-6-isocyano-1,4-benzodioxine (86.0 mg, 0.53 mmol), 3-fluoro-2-formylpyridine (66.0 mg, 0.53 mmol), indium chloride (11.0 mg, 0.05 mmol) in toluene (8.0 mL) to give the title compound (34.7 mg, 18%). ¹H NMR (CDCl₃, 400 MHz) δ 9.09.9-9.08 (s, 1H, ArH), 8.48-8.46 (m, 1H, ArH), 7.72-7.71 (m, 1H, ArH), 7.64-7.59 (m, 1H, ArH), 7.46-7.44 (m, 1H, ArH), 7.35-7.32 (m, 1H, ArH), 6.81-6.79 (m, 1H, ArH), ), 6.32-6.29 (m, 2H, ArH), 4.28-4.27 (m, 4H, CH₂CH₂). LCMS *m*/*z* 364 [M+H]⁺, purity (UV/MS) 100/90.

### N-(2,3-dihydrobenzo[b][1,4]dioxin-6-yl)-2-(2-fluoropyridin-3-yl)imidazo[1,2-a]pyrazin-3-amine

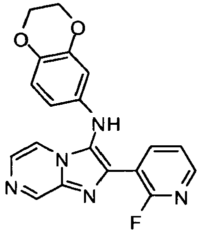

Prepared according to GP7 by using aminopyrazine (50.0 mg, 0.53 mmol), 2,3-dihydro-6-isocyano-1,4-benzodioxine (86.0 mg, 0.53 mmol), 2-fluoro-3-formylpyridine (66.0 mg, 0.53 mmol), indium chloride (11.0 mg, 0.05 mmol) in toluene (8.0 mL) to give the title compound (5.3 mg, 3%). ¹H NMR (CDCl₃, 400 MHz) δ 9.19-9.18 (s, 1H, ArH), 8.43-8.35 (m, 2H, ArH), 8.04-8.02 (m, 1H, ArH), 7.92-7.91 (m, 1H, ArH), 7.43-7.39 (m, 1H, ArH), 6.74-6.72 (m, 1H, ArH), 6.19-6.18 (m, 2H, ArH), ), 4.20-4.15 (m, 4H, CH₂CH₂). LCMS *m*/*z* 364 [M+H]⁺, purity (UV/MS) 100/100.

### N-(2,3-dihydrobenzo[b][1,4]dioxin-6-yl)-2-(6-fluoropyridin-2-yl)imidazo[1,2-a]pyrazin-3-amine

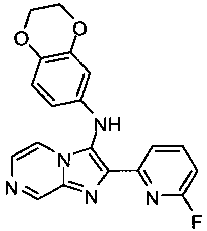

Prepared according to GP7 by using aminopyrazine (50.0 mg, 0.53 mmol), 2,3-dihydro-6-isocyano-1,4-benzodioxine (86.0 mg, 0.53 mmol), 2-fluoro-6-formylpyridine (66.0 mg, 0.53 mmol), indium chloride (11.0 mg, 0.05 mmol) in toluene (8.0 mL) to give the title compound (30.0 mg, 16%). LCMS *m*/*z* 364 [M+H]⁺, purity (UV/MS) 99/98.

### N-(2,3-dihydrobenzo[b][1,4]dioxin-6-yl)-2-(2-fluoropyridin-4-yl)imidazo[1,2-a]pyrazin-3-amine

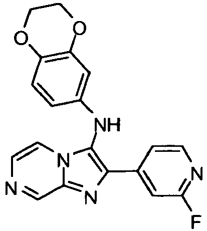

Prepared according to GP7 by using aminopyrazine (50.0 mg, 0.53 mmol), 2,3-dihydro-6-isocyano-1,4-benzodioxine (86.0 mg, 0.53 mmol), 2-fluoro-4-formylpyridine (66.0 mg, 0.53 mmol), indium chloride (11.0 mg, 0.05 mmol) in toluene (8.0 mL) to give the title compound (5.5 mg, 3%). LCMS *m*/*z* 364 [M+H]⁺, purity (UV/MS) 96/90.

### General procedure GP8

### 2-(2-chloro-6-fluorophenyl)-N-(2,3-dihydrobenzo[b][1,4]dioxin-6-yl)-5-methylimidazo[1,2-a]pyridin-3-amine

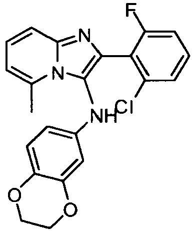

A 4 mL disposable glass reaction vessel equipped with a magnestic stirring bar was charged with 2-chloro-6-fluorobenzaldehyde (206 mg, 1.30 mmol), 6-isocyano-2,3-dihydrobenzo[b][1,4]dioxine (180 mg, 1.12 mmol), 6-methylpyridin-2-amine (111 mg, 1.03 mmol) and 1-butyl-3-methylimidazolium bromide (340 mg, 1.55 mmol). The mixture was stirred at 60 °C overnight and then at 100 °C for 3h. The mixture was dissolved in a mixture of H₂O (2 mL) and EtOAc (5 mL) by vigerous shaking. The organic layer was adsorbed onto silica, and after purification by flash CC (eluent: 10-30 % EtOAc in heptane) the title compound was obtained as purple crystals (286 mg, 67%). ¹H NMR (400 MHz, CDCl₃) δ 7.51 (d, *J*= 9.1 Hz, 1H), 7.25-7.22 (m, 2H), 7.13-7.09 (m, 1H), 7.04-6.99 (m, 1H), 6.61 (d, *J* = 8.5 Hz, 1H), 6.50 (d, *J* = 6.8 Hz, 1H), 6.00-5.95 (m, 2H), 5.11 (s, 1H), 4.16-4.12 (m, 4H), 2.72 (s, 3H). ¹³C NMR (100 MHz, CDCl₃) (meaningful signals) δ 162.4+159.9 (d, *J* = 250 Hz), 144.1, 142.1, 136.8, 136.3, 135.5+135.4 (d, *J* = 4 Hz), 130.2+130.1 (d, *J* = 10 Hz), 125.3+125.2 (d, *J* = 4 Hz), 122.7, 117.7, 116.2, 114.3+114.0 (d, *J* = 23 Hz), 113.6, 106.7, 102.5, 64.6, 64.0, 19.0. LCMS *m*/*z* 410 [M+H]⁺, purity (UV/MS) 100/85.

### (2-(2-chloro-6-fluorophenyl)-3-(2,3-dihydrobenzo[b][1,4]dioxin-6-ylamino)imidazo[1,2-a]pyridin-7-yl)methanol

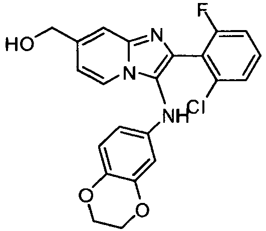

Prepared according to GP8 by using 2-chloro-6-fluorobenzaldehyde (332 mg, 2.09 mmol), 6-isocyano-2,3-dihydrobenzo[b][1,4]dioxine (329 mg, 2.04 mmol), (2-aminopyridin-4-yl)methanol (276 mg, 2.22 mmol) and 1-butyl-3-methylimidazolium bromide (639 mg, 2.91 mmol) yielding the title compound after flash CC (eluent: 20-90 % EtOAc in heptane) (250 mg, 29 %). ¹H NMR (400 MHz, CDCl₃) δ 7.80-7.79 (m, 1H), 7.62-7.61 (m, 1H), 7.27-7.23 (m, 3H), 7.05-7.01 (m, 1H), 6.83-6.81 (m, 1H), 6.65-6.63 (m, 1H), 6.06-6.03 (m, 2H), 5.37 (s, 1H), 4.73 (s, 1H), 4.17-4.12 (m, 4H). LCMS *m*/*z* 426 [M+H]⁺, purity (UV/MS) 99/60.

### 2-(2-chloro-6-fluorophenyl)-3-(2,3-dihydrobenzo[b][1,4]dioxin-6-ylamino)imidazo[1,2-a]pyridine-6-carbonitrile

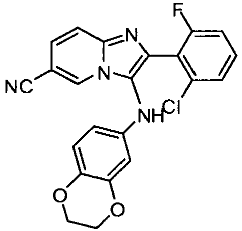

Prepared according to GP8 by using 2-chloro-6-fluorobenzaldehyde (23 mg, 0.15 mmol), 6-isocyano-2,3-dihydrobenzo[b][1,4]dioxine (28 mg, 0.17 mmol), 6-aminonicotinonitrile (12 mg, 0.10 mmol) and 1-butyl-3-methylimidazolium bromide (28 mg, 0.13 mmol) yielding the title compound after pTLC (silica, EtOAc:heptanes 1:1) (5.4 mg, 13 %). ¹H NMR (400 MHz, CDCl₃) δ 8.26-8.25 (m, 1H), 7.75-7.72 (m, 1H), 7.35-7.28 (m, 3H), 7.10-7.06 (m, 1H), 6.69 (d, J= 8.4 Hz, 1H), 6.08-6.03 (m, 2H), 5.36 (s, 1H), 4.21-4.16 (m, 4H). LCMS *m*/*z* 421 [M+H]⁺, purity (UV/MS) 91/77.

### 2-(2-chloro-6-fluorophenyl)-3-(2,3-dihydrobenzo[b][1,4]dioxin-6-ylamino)-N,N-dimethylimidazo[1,2-a]pyridine-6-sulfonamide

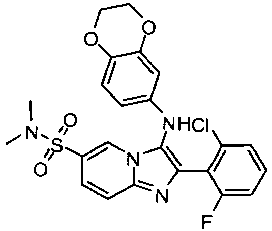

Prepared according to GP8 by using 2-chloro-6-fluorobenzaldehyde (48 mg, 0.30 mmol), 6-isocyano-2,3-dihydrobenzo[b][1,4]dioxine (46 mg, 0.29 mmol), 6-amino-N,N-dimethylpyridine-3-sulfonamide (51 mg, 0.25 mmol) and 1-butyl-3-methylimidazolium bromide (77 mg, 0.35 mmol) yielding the title compound after flash CC (eluent: 0-100 % EtOAc in heptane) (40 mg, 31 %). ¹H NMR (400 MHz, CDCl₃) δ 8.35-8.35 (m, 1H), 7.77-7.75 (m, 1H), 7.49-7.46 (m, 1H), 7.33-7.27 (m, 2H), 7.09-7.05 (m, 1H), 6.64 (d, *J* = 8.4 Hz, 1H), 6.07-6.02 (m, 2H), 5.45 (s, 1H), 4.16-4.12 (m, 4H), 2.74 (s, 6H). LCMS *m*/*z* 503 [M+H]⁺, purity (UV/MS) 95/97.

### 2-(2-chloro-6-nuorophenyl)-N-(2,3-dihydrobenzo[b][1,4]dioxin-6-yl)-5-fluoroimidazo[1,2-a]pyridin-3-amine

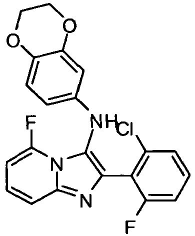

Prepared according to GP8 by using 2-chloro-6-fluorobenzaldehyde (89 mg, 0.56 mmol), 6-isocyano-2,3-dihydrobenzo[b][1,4]dioxine (78 mg, 0.48 mmol), 6-fluoropyridin-2-amine (54 mg, 0.48 mmol) and 1-butyl-3-methylimidazolium bromide (139 mg, 0.63 mmol) yielding the title compound after flash CC (eluent: 0-50 % EtOAc in heptane) (90 mg, 45 %). ¹H NMR (400 MHz, CDCl₃) δ 7.47-7.45 (m, 1H), 7.28-7.24 (m, 2H), 7.22-7.17 (m, 1H), 7.06-7.02 (m, 1H), 6.64-6.62 (m, 1H), 6.39-6.36 (m, 1H), 6.08-6.05 (m, 2H), 5.21 (s, 1H), 4.17-4.12 (m, 4H). LCMS *m*/*z* 414 [M+H]⁺, purity (UV/MS) 97/78.

### 2-(2-chloro-6-fluorophenyl)-N-(2,3-dihydrobenzo[b][1,4]dioxin-6-yl)-5-methylimidazo[1,2-a]pyrazin-3-amine

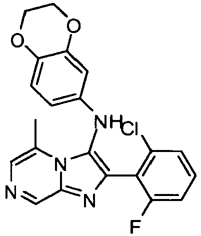

Prepared according to GP8 by using 2-chloro-6-fluorobenzaldehyde (194 mg, 1.22 mmol), 6-isocyano-2,3-dihydrobenzo[b][1,4]dioxine (177 mg, 1.10 mmol), 6-methylpyrazin-2-amine (103 mg, 0.94 mmol) and 1-butyl-3-methylimidazolium bromide (315 mg, 1.44 mmol) yielding the title compound after flash CC (eluent: 0-100 % EtOAc in heptane) (58 mg, 15 %). ¹H NMR (400 MHz, CDCl₃) δ 8.97 (s, 1H), 7.58-7.58 (m, 1H), 7.29-7.26 (m, 2H), 7.06-7.01 (m, 1H), 6.63-6.61 (m, 1H), 5.98-5.93 (m, 2H), 5.18 (s, 1H), 4.16-4.10 (m, 4H), 2.67 (s, 3H). LCMS *m*/*z* 411[M+H]⁺, purity (UV/MS) 82/-.

### 2-(3-(2,3-dihydrobenzo[b][1,4]dioxin-6-ylamino)-5-methylimidazo[1,2-a]pyridin-2-yl)-3-fluorophenol

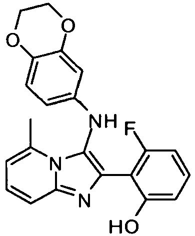

Prepared according to GP8 by using 2-fluoro-6-hydroxybenzaldehyde (185 mg, 1.32 mmol), 6-isocyano-2,3-dihydrobenzo[b][1,4]dioxine (179 mg, 1.11 mmol), 6-methylpyridin-2-amine (104 mg, 0.96 mmol) and 1-butyl-3-methylimidazolium bromide (348 mg, 1.41 mmol) yielding the title compound after flash CC (eluent: 0-50 % EtOAc in heptane) (43 mg, 11 %). ¹H NMR (400 MHz, CDCl₃) δ 7.48-7.46 (m, 1H), 7.20-7.09 (m, 2H), 6.82-6.80 (m, 1H), 6.62-6.55 (m, 3H), 5.91-5.88 (m, 1H), 5.82-5.81 (m, 1H), 5.53 (br d, *J* = 8.0 Hz, 1H), 4.14-4.11 (m, 4H), 2.78 (s, 3H). LCMS *m*/*z* 392 [M+H]⁺, purity (UV/MS) 95/65.

### tert-butyl 2-(2-chloro-6-fluorophenyl)-3-(2,3-dihydrobenzo[b][1,4]dioxin-6-ylamino)imidazor[1,2-a]pyridin-5-ylcarbamate

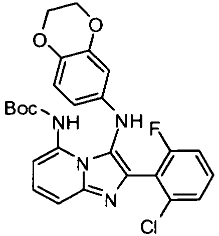

Prepared according to GP8 by using 2-chloro-6-fluorobenzaldehyde (535 mg, 3.37 mmol), 6-isocyano-2,3-dihydrobenzo[b][1,4]dioxine (419 mg, 2.60 mmol), *tert*-butyl 6-aminopyridin-2-ylcarbamate (497 mg, 2.38 mmol) and 1-butyl-3-methylimidazolium bromide (767 mg, 3.50 mmol) yielding the title compound after flash CC (eluent: 0-50 % EtOAc in heptane) (1.03 g, 85 %). ¹H NMR (400 MHz, CDCl₃) δ 10.08 (s, 1H), 7.42-7.37 (m, 2H), 7.32-7.23 (m, 3H), 7.05-7.00 (m, 1H), 6.64-6.62 (m, 1H), 6.19-6.14 (m, 2H), 5.49 (s, 1H), 4.16-4.12 (m, 4H), 1.41 (s, 9H). LCMS *m*/*z* 511 [M+H]⁺, purity (UV/MS) 99/97.

### 2-(2,6-difluoro-3-(2-fluoroethoxy)phenyl)-N-(2,3-dihydrobenzo[b][1,4]dioxin-6-yl)-5-methoxyimidazo[1,2-a]pyridin-3-amine

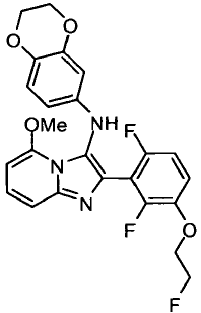

Prepared according to GP8 by using 2,6-difluoro-3-(2-fluoroethoxy)benzaldehyde (116 mg, 0.57 mmol), 6-isocyano-2,3-dihydrobenzo[b][1,4]dioxine (76 mg, 0.47 mmol), 6-methoxypyridin-2-amine (50 mg, 0.40 mmol) and 1-butyl-3-methylimidazolium bromide (150 mg, 0.68 mmol) yielding the title compound after flash CC (eluent: 0-100 % EtOAc in heptane) (34 mg, 18 %). ¹H NMR (400 MHz, CDCl₃) δ 7.28-7.26 (m, 1H), 7.18-7.13 (m, 1H), 7.00-6.94 (m, 1H), 6.86-6.82 (m, 1H), 6.61-6.59 (m, 1H), 6.03-5.96 (m, 3H), 5.39 (s, 1H), 4.77-4.75 (m, 1H), 4.65-4.63 (m, 1H), 4.29-4.27 (m, 1H), 4.22-4.20 (m, 1H), 4.15-4.11 (m, 4H), 3.77 (s, 3H). LCMS *m*/*z* 472 [M+H]⁺, purity (UV/MS) 96/79.

### 4-(3-(2,3-dihydrobenzo[b][1,4]dioxin-6-ylamino)-6-fluoroimidazo[1,2-a]pyridin-2-yl)-3,5-dimethylphenol

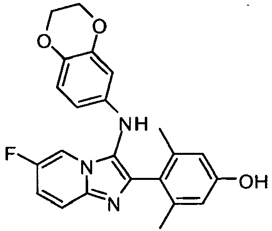

Prepared according to GP8 by using 4-hydroxy-2,6-dimethylbenzaldehyde (115 mg, 0.77 mmol), 6-isocyano-2,3-dihydrobenzo[b][1,4]dioxine (93 mg, 0.58 mmol), 5-fluoropyridin-2-amine (57 mg, 0.51 mmol) and 1-butyl-3-methylimidazolium bromide (158 mg, 0.72 mmol) yielding the title compound after flash CC (eluent: 20-100 % EtOAc in heptane) (161 mg, 78 %). LCMS *m*/*z* 406 [M+H]⁺, purity (UV/MS) 99/93.

### N-(2,3-dihydrobenzo[b][1,4]dioxin-6-yl)-5-fluoro-2-(4-(2-fluoroethoxy)-2,6-dimethylphenyl)imidazo[1,2-a]pyridin-3-amine

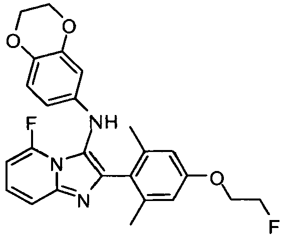

Prepared according to GP8 by using 4-(2-fluoroethoxy)-2,6-dimethylbenzaldehyde (126 mg, 0.64 mmol), 6-isocyano-2,3-dihydrobenzo[b][1,4]dioxine (78 mg, 0.48 mmol), 6-fluoropyridin-2-amine (52 mg, 0.46 mmol) and 1-butyl-3-methylimidazolium bromide (178 mg, 0.81 mmol) yielding the title compound after flash CC (eluent: 20-100 % EtOAc in heptane) (78 mg, 37 %). LCMS *m*/*z* 452 [M+H]⁺, purity (UV/MS) 94/58.

### 2-(2-chloro-6-fluoro-3-(fluoromethoxy)phenyl)-N-(2,3-dihydrobenzo[b][1,4]dioxin-6-yl)-5-methoxyimidazo[1,2-a]pyridin-3-amine

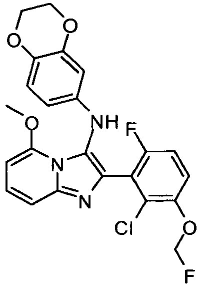

Prepared according to GP8 by using 2-chloro-6-fluoro-3-(fluoromethoxy)benzaldehyde (142 mg, 0.69 mmol), 6-isocyano-2,3-dihydrobenzo[b][1,4]dioxine (94 mg, 0.58 mmol), 6-methoxypyridin-2-amine (67 mg, 0.54 mmol) and 1-butyl-3-methylimidazolium bromide (159 mg, 0.73 mmol) yielding the title compound after pTLC (eluding with EtOAc:heptanes 2:1) (42 mg, 16 %). ¹H NMR (400 MHz, CDCl₃) δ 7.24-7.12 (m, 3H), 7.02-6.98 (m, 1H), 6.61-6.59 (m, 1H), 6.07-6.04 (m, 2H), 5.97-5.95 (m, 1H), 5.73 (s, 1H), 5.59 (s, 1H), 5.34 (s, 1H), 4.16-4.12 (m, 4H), 3.78 (s, 3H). LCMS *m*/*z* 474 [M+H]⁺, purity (UV/MS) 99/80.

### 2-(2-chloro-6-fluoro-3-(fluoromethoxy)phenyl)-N-(2,3-dihydrobenzo[b][1,4]dioxin-6-yl)-6-methoxyimidazo[1,2-a]pyridin-3-amine

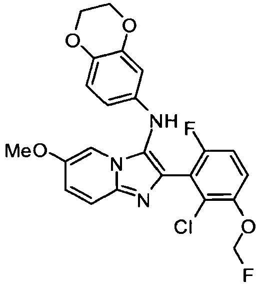

Prepared according to GP8 by using 2-chloro-6-fluoro-3-(fluoromethoxy)benzaldehyde (124 mg, 0.60 mmol), 6-isocyano-2,3-dihydrobenzo[b][1,4]dioxine (102 mg, 0.63 mmol), 5-methoxypyridin-2-amine (53 mg, 0.43 mmol) and 1-butyl-3-methylimidazolium bromide (160g, 0.73mmol) yielding the title compound after flash CC (eluent: 25-100 % EtOAc in heptane) (115 mg, 57 %). ¹H NMR (400 MHz, CDCl₃) δ 7.55 (d, *J* = 10.1 Hz, 1H), 7.36-7.35 (m, 1H), 7.20-7.17 (m, 1H), 7.04-6.98 (m, 2H), 6.64-6.62 (m, 1H), 6.06-6.03 (m, 2H), 5.73 (s, 1H), 5.59 (s, 1H), 5.37 (s, 1H), 4.17-4.12 (m, 4H), 3.73 (s, 3H). LCMS *m*/*z* 474 [M+H]⁺, purity (UV/MS) 100/88.

### 2-(2-chloro-6-fluorophenyl)-N-(2,3-dihydrobenzo[b][1,4]dioxin-6-yl)-6-methoxyimidazo[1,2-a]pyrimidin-3-amine

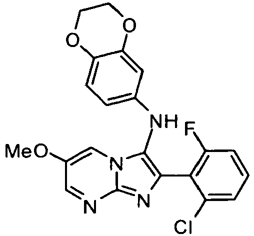

Prepared according to GP8 by using 2-chloro-6-fluorobenzaldehyde (98 mg, 0.62 mmol), 6-isocyano-2,3-dihydrobenzo[b][1,4]dioxine (83 mg, 0.52 mmol), 5-methoxypyrimidin-2-amine (53 mg, 0.42 mmol) and 1-butyl-3-methylimidazolium bromide (111 mg, 0.51 mmol) yielding the title compound after flash CC (eluent: 50-100 % EtOAc in heptane) (79 mg, 44 %).

¹H NMR (400 MHz, CDCl₃) δ 8.43 (d, *J* = 3.0 Hz, 1H), 7.62 (d, *J* = 3.0 Hz, 1H), 7.26-7.22 (m, 2H), 7.02-6.98 (m, 1H), 6.64 (d, *J* = 8.4 Hz, 1H), 6.07-6.03 (m, 2H), 5.56 (s, 1H), 4.16-4.12 (m, 4H), 3.78 (s, 3H).

LCMS *m*/*z* 427 [M+H]⁺, purity (UV/MS) 90/70.

### 2-(2-chloro-6-fluorophenyl)-N-(2,3-dihydrobenzo[b][1,4]dioxin-6-yl)-7-methylimidazo[1,2-a]pyridin-3-amine

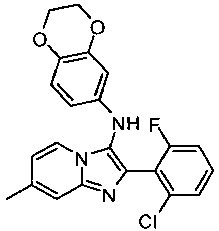

Prepared according to GP8 by using 2-amino-4-methylpyridine (10.8 mg, 0.1 mmol), 2,3-Dihydro-6-isocyano-1,4-benzodioxine (17.7 mg, 0.115 mmol), 2-chloro-6-fluorobenzaldehyde (23.8 mg, 0.15 mmol), 1-Butyl-3-methylimidazolium bromide (26.3 mg, 0.12 mmol) to give the title compound after purification by preparative TLC (EtOAc/Heptane, 1:1) (12.3mg, 0.03mmol, 30%).

¹H NMR (CDCl₃, 400 MHz) δ 7.54-7.53 (m, 1H, ArH), 7.05-7.03 (m, 3H, ArH), 6.80-6.82 (m, 1H, ArH), 6.46-6.42 (m, 2H, ArH), 5.85-5.84 (m, 2H, ArH), 5.19 (br s, 1H, NH), 3.94-3.93 (m, 4H, CH₂CH₂), 2.06 (s, 3H, CH₃).

LCMS *m*/*z* 410 [M+H]⁺, purity (UV/MS) 92/46.

### General procedure GP9

### 5-chloro-2-(2-chloro-6-fluorophenyl)-N-(2,3-dihydrobenzo[b][1,4]dioxin-6-yl)-6-methoxyimidazo[1,2-a]pyridin-3-amine

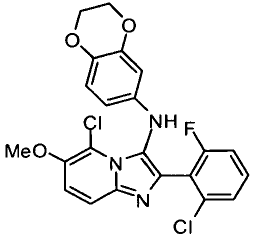

To a solution of 6-chloro-5-methoxypyridin-2-amine (196 mg, 1.24 mmol) and 2-chloro-6-fluorobenzaldehyde (215 mg, 1.36 mmol) in 2:1 CH₂Cl₂:MeOH (4.5 mL) was added Sc(OTf)₃ (35 mg, 0.07 mmol). The mixture was stirred for 30 min and then 6-isocyano-2,3-dihydrobenzo[b][1,4]dioxine (229 mg, 1.42 mmol) was added in one portion. The solution was stirred at room temperature overnight. The mixture was evaporated to dryness and dissolved in CH₂Cl₂ (6 mL). This CH₂Cl₂ layer was passed through plug of silica gel which was subsequently washed with CH₂Cl₂ (10 mL). After eluding with EtOAc and evaporation the title compound was obtained as a colorless foam (492 mg, 86%).

¹H NMR (400 MHz, CDCl₃) δ 7.87-7.83 (m, 1H), 7.36-7.26 (m, 3H), 7.08-7.03 (m, 1H), 6.64-6.61 (m, 1H), 6.01-5.99 (m, 2H), 5.20 (s, 1H), 4.17-4.11 (m, 4H), 3.95 (s, 3H).

LCMS *m*/*z* 460 [M+H]⁺, purity (UV/MS) 98/85.

### 2-(2-chloro-6-fluoro-3-(2-fluoroethoxy)phenyl)-N-(2,3-dihydrobenzo[b][1,4]dioxin-6-yl)-5-methoxyimidazo[1,2-a]pyridin-3-amine

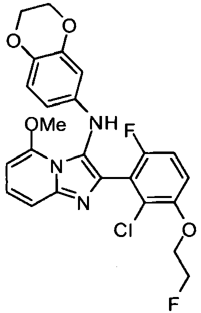

Prepared according to GP9 by using 6-methoxypyridin-2-amine (74 mg, 0.60 mmol), 2-chloro-6-fluoro-3-(2-fluoroethoxy)benzaldehyde (136 mg, 0.62 mmol), 6-isocyano-2,3-dihydrobenzo[b][1,4]dioxine (116 mg, 0.72 mmol) and Sc(OTf)₃ (15 mg, 0.03 mmol) in CH₂Cl₂:MeOH 2:1 (3 mL) with the exception that all four compounds were mixed prior to dissolution. The crude mixture was adsorbed onto silica, and after flash CC (eluent: 40-70 % EtOAc in heptane) the title compound was obtained (141 mg, 48%).

¹H NMR (400 MHz, CDCl₃) δ 7.16-7.13 (m, 1H), 7.06-7.02 (m, 1H), 6.88-6.82 (m, 2H), 6.53-6.51 (m, 1H), 5.99-5.97 (m, 2H), 5.87-5.85 (m, 1H), 5.26 (s, 1H), 4.72-4.70 (m, 1H), 4.61-4.59 (m, 1H), 4.18-4.16 (m, 1H), 4.12-4.10 (m, 1H), 4.07-4.02 (m, 4H), 3.68 (s, 3H).

LCMS *m*/*z* 488 [M+H]⁺, purity (UV/MS) 87/70.

### 2-(6-chloro-2-fluoro-3-(fluoromethoxy)phenyl)-N-(2,3-dihydrobenzo[b][1,4]dioxin-6-yl)-5-methoxyimidazo[1,2-a]pyridin-3-amine

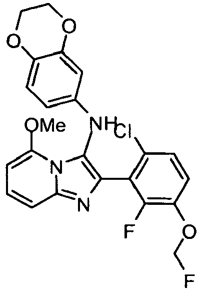

Prepared according to GP9 by using 6-methoxypyridin-2-amine (51 mg, 0.41 mmol), 6-chloro-2-fluoro-3-(fluoromethoxy)benzaldehyde (92 mg, 0.45 mmol), 6-isocyano-2,3-dihydrobenzo[b][1,4]dioxine (90 mg, 0.56 mmol) and Sc(OTf)₃ (14 mg, 0.03 mmol) in CH₂Cl₂:MeOH 2:1 (3 mL) with the exception that all four compounds were mixed prior to dissolution. The crude mixture was adsorbed onto silica, and after flash CC (eluent: 40-70 % EtOAc in heptane) the title compound was obtained (92 mg, 47%).

¹H NMR (400 MHz, CDCl₃) δ 7.28-7.26 (m, 1H), 7.19-7.13 (m, 3H), 6.61-6.59 (m, 1H), 6.07-6.05 (m, 2H), 5.99-5.97 (m, 1H), 5.71 (s, 1H), 5.58 (s, 1H), 5.37 (s, 1H), 4.15-4.10 (m, 4H), 3.78 (s, 3H).

LCMS *m*/*z* 474 [M+H]⁺, purity (UV/MS) 78/81.

### 2-(4,6-dichloropyrimidin-5-yl)-N-(2,3-dihydrobenzo[b][1,4]dioxin-6-yl)-5-methoxyimidazon[1,2-a]pyridin-3-amine

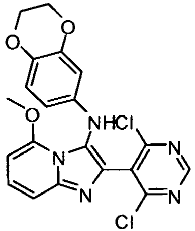

Prepared according to GP9 by using 6-methoxypyridin-2-amine (0.25 mmol, 31 mg), 4,6-dichloro-5-pyrimidinecarboxaldehyde (0.26 mmol, 46 mg), Sc(OTf)₃ (0.01 mmol, 6 mg), 6-isocyano-2,3-dihydrobenzo[b][1,4]dioxine (0.28 mmol, 44 mg) in CH₂Cl₂ (1 mL) and MeOH (0.5 mL). The crude product was concentrated in vacuo and purified by prep. TLC (eluent 5 % methanol in heptane) yielding the title compound (27 mg, 24%).

¹H NMR (400 MHz, CDCl₃) δ 8.70 (s, 1H), 7.25-7.14 (m, 2H), 6.68-6.54 (m, 1H), 6.14-6.05 (m, 2H), 6.00 (d, *J* = 6.6 Hz, 1H), 5.45-5.36 (m, 1H), 4.20-4.07 (m, 4H), 3.81 (s, 3H).

LCMS *m*/*z* 414 [M+H]⁺, purity (UV/MS) 93/50.

### N-(2,3-dihydrobenzo[b][1,4]dioxin-6-yl)-2-(4-(fluoromethoxy)-2,6-dimethylphenyl)-5-methoxyimidazo[1,2-a]pyridin-3-amine

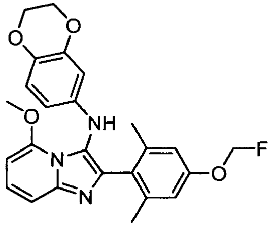

Prepared according to GP9 by using 6-Methoxy-pyridin-2-ylamine (162.0 mg, 1.30 mmol), 4-(fluoromethoxy)-2,6-dimethylbenzaldehyde (240.0 mg, 1.32 mmol), 2,3-dihydro-6-isocyano-1,4-benzodioxine (209.0 mg, 1.30 mmol), scandium triflate (32.0 mg, 0.07 mmol) in toluene (10.0 mL) to give the title compound (212.6 mg, 0.47 mmol, 36%)

¹H NMR (CDCl₃, 400 MHz) δ 7.18-7.08 (m, 2H, ArH), 6.76-6.75 (m, 2H, ArH), 6.59-6.58 (m, 1H, ArH), 6.00-5.93 (m, 3H, ArH), 5.73 (s, 1H, CH₂F), 5.59 (s, 1H, CH₂F), 4.14-4.11 (m, 4H, CH₂CH₂), 3.74 (s, 3H, OCH₃).

LCMS *m*/*z* 396 [M+H]⁺, purity (UV/MS) 99/70.

### N-(2,3-dihydrobenzo[b][1,4]dioxin-6-yl)-2-(3,5-dimethylpyridin-4-yl)-5-methoxyimidazo[1,2-a]pyridin-3-amine

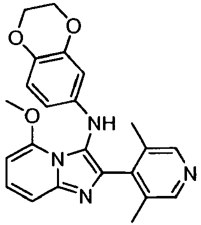

Prepared according to GP9 by using 6-Methoxy-pyridin-2-ylamine (50.0 mg, 0.40 mmol), 3,5-dimethylisonicotinaldehyde (55.0 mg, 0.41 mmol), 2,3-dihydro-6-isocyano-1,4-benzodioxine (65.0 mg, 0.40 mmol), scandium triflate (11.0 mg, 0.02 mmol) in toluene (5.0 mL) to give the title compound (1.7 mg, 0.004 mmol, 1%)

¹H NMR (CDCl₃, 400 MHz) δ 8.30-8.29 (m, 2H, ArH), 7.20-7.12 (m, 2H, ArH), 6.61-6.59 (m, 1H, ArH), 6.01-5.97 (m, 3H, ArH), 6.00-5.93 (m, 3H, ArH), 5.33 (s, 1H, NH), 4.15-4.09 (m, 4H, CH₂CH₂), 3.81 (s, 3H, OCH₃) 2.22 (s, 6H, 2 x CH₃). LCMS *m*/*z* 396 [M+H]⁺, purity (UV/MS) 95/90.

### Final compound examples, by modification of MCR products

### 2-(2-chloro-6-fluorophenyl)-N-(2,3-dihydrobenzo[b][1,4]dioxin-6-yl)-N,5-dimethylimidazo[1,2-a]pyridin-3-amine

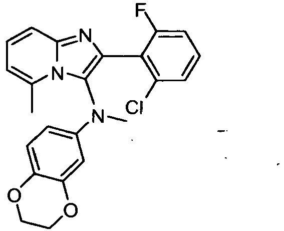

A dry flask was charged with 2-(2-chloro-6-fluorophenyl)-N-(2,3-dihydrobenzo[b][1,4]dioxin-6-yl)-5-methylimidazo[1,2-a]pyridin-3-amine (50 mg, 0.12 mmol) and NaH (50% w/w, 11 mg, 0.23 mmol). To this mixture was added dry DMF (1 mL). The mixture was stirred at 45 °C for 30 min to yield a deep red solution. This solution was cooled to 0 °C and a solution of MeI in DMF (1M, 0.15 mL, 0.15 mmol) was added and the mixture stirred at room temperature overnight. The mixture was evaporated to dryness and subjected to purification by pTLC (eluent: EtOAc:Heptanes 1:1) to give the title compound (49 mg, 95%).

¹H NMR (400 MHz, CDCl₃) δ 7.52-7.50 (m, 1H), 7.27-7.20 (m, 2H), 7.15-7.11 (m, 1H), 6.99-6.95 (m, 1H), 6.63 (d, *J* =8.9 Hz, 1H), 6.53-6.51 (m, 1H), 6.05-6.04 (m, 1H), 5.95-5.93 (m, 1H), 4.19-4.14 (m, 4H), 3.14 (s, 3H), 2.44 (s, 3H).

LCMS *m*/*z* 424 [M+H]⁺, purity (UV/MS) 99/82.

### 2-(2-Chloro-6-fluoro-phenyl)-3-(2,3-dihydro-benzo[1,4]dioxin-6-yl)-3,5-dihydro-1,3,5,8b-tetraaza-acenaphthylen-4-one

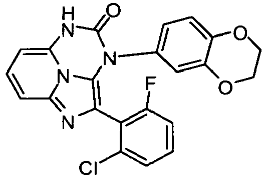

A MW reaction vessel was charged with tert-butyl 2-(2-chloro-6-fluorophenyl)-3-(2,3-dihydrobenzo[b][1,4]dioxin-6-ylamino)imidazo[1,2-a]pyridin-5-ylcarbamate (151 mg, 0.30 mmol) in 96% EtOH (4 mL). A solution of NaOH(aq) (2.0 M, 0.50 mL, 1.0 mmol) was added. The vial was capped and heated to 140 °C for 20 min. The mixture was poured into saturated aqueous NH₄Cl (5 mL) and the resulting mixture extracted with EtOAc (3x 25 mL). The combined organic layers were dried over brine, Na₂SO₄ and adsorbed onto celite. Purification by flash CC (eluent: 20-100 % EtOAc in heptane) gave the title compound as greenish crystals (63 mg, 49%).

¹H NMR (400 MHz, dmso-*d6*) δ 10.63 (br s, 1H), 7.25-7.19 (m, 1H), 7.08-7.06 (m, 1H), 6.92-6.83 (m, 2H), 6.68-6.58 (m, 3H), 6.51-6.49 (m, 1H), 5.59-5.58 (m, 1H), 4.11-4.02 (m, 4H).

LCMS *m*/*z* 437 [M+H]⁺, purity (UV/MS) 98/91

### 2-(2-chloro-6-fluorophenyl)-N-(2,3-dihydrobenzo[b][1,4]dioxin-6-yl)-5,6-dimethoxyimidazo[1,2-a]pyridin-3-amine

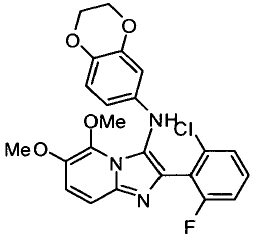

To a solution of 5-chloro-2-(2-chloro-6-fluorophenyl)-N-(2,3-dihydrobenzo[b][1,4]dioxin-6-yl)-6-methoxyimidazo[1,2-a]pyridin-3-amine (76 mg, 0.17 mmol) in dry MeOH (3 mL) in a MW reaction vessel was added NaH (50% w/w, 38 mg, 0.79 mmol). The vial was capped and heated to 130 °C for 1h. The mixture was poured into saturated aqueous NH₄Cl (5 mL), and this mixture was extracted with CH₂Cl₂ (3x 15 mL). The combined organic layers were washed with brine, dried over Na₂SO₄ and evaporated to dryness. The title compound was isolated by pTLC (Eluent: heptanes:EtOAc 1:2)(6 mg, 8%).

LCMS *m*/*z* 456 [M+H]⁺, purity (UV/MS) 99/76.

### General procedure GP 10

### 2-(2-chloro-6-fluorophenyl)-N-(2,3-dihydrobenzo[b][1,4]dioxin-6-yl)-5-ethoxyimidazo[1,2-a]pyridin-3-amine

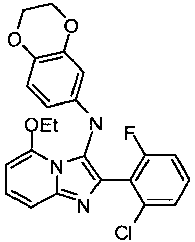

A MW reaction vessel equipped with a magnetic stirring bar was charged with 2-(2-chloro-6-fluorophenyl)-N-(2,3-dihydrobenzo[b][1,4]dioxin-6-yl)-5-fluoroimidazo[1,2-a]pyridin-3-amine (0.05 mmol, 20 mg) and dry ethanol (0.5 mL). The vial was flushed with argon, and then sodium hydride (50%, 0.08 mmol, 3.2 mg) was added. The reaction mixture was left stirring at room temperature until hydrogen generation ceased and then heated in the MW at 120 °C for 30 min. Upon concentration *in vacuo* the product precipitated. The mixture was filtered and the crystals washed with methanol. After drying the title product was obtained (8.9 mg, 40%).

¹H NMR (400 MHz, CDCl₃) δ 7.25-7.20 (m, 3H), 7.13 (dd, *J* = 9.0, 7.4 Hz, 1H), 7.05-7.00 (m, 1H), 6.61 (td, *J* = 2.7, 1.1 Hz, 1H), 6.06-6.02 (m, 2H), 5.93 (dd, *J* = 7.4, 0.9 Hz, 1H), 5.31 (s, 1H), 4.18-4.10 (m, 4H), 4.03 (q, *J* = 7.0 Hz, 2H), 1.19 (t, *J* = 7.0 Hz, 3H).

LCMS *m*/*z* 440 [M+H]⁺, purity (UV/MS) 100/95.

### 2-(2-chloro-6-fluorophenyl)-N-(2,3-dihydrobenzo[b][1,4]dioxin-6-yl)-5-methoxyimidazo[1,2-a]pyridin-3-amine

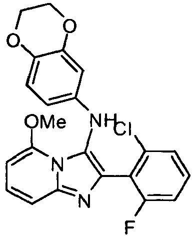

Prepared according to GP10 by using 2-(2-chloro-6-fluorophenyl)-N-(2,3-dihydrobenzo[b][1,4]dioxin-6-yl)-5-fluoroimidazo[1,2-a]pyridin-3-amine (80 mg, 0.19 mmol), NaH (50% w/w, 15 mg, 0.31 mmol) in dry MeOH (2 mL). The title compound was obtained after flash CC (eluent: 0-100 % EtOAc in heptane) (49 mg, 60%).

¹H NMR (400 MHz, CDCl₃) δ 7.29-7.25 (m, 3H), 7.18-7.14 (m, 1H), 7.06-7.02 (m, 1H), 6.64-6.62 (m, 1H), 6.10-6.07 (m, 2H), 5.99-5.97 (m, 1H), 5.36 (s, 1H), 4.18-4.13 (m, 4H), 3.79 (s, 3H).

¹³C NMR (100 MHz, CDCl₃) (meaningful signals) δ 171.0, 161.2 (*J* = 250 Hz), 151.8, 144.9, 143.8, 142.1, 136.6, 135.7, 130.0 (d, *J* = 9.6 Hz), 126.2, 125.2 (d, *J* = 22.8 Hz), 122.3, 117.3, 117.2, 114.1, 113.9, 110.5, 107.3, 103.0, 89.0, 64.5, 64.0, 56.5.

LCMS *m*/*z* 426 [M+H]⁺, purity (UV/MS) 98/87.

### 2-(2-chloro-6-fluorophenyl)-N-(2,3-dihydrobenzo[b][1,4]dioxin-6-yl)-5-isopropoxyimidazo[1,2-a]pyridin-3-amine

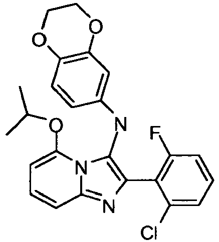

Prepared according to GP10 using 2-(2-chloro-6-fluorophenyl)-N-(2,3-dihydrobenzo[b][1,4]dioxin-6-yl)-5-fluoroimidazo[1,2-a]pyridin-3-amine (0.05 mmol, 20 mg) and dry isopropanol (0.5 mL). Purified by flash CC (eluent: 50% EtOAc in heptane) yielding the title compound (13 mg, 56%).

¹H NMR (400 MHz, CDCl₃) δ 7.27-7.20 (m, 3H), 7.17-7.11 (m, 1H), 7.05-6.99 (m, 1H), 6.62 (d, *J* = 8.1 Hz, 1H), 6.05-6.00 (m, 2H), 5.94 (d, *J* = 7.5 Hz, 1H), 5.28 (s, 1H), 4.63-4.56 (hep, *J* = 6.1 Hz, 1H), 4.17-4.10 (m, 4H), 1.14 (d, *J* = 6.1 Hz, 6H).

¹³C NMR (100 MHz, CDCl₃) δ 162.5+160.0 (d, *J* = 250 Hz), 149.9, 143.8, 142.4, 136.4, 130.0, 129.9, 125.2, 125.1, 121.7, 117.2, 114.1, 113.9, 109.7, 107.1, 102.7, 90.2, 72.2, 64.6, 64.1, 21.1.

LCMS *m*/*z* 454 [M+H]⁺, purity (UV/MS) 93/60.

### 5-sec-butoxy-2-(2-chloro-6-fluorophenyl)-N-(2,3-dihydrobenzo[b][1,4]dioxin-6-yl)imidazo[1,2-a]pyridin-3-amine

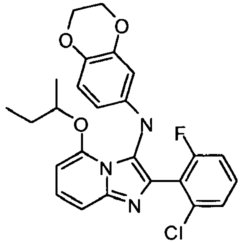

Prepared according to GP10 using 2-(2-chloro-6-fluorophenyl)-N-(2,3-dihydrobenzo[b][1,4]dioxin-6-yl)-5-fluoroimidazo[1,2-a]pyridin-3-amine (0.05 mmol, 20 mg) and dry 2-butanol (0.5 mL). Purified by flash CC (eluent: 50% EtOAc in heptane) yielding the title compound (17 mg, 74%).

¹H NMR (400 MHz, CDCl₃) δ 7.30-7.17 (m, 3H), 7.17-6.96 (m, 2H), 6.65-6.56 (m, 1H), 6.07-5.98 (m, 2H), 5.92 (s, 1H), 5.26 (s, 1H), 4.43-4.30 (m, 1H), 4.19-4.05 (m, 4H), 1.64-1.35 (m, 2H), 1.17-1.04 (m, 3H), 0.84-0.74 (m, 3H).

¹³C NMR (100 MHz, CDCl₃) δ 162.5+160.0 (d, *J* = 250 Hz), 150.2, 145.5, 143.8, 142.4, 136.4, 130.0, 129.9, 126.3, 125.2, 125.1, 121.7, 117.2, 114.1, 113.9, 109.7, 107.1, 102.7, 89.8, 77.2, 64.6, 64.1, 28.5, 18.3, 9.6.

LCMS *m*/*z* 468 [M+H]⁺, purity (UV/MS) 95/64.

### 2-(2-chloro-6-fluorophenyl)-N3-(2,3-dihydrobenzo[b][1,4]dioxin-6-yl)-N5,N5-dimethylimidazo[1,2-a]pyridine-3,5-diamine

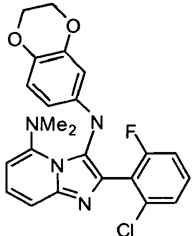

Prepared according to GP10 using 2-(2-chloro-6-fluorophenyl)-N-(2,3-dihydrobenzo[b][1,4]dioxin-6-yl)-5-fluoroimidazo[1,2-a]pyridin-3-amine (0.05 mmol, 20 mg) and dimethylamine (2M in THF, 0.5 mL). Purified by flash CC (eluent: 50% EtOAc in heptane) yielding the title compound (5 mg, 23%).

¹H NMR (400 MHz, CD₃OD) δ 7.37-7.18 (m, 4H), 7.11-7.04 (m, 1H), 6.47 (d, *J* = 8.4 Hz, 1H), 6.44 (dd, *J* = 6.1, 2.2 Hz, 1H), 6.03-5.96 (m, 2H), 4.12-4.04 (m, 4H), 2.64 (s, 6H)

¹³C NMR (100 MHz, CD₃OD) δ (meaningful signals) 153.0, 148.4, 147.6, 145.2, 140.1, 134.2, 134.1, 129.7, 128.8, 128.7, 120.3, 117.7, 117.4, 115.0, 110.6, 106.1, 104.7, 68.3, 67.8, 46.7.

LCMS *m*/*z* 439 [M+H]⁺, purity (UV/MS) 96/84.

### 2-(2-chloro-6-fluorophenyl)-N-(2,3-dihydrobenzo[b][1,4]dioxin-6-yl)-5-(2-fluoroethoxy)imidazo[1,2-a]pyridin-3-amine

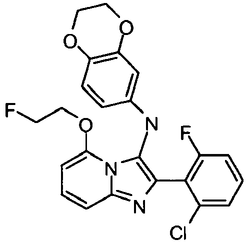

Prepared according to GP10 using 2-(2-chloro-6-fluorophenyl)-N-(2,3-dihydrobenzo[b][1,4]dioxin-6-yl)-5-fluoroimidazo[1,2-a]pyridin-3-amine (0.05 mmol, 20 mg) and 2-fluoroethanol (2M in THF, 0.5 mL). Purified by flash CC (eluent: 50% EtOAc in heptane) yielding the title compound (10 mg, 44%).

¹H NMR (400 MHz, CDCl₃) δ 7.30 (d, *J* = 9.1 Hz, 1H), 7.27-7.22 (m, 2H), 7.15 (t, *J* = 8.1, 8.1 Hz, 1H), 7.07-6.99 (m, 1H), 6.65-6.60 (m, 1H), 6.11-6.05 (m, 2H), 5.97 (d, *J* = 7.4 Hz, 1H), 5.34 (s, 1H), 4.53-4.47 (m, 1H), 4.41-4.35 (m, 1H), 4.24-4.19 (m, 1H), 4.18-4.08 (m, 5H).

LCMS *m*/*z* 458 [M+H]⁺, purity (UV/MS) 96/93.

### 4-(3-(2,3-dihydrobenzo[b][1,4]dioxin-6-ylamino)-5-methoxyimidazo[1,2-a]pyridin-2-yl)-3,5-dimethylphenol

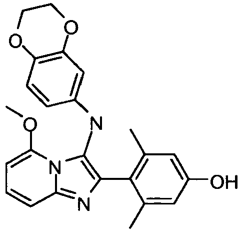

Prepared according to GP10 using 4-(3-(2,3-dihydrobenzo[b][1,4]dioxin-6-ylamino)-5-fluoroimidazo[1,2-a]pyridin-2-yl)-3,5-dimethylphenol (0.3 mmol, 120 mg) and dry methanol (2.0 mL). Purified by flash CC (eluent: 50% EtOAc in heptane) yielding the title compound (83 mg, 66%).

¹H NMR (400 MHz, CD₃OD) δ 7.27 (ddd, *J* = 8.8, 7.5, 0.5 Hz, 1H), 7.09 (d, *J* = 8.9 Hz, 1H), 6.54 (d, *J* = 8.6 Hz, 1H), 6.51 (s, 2H), 6.19 (d, *J* = 7.6 Hz, 1H), 5.99 (dd, *J* = 8.9, 2.4 Hz, 1H), 5.93 (d, *J* = 2.7 Hz, 1H), 4.14-4.05 (m, 4H), 3.77 (s, 3H), 2.09 (s, 6H).

¹³C NMR (100 MHz, CD₃OD) δ 160.5, 156.1, 148.1, 147.7, 147.1, 143.1, 143.0, 140.0, 131.1, 127.6, 125.8, 120.5, 117.5, 111.8, 110.6, 106.0, 93.0, 68.3, 67.8, 59.8, 23.3.

LCMS *m*/*z* 418 [M+H]⁺, purity (UV/MS) 100/83.

### 3,5-dichloro-4-(3-(2,3-dihydrobenzo[b][1,4]dioxin-6-ylamino)-5-methoxyimidazo[1,2-a]pyridin-2-yl)phenol

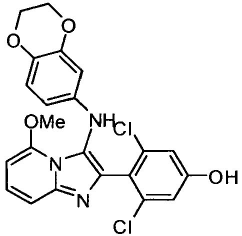

Prepared according to GP10 using 3,5-dichloro-4-(3-(2,3-dihydrobenzo[b][1,4]dioxin-6-ylamino)-5-fluoroimidazo[1,2-a]pyridin-2-yl)phenol (0.13 mmol, 56 mg) and dry methanol (0.5 mL). Purified by prep. TLC (eluent: 50% EtOAc in heptane) yielding the title compound (11 mg, 19%).

¹H NMR (400 MHz, CD₃OD) δ 7.25 (dd, *J* = 8.9, 7.5 Hz, 1H), 7.09 (dd, *J* = 8.9, 0.9 Hz, 1H), 6.84 (s, 2H), 6.55-6.50 (m, 1H), 6.16 (dd, *J* = 7.5, 0.8 Hz, 1H), 6.09 (dd, *J* = 8.6, 2.7 Hz, 1H), 6.07-6.04 (m, 1H), 4.13-4.05 (m, 4H), 3.77 (s, 3H).

¹³C NMR (100 MHz, CD₃OD) δ 158.6, 152.2, 144.3, 143.7, 142.9, 136.3, 136.2, 136.2, 127.1, 122.3, 116.5, 114.8, 108.3, 107.0, 102.5, 89.0, 64.4, 63.9, 55.8.

LCMS *m*/*z* 457 [M+H]⁺, purity (UV/MS) 95/60.

### 3,5-dichloro-4-(3-(2,3-dihydrobenzo[b][1,4]dioxin-6-ylamino)-5-(2-fluoroethoxy)imidazo[1,2-a]pyridin-2-yl)phenol

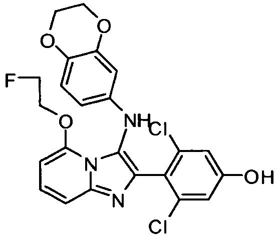

Prepared according to GP10 using 3,5-dichloro-4-(3-(2,3-dihydrobenzo[b][1,4]dioxin-6-ylamino)-5-fluoroimidazo[1,2-a]pyridin-2-yl)phenol (0.05 mmol, 22 mg) and 2-fluoroethanol (0.25 mmol, 16 mg)in dry THF (0.5 mL). Purified by prep. TLC (eluent: 50% EtOAc in heptane) yielding the title compound (7 mg, 29%).

¹H NMR (400 MHz, CD₃OD) δ 7.32-7.21 (m, 1H), 7.13 (d, *J* = 8.98 Hz, 1H), 6.85 (d, *J* = 2.58 Hz, 2H), 6.55 (dd, *J* = 8.62, 0.44 Hz, 1H), 6.22 (d, *J* = 7.52 Hz, 1H), 6.08 (ddd, *J* = 8.61, 2.68, 0.71 Hz, 1H), 6.05-6.02 (m, 1H), 4.46 (d, *J* = 7.75 Hz, 1H), 4.39-4.31 (m, 1H), 4.26 (dd, *J* = 4.91, 2.99 Hz, 1H), 4.20 (dd, *J* = 5.03, 2.83 Hz, 1H), 4.15-4.04 (m, 1H).

¹³C NMR (100 MHz, CD₃OD) δ 158.7, 151.0, 144.5, 143.8, 142.8, 136.3, 136.1, 127.1, 122.4, 116.6, 114.8, 108.8, 106.7, 102.2, 90.1, 81.5, 79.8, 69.0, 64.4, 63.9, 60.1.

LCMS *m*/*z* 457 [M+H]⁺, purity (UV/MS) 95/60.

### 2-(2-chloro-6-fluorophenyl)-N-(2,3-dihydrobenzo[b][1,4]dioxin-6-yl)-6-(2-fluoroethoxy)imidazo[1,2-a]pyridin-3-amine

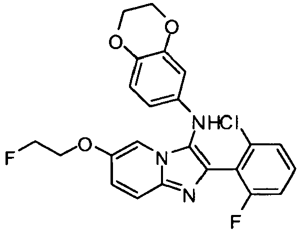

Prepared according to GP10 using 3,5-dichloro-4-(3-(2,3-dihydrobenzo[b][1,4]dioxin-6-ylamino)-6-fluoroimidazo[1,2-a]pyridin-2-yl)phenol (0.1 mmol, 41 mg) and 2-fluoroethanol (0. 5 mmol, 32 mg) in dry THF (0.5 mL). Purified by prep. TLC (eluent: 50% EtOAc in heptane) yielding the title compound (12 mg, 27%).

¹H NMR (400 MHz, CDCl₃) δ 7.78-7.73 (m, 1H), 7.62 (dd, *J* = 9.7, 4.9 Hz, 1H), 7.35-7.24 (m, 3H), 7.18-7.11 (m, 1H), 7.06-6.98 (m, 1H), 6.68 (d, *J* = 8.8 Hz, 1H), 6.27 (d, *J* = 2.8 Hz, 1H), 6.17 (dd, *J* = 8.8, 2.8 Hz, 1H), 4.23-4.13 (m, 4H), 3.73-3.63 (m, 4H).

LCMS *m*/*z* 457 [M+H]⁺, purity (UV/MS) 75/30.

A byproduct was further collected from the prep. TLC:

### 2-(2-chloro-6-fluorophenyl)-N-(2,3-dihydrobenzo[b][1,4]dioxin-6-yl)-6-(vinyloxy)imidazo[1,2-a]pyridin-3-amine

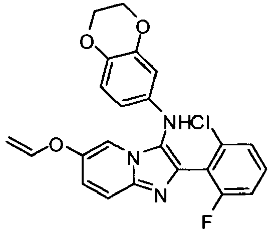

Yield: 10 mg.

¹H NMR (400 MHz, CDCl₃) δ 7.85-7.79 (m, 1H), 7.70 (dd, *J* = 9.8, 4.8 Hz, 1H), 7.29-7.20 (m, 3H), 7.16 (dd, *J* = 10.2, 8.0 Hz, 1H), 7.06-7.00 (m, 1H), 6.64-6.58 (m, 1H), 6.06 (d, *J* = 2.8 Hz, 1H), 5.99-5.92 (m, 1H), 4.36-4.32 (m, 2H), 4.20-4.07 (m, 4H).

LCMS *m*/*z* 457 [M+H]⁺, purity (UV/MS) 94/93.

### 3-Chloro-4-(3-(2,3-dihydrobenzo[b][1,4]dioxin-6-ylamino)-5-methoxyimidazo[1,2-alpyridin-2-yl)-5-fluorophenol

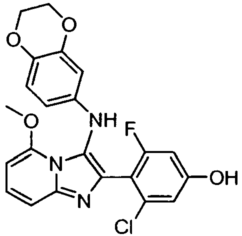

Prepared according to GP10 using 3-chloro-4-(3-(2,3-dihydrobenzo[b][1,4]dioxin-6-ylamino)-5-fluoroimidazo[1,2-a]pyridin-2-yl)-5-fluorophenol (0.2 mmol, 85 mg) and dry methanol (0.5 mL). Purified by flash chromatography (eluent: 50-100% EtOAc in heptane) yielding the title compound (23 mg, 26%).

¹H NMR (400 MHz, CD₃OD) δ 7.21 (dd, *J* = 8.96, 7.51 Hz, 1H), 7.07 (dd, *J* = 8.96, 0.67 Hz, 1H), 6.74 (dd, *J* = 2.26, 1.39 Hz, 1H), 6.56-6.49 (m, 2H), 6.10 (d, *J* = 7.53 Hz, 1H), 6.03 (dd, *J* = 8.63, 2.66 Hz, 1H), 5.98 (d, *J* = 2.62 Hz, 1H), 4.10-4.03 (m, 4H).

¹³C NMR (101 MHz, CD₃OD) δ 163.17+160.72 (d, *J =* 247 Hz), 159.31+159.18 (d, *J* = 13 Hz), 152.1, 144.5, 143.8, 142.8, 136.2, 135.88+135.81 (d, *J* = 8 Hz), 132.9, 127.2, 123.2, 116.6, 112.09, 112.06, 111.9, 108.2, 106.8, 102.3, 101.49+101.23 (d, *J* = 26 Hz), 89.0, 64.4, 63.8, 55.8.

LCMS *m*/*z* 457 [M+H]⁺, purity (UV/MS) 93/70.

### N-(2,3-Dihydrobenzo[b][1,4]dioxin-6-yl)-2-(3-fluoro-5-(2-fluoroethoxy)pyridin-4-yl)-5-(2-fluoroethoxy)imidazo[1,2-a]pyridin-3-amine

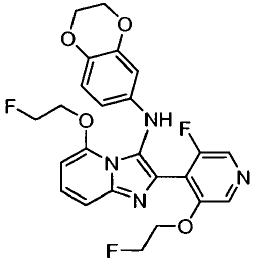

Prepared according to GP10 using 2-(3,5-difluoropyridin-4-yl)-N-(2,3-dihydrobenzo[b][1,4]dioxin-6-yl)-5-fluoroimidazo[1,2-a]pyridin-3-amine (0.25 mmol, 100 mg) and fluoroethanol (1.25 mmol, 80 mg) in THF (1 mL). Purified by flash chromatography (eluent: 5% MeOH in CH₂Cl₂) yielding the title compound (54 mg, 44%).

¹H NMR (400 MHz, CD₃OD) δ 8.12 (d, *J* = 9.5, 2H), 7.31 - 7.22 (m, 1H), 7.20 - 7.11 (m, 1H), 6.54 (d, *J* = 8.6, 1H), 6.19 (d, *J* = 7.5, 1H), 6.00 (dd, *J* = 2.7, 8.6, 1H), 5.95 (d, *J =* 2.7, 1H), 4.72 - 4.68 (m, 1H), 4.58 (dd, *J =* 3.2, 4.8, 1 H), 4.47 (dd, *J =* 3.1, 4.9, 1H), 4.41 - 4.36 (m, 1H), 4.35 (dd, *J =* 3.1, 5.0, 1 H), 4.33 - 4.29 (m, 1H), 4.29 - 4.24 (m, 1H), 4.20 (dd, *J* = 3.0, 5.0, 1H), 4.15 - 4.01 (m, 4H).

LCMS *m*/*z* 487 [M+H]⁺, purity (UV/MS) 98/60.

### 2-(3,5-Difluoropyridin-4-yl)-N-(2,3-dihydrobenzo[b][1,4]dioxin-6-yl)-5-(2-fluoroethoxy)imidazo[1,2-a]pyridin-3-amine

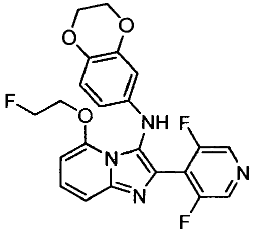

Prepared according to GP10 using 2-(3,5-difluoropyridin-4-yl)-N-(2,3-dihydrobenzo[b][1,4]dioxin-6-yl)-5-fluoroimidazo[1,2-a]pyridin-3-amine (0.25 mmol, 100 mg) and fluoroethanol (0.25 mmol, 16 mg) in THF (1 mL). Purified by flash chromatography (eluent: 5% MeOH in CH₂Cl₂) yielding the title compound (47 mg, 43%).

¹H NMR (400 MHz, CDCl₃) δ 8.27 (s, 2H), 7.27 - 7.18 (m, 1H), 7.17 - 7.05 (m, 1H), 6.59 (d, *J* = 8.6, 1H), 6.05 - 5.83 (m, 3H), 5.46 (s, 1H), 4.54 - 4.41 (m, 1H), 4.41 - 4.30 (m, 1H), 4.30 - 4.15 (m, 1H), 4.15 - 3.98 (m, 5H).

LCMS *m*/*z* 443 [M+H]⁺, purity (UV/MS) 98/56.

### 4-(3-(2,3-Dihydrobenzo[b][1,4]dioxin-6-ylamino)-5-(2-fluoroethoxy)imidazo[1,2-a]pyridin-2-yl)-3,5-difluorophenol

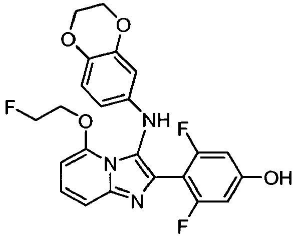

Prepared according to GP10 using 4-(3-(2,3-dihydrobenzo[b][1,4]dioxin-6-ylamino)-5-fluoroimidazo[1,2-a]pyridin-2-yl)-3,5-difluorophenol (0.15 mmol, 61 mg) and fluoroethanol (0.75 mmol, 48 mg) in THF (1 mL). The title compound precipitated out of the crude reaction mixture and was collected by filtration, washed with MeOH (54 mg, 79%).

¹H NMR (400 MHz, DMSO) δ 7.14 - 7.05 (m, 1H), 7.05 - 6.99 (m, 1H), 6.91 (s, 1H), 6.45 (d, *J* = 8.6, 1H), 6.13 (d, *J* = 7.3, 1H), 5.88 (d, *J* = 8.7, 1H), 5.85 - 5.71 (m, 2H), 4.49 - 4.41 (m, 1H), 4.37 - 4.28 (m, 1H), 4.26 - 4.18 (m, 1H), 4.16 - 4.10 (m, 1H), 4.08 - 3.93 (m, 4H).

>

LCMS m/z 443 [M+H]+, purity (UV/MS) 95/50.

### 1-(2-(2-chloro-6-fluorophenyl)-5-methoxyimidazo[1,2-a]pyridin-3-yl)-3-(2,3-dihydrobenzo[b][1,4]dioxin-6-yl)urea

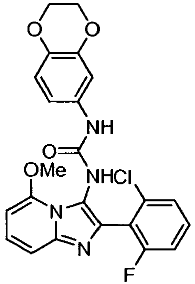

A vial was charged with 2-(2-Chloro-6-fluorophenyl)-5-methoxyimidazo[1,2-a]pyridin-3-amine (0.06 mmol, 18 mg) and 6-isocyanato-1,4-benzodioxane (0.06 mmol, 11 mg) in CH₂Cl₂ (0.5 mL). The vial was sealed and shaken at 50 °C for 16 h. The mixture was evaporated to dryness and then the crude product was taken up in methanol (1 mL). Upon stading the title compound precipitated and could be collected by filtration (16.2 mg, 58%).

¹H NMR (400 MHz, CD₃OD) δ 8.59 (s, 1H), 7.85 (s, 1H), 7.54-7.45 (m, 1H), 7.44-7.39 (m, 1H), 7.36-7.24 (m, 2H), 7.17 (d, *J* = 8.9 Hz, 1H), 6.99 (s, 1H), 6.71 (s, 2H), 6.34 (d, *J* = 7.6 Hz, 1H), 4.25-4.10 (m, 4H), 3.92 (s, 3H).

LCMS *m*/*z* 469 [M+H]⁺, purity (UV/MS) 100/94.

### 1-(2-(2-chloro-6-fluorophenyl)-5-methoxyimidazo[1,2-a]pyridin-3-yl)-3-(2,3-dihydrobenzo[b][1,4]dioxin-6-yl)urea

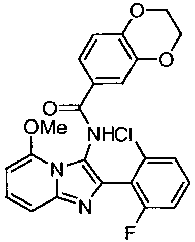

2-(2-Chloro-6-fluorophenyl)-5-methoxyimidazo[1,2-a]pyridin-3-amine (0.06 mmol, 18 mg) was weighed into a vial, then pyridine (0.06 mmol, 5 mg) and 2,3-Dihydro-1,4-benzodioxine-6-carbonyl chloride (0.07 mmol, 14 mg) was added. The reaction mixture was heated on a shaker at 50 °C for 16 h. The mixture was concentrated in vacuo and purified by preparative LCMS to yield the title compound (3.1 mg, 11%).

LCMS *m*/*z* 454 [M+H]⁺, purity (UV/MS) 93/84.

### 2-(3-(2,3-Dihydrobenzo[b][1,4]dioxin-6-ylamino)-2-(4-hydroxy-2,6-dimethylphenyl)imidazo-[1,2-a]pyridin-5-yloxy)ethyl 4-methylbenzenesulfonate

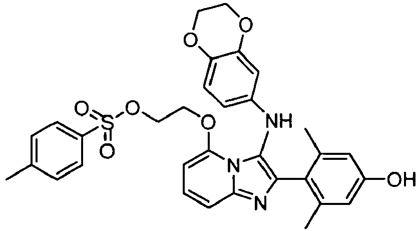

TBAF (0.36 mmol, 1M, 360 µL) and acetic acid (0.36 mmol, 22 mg) mixed and taken up in THF (2 mL), then added to 2-(2-(4-(*t-*butyldimethylsilyloxy)-2,6-dimethylphenyl)-3-(2,3-dihydrobenzo[b][1,4]dioxin-6-ylamino)imidazo[1,2-a]pyridin-5-yloxy)ethyl 4-methyl-benzenesulfonate (0.18 mmol, 130 mg) in THF (8 mL). The reaction was left at rt for 2h, then quenched with NH₄Cl aq. and extracted with DCM, washed with water and dried over Na₂SO₄ and concentrated *in vacuo.* Purified by flash chromatography (eluent: 2-5% MeOH in DCM) to yiled the title compound (62 mg, 57%).

¹H NMR (400 MHz, CD₃OD) δ 7.73 (d, *J*= 7.7, 2H), 7.43 - 7.38 (m, 1H), 7.33 (d, *J*= 7.9, 2H), 7.14 - 7.08 (m, 2H), 6.51 - 6.45 (m, 3H), 6.04 - 5.86 (m, 3H), 4.15 - 4.05 (m, 6H), 4.03 - 3.95 (m, 2H), 2.42 (s, 3H), 2.05 (s, 6H).

LCMS *m*/*z* 602 [M+H]⁺, purity (UV/MS) 83/43.

### 2-(2-(2,6-Difluoro-4-hydroxyphenyl)-3-(2,3-dihydrobenzo[b][1,4]dioxin-6-ylamino)imidazo-[1,2-a]pyridin-5-yloxy)ethyl 4-methylbenzenesulfonate

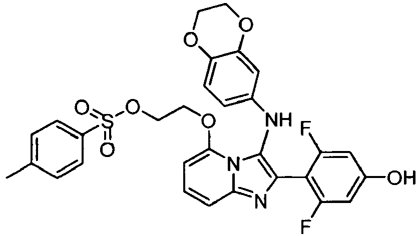

TBAF (0.48 mmol, 1M, 480 µL) and acetic acid (0.48 mmol, 29 mg) mixed and taken up in THF (2 mL), then added to 2-(2-(4-(*t-*butyldimethylsilyloxy)-2,6-difluorophenyl)-3-(2,3-dihydrobenzo[b][1,4]dioxin-6-ylamino)imidazo[1,2-a]pyridin-5-yloxy)ethyl 4-methyl-benzenesulfonate (0.24 mmol, 176 mg) in THF (8 mL). The reaction was left at rt for 2h, then quenched with NH₄Cl aq. and extracted with DCM, washed with water and dried over Na₂SO₄ and concentrated *in vacuo.* Purified by flash chromatography (eluent: 2-5% MeOH in DCM) to yiled the title compound (104 mg, 72%).

¹H NMR (400 MHz, CD₃OD) δ 7.75 (d, *J* = 8.2, 2H), 7.30 - 7.18 (m, 4H), 7.14 - 7.05 (m, 1H), 6.55 - 6.48 (m, 1H), 6.39 - 6.29 (m, 2H), 5.94 - 5.82 (m, 3H), 5.25 (s, 1H), 4.20 - 4.02 (m, 8H), 2.36 (s, 3H).

LCMS *m*/*z* 610 [M+H]⁺, purity (UV/MS) 80/40.

### 2-(2-(3,5-Difluoropyridin-4-yl)-3-(2,3-dihydrobenzo[b][1,4]dioxin-6-ylamino)imidazo[1,2-a]pyridin-5-yloxy)ethyl 4-methylbenzenesulfonate

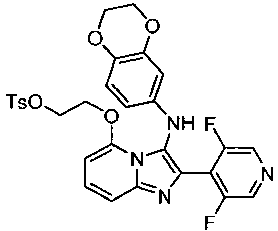

The mixture containing 2-(2-(3,5-difluoropyridin-4-yl)-3-(2,3-dihydrobenzo[b][1,4]dioxin-6-ylamino)imidazo[1,2-a]pyridin-5-yloxy)ethanol (418 mg) was dissolved in DCM and TsCI (217.1 mg, 1.14 mmol) and Et₃N (0.66 mL) was added. Stirred 3 days at room temperature before brine was added and the mixture shaken and separated. The organic phase was dried, concentrated, and purified by flash chromatography (silica, 0-5% MeOH/DCM) to give 97.6 mg of the desired product.

¹H NMR (400 MHz, CDCl₃) δ 8.34 (s, 2H), 7.75 (d, *J* = 8.3 Hz, 2H), 7.27 (d, *J* = 8.5 Hz, 2H), 7.23 (d, *J* = 8.7 Hz, 1H), 7.09 (dd, *J* = 7.5 Hz, 9.0 Hz, 1H), 6.51 (d, *J =* 8.3 Hz, 1H), 5.91 (dd, *J* = 2.6 Hz, 12.2 Hz, 2H), 5.86 (d, *J* = 7.4 Hz, 1H), 5.51 (s, 1H), 4.16 - 4.05 (m, 8H), 2.38 (s, 3H).

¹³C NMR (100 MHz, CDCl₃) δ 158.3, 155.7, 150.2, 145.7, 145.5, 144.2, 141.6, 136.9, 134.7, 134.4, 133.0, 130.1, 129.0, 127.8, 126.7, 125.3, 123.3, 117.6, 111.5, 107.0, 102.6, 90.3, 67.3, 67.0, 64.7, 64.2, 21.7.

LCMS *m*/*z* 595 [M+H]⁺, purity (UV/MS) 94/92.

### Library synthesis, in vials

General procedure GP11

### 4-(3-((2,3-dihydrobenzo[b][1,4]dioxin-6-yl)methylamino)-7-methylimidazo[1,2-a]pyridin-2-yl)-2-methoxyphenol

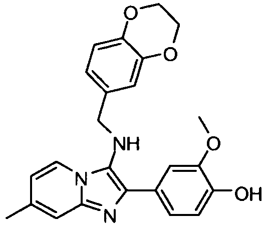

4-Methylpyridin-2-amine (0.12 mmol, 13 mg), vanillin (0.13 mmol, 20 mg) and 6-(isocyanomethyl)-2,3-dihydrobenzo[b][1,4]dioxine (0.10 mmol, 18 mg) were all weighed into a vial. 1,4-Dioxane (1 mL) and zinc chloride (cat) were added and the vial sealed. The reaction mixture was heated on a shaker at 90 °C for 16 h. The crude product was worked up by ion exchange using a SCX cartridge followed by purification by preparative LCMS. Yield 4.8 mg, (11%).

LCMS *m*/*z* 418 [M+H]⁺, purity (UV/MS) 96/40.

### 2-(2-chloro-6-fluorophenyl)-N-((2,3-dihydrobenzo[b][1,4]dioxin-6-yl)methyl)-7-methylimidazo[1,2-a]pyridin-3-amine

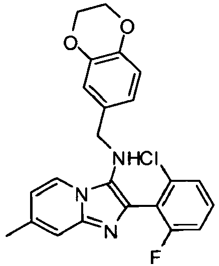

Prepared according to GP11 by using 4-methylpyridin-2-amine (0.12 mmol, 13 mg), 2-fluoro-6-methoxybenzaldehyde (0.13 mmol, 21 mg) and 6-(isocyanomethyl)-2,3-dihydrobenzo[b][1,4]dioxine (0.10 mmol, 18 mg) yielding the title compound (3.1 mg, 7%).

LCMS *m*/*z* 424 [M+H]⁺, purity (UV/MS) 100/60.

### 4-(3-((2,3-dihydrobenzo[b][1,4]dioxin-6-yl)methylamino)imidazo[1,2-a]pyridin-2-yl)-2-methoxyphenol

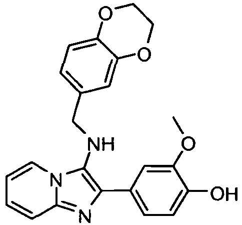

Prepared according to GP11 by using 2-aminopyridine (0.12 mmol, 12 mg), vanillin (0.13 mmol, 20 mg) and 6-(isocyanomethyl)-2,3-dihydrobenzo[b][1,4]dioxine (0.10 mmol, 18 mg) yielding the title compound (2.1 mg, 5%).

LCMS *m*/*z* 404 [M+H]⁺, purity (UV/MS) 96/40.

### 2-(2-chloro-6-fluorophenyl)-N-((2,3-dihydrobenzo[b][1,4]dioxin-6-yl)methyl)imidazo[1,2-a]pyridin-3-amine

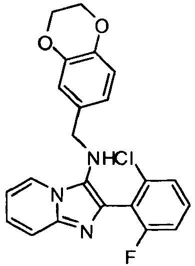

Prepared according to GP11 by using 2-aminopyridine (0.12 mmol, 12 mg), 2-fluoro-6-methoxybenzaldehyde (0.13 mmol, 21 mg) and 6-(isocyanomethyl)-2,3-dihydrobenzo[b][1,4]dioxine (0.10 mmol, 18 mg) yielding the title compound (1.4 mg, 3%).

LCMS *m*/*z* 410 [M+H]⁺, purity (UV/MS) 98/60.

### 4-(3-((2,3-dihydrobenzo[b][1,4]dioxin-6-yl)methylamino)-5-methylimidazo[1,2-a]pyridin-2-yl)-2-methoxyphenol

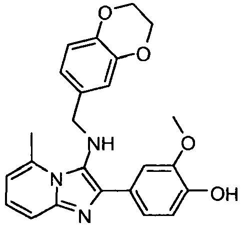

Prepared according to GP11 by using 6-methylpyridin-2-amine (0.12 mmol, 13 mg), vanillin (0.13 mmol, 20 mg) and 6-(isocyanomethyl)-2,3-dihydrobenzo[b][1,4]dioxine (0.10 mmol, 18 mg) yielding the title compound (3.4 mg, 8%).

LCMS *m*/*z* 418 [M+H]⁺, purity (UV/MS) 99/50.

>

### 2-(2-chloro-6-fluorophenyl)-N-((2,3-dihydrobenzo[b][1,4]dioxin-6-yl)methyl)-5-methylimidazo[1,2-a]pyridin-3-amine

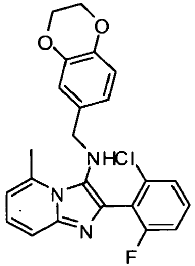

Prepared according to GP112 by using 6-methylpyridin-2-amine (0.12 mmol, 13 mg), 2-fluoro-6-methoxybenzaldehyde (0.13 mmol, 21 mg) and 6-(isocyanomethyl)-2,3-dihydrobenzo[b][1,4]dioxine (0.10 mmol, 18 mg) yielding the title compound (5.3 mg, 12%).

LCMS *m*/*z* 424 [M+H]⁺, purity (UV/MS) 98/50.

### 4-(3-((2,3-dihydrobenzo[b][1,4]dioxin-6-yl)methylamino)-6-methylimidazo[1,2-a]pyridin-2-yl)-2-methoxyphenol

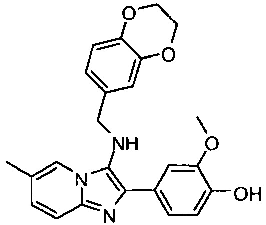

Prepared according to GP11 by using 5-methylpyridin-2-amine (0.12 mmol, 13 mg), vanillin (0.13 mmol, 20 mg) and 6-(isocyanomethyl)-2,3-dihydrobenzo[b][1,4]dioxine (0.10 mmol, 18 mg) yielding the title compound (4.2 mg, 10%).

LCMS *m*/*z* 418 [M+H]⁺, purity (UV/MS) 90/40.

### 2-(2-chloro-6-fluorophenyl)-N-((2,3-dihydrobenzo[b][1,4]dioxin-6-yl)methyl)-6-methylimidazo[1,2-a]pyridin-3-amine

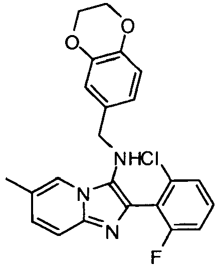

Prepared according to GP11 by using 5-methylpyridin-2-amine (0.12 mmol, 13 mg), 2-fluoro-6-methoxybenzaldehyde (0.13 mmol, 21 mg) and 6-(isocyanomethyl)-2,3-dihydrobenzo[b][1,4]dioxine (0.10 mmol, 18 mg) yielding the title compound (3.3 mg, 8%).

LCMS *m*/*z* 424 [M+H]⁺, purity (UV/MS) 100/70.

### 2-(2-chloro-6-fluorophenyl)-N-((2,3-dihydrobenzo[b][1,4]dioxin-6-yl)methyl)-8-methylimidazo[1,2-a]pyridin-3-amine

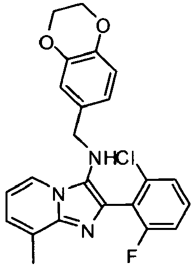

Prepared according to GP11 by using 3-methylpyridin-2-amine (0.12 mmol, 13 mg), 2-fluoro-6-methoxybenzaldehyde (0.13 mmol, 21 mg) and 6-(isocyanomethyl)-2,3-dihydrobenzo[b][1,4]dioxine (0.10 mmol, 18 mg) yielding the title compound (0.5 mg, 1%).

LCMS *m*/*z* 424 [M+H]⁺, purity (UV/MS) 95/50.

### 4-(3-((2,3-dihydrobenzo[b][1,4]dioxin-6-yl)methylamino)imidazo[1,2-a]pyrazin-2-yl)-2-methoxyphenol

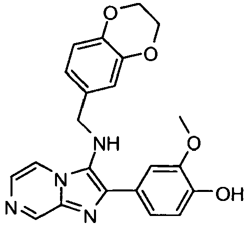

Prepared according to GP11 by using pyrazin-2-amine (0.12 mmol, 12 mg), vanillin (0.13 mmol, 20 mg) and 6-(isocyanomethyl)-2,3-dihydrobenzo[b][1,4]dioxine (0.10 mmol, 18 mg) yielding the title compound (1.0 mg, 3%).

LCMS *m*/*z* 405 [M+H]⁺, purity (UV/MS) 100/50.

### 2-(2-chloro-6-fluorophenyl)-N-((2,3-dihydrobenzo[b][1,4]dioxin-6-yl)methyl)imidazo[1,2-a]pyrazin-3-amine

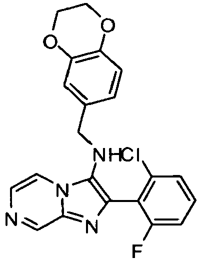

Prepared according to GP11 by using pyrazin-2-amine (0.12 mmol, 12 mg), 2-fluoro-6-methoxybenzaldehyde (0.13 mmol, 21 mg) and 6-(isocyanomethyl)-2,3-dihydrobenzo[b][1,4]dioxine (0.10 mmol, 18 mg) yielding the title compound (0.6 mg, 1%).

LCMS *mlz* 411 [M+H]⁺, purity (UV/MS) 94/40.

### 4-(3-((2,3-dihydrobenzo[b][1,4]dioxin-6-yl)methylamino)imidazo[1,2-a]pyrimidin-2-yl)-2-methoxyphenol

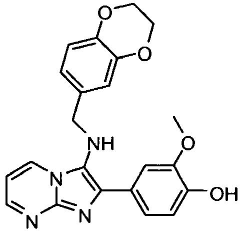

Prepared according to GP11 by using pyrimidin-2-amine (0.12 mmol, 12 mg), vanillin (0.13 mmol, 20 mg) and 6-(isocyanomethyl)-2,3-dihydrobenzo[b][1,4]dioxine (0.10 mmol, 18 mg) yielding the title compound (0.7 mg, 2%).

LCMS *m*/*z* 405 [M+H]⁺, purity (UV/MS) 95/50.

### Library synthesis, 96 well plates

### General Procedure GP12

The isonitrile (0.08 mmol), aldehyde (0.11 mmol) and the amine (0.07 mmol) were dissolved in MeOH and HOAc (200%) was added. The 96 well plate was shaken overnight at room temperature, and then at 40 °C for 12 h. After evaporation of the solvents and purification by preparative LCMS the products were obtained.

### General Procedure GP 13

The isonitrile (0.05 mmol), aldehyde (0.05 mmol) and the amine (0.05 mmol) were dissolved in 1,4-dioxane and ZnCl₂ (10%) was added to each well. The 96 well plate was shaken at 90 °C for 24 h. The product was worked up by passing the crude material through a SCX cartridge, that was subsequently eluded with NH₃(MeOH). After evaporation of the solvents and purification by preparative LCMS the products were obtained.

The following compounds were prepared as described in GP12:

4-(3-(2.3-dihydrobenzo[b][1,4]dioxin-6-ylamino)-7-methylimidazo[1,2-a]pyridin-2-yl)-2-methoxphenol Amount made: 2.0 mg. LCMS *m*/*z* 404 [M+H]⁺, purity (UV/MS) 96/70.

N-(2,3-dihydrobenzo[b][1,4]dioxin-6-yl)-7-methyl-2-(pyridin-2-yl)imidazo[1,2-a]pyridin-3-amine Amount made: 11.8 mg. LCMS *m*/*z* 359 [M+H]⁺, purity (UV/MS) 100/100.

2-(2,6-dichlorophenyl)-N-(2,3-dihydrobenzo[b][1,4]dioxin-6-yl)-7-methylimidazo[1_{,}2-a]pyridin-3-amine Amount made: 3.6 mg. LCMS *m*/*z* 426 [M+H]⁺, purity (UV/MS) 99/80.

N-(2,3-dihydrobenzo[b][1,4]dioxin-6-yl)-2-(2,6-dimethoxyphenyl)-7-methylimidazo[1,2-a]pyridin-3-amine Amount made: 4.9 mg. LCMS *m*/*z* 418 [M+H]⁺, purity (UV/MS) 100/100.

4-(3-(2,3-dihydrobenzo[b][1,4]dioxin-6-ylamino)-7-methylimidazo[1,2-a]pyridin-2-yl)-3-methoxyphenol Amount made: 12.9 mg. LCMS *m*/*z* 404 [M+H]⁺, purity (UV/MS) 94/90.

N-(2,3-dihydrobenzo[b][1,4]dioxin-6-yl)-2-(pyridin-2-yl)imidazo[1,2-a]pyridin-3-amine Amount made: 2.2 mg. LCMS *m*/*z* 345 [M+H]⁺, purity (UV/MS) 100/90.

N-(2,3-dihydrobenzo[b]1,4]dioxin-6-yl)-2-(2,6-dimethoxyphenyl)imidazo[1,2-a]pyridin-3-amine Amount made: 10.6 mg. LCMS *m*/*z* 404 [M+H]⁺, purity (UV/MS) 99/90.

2-(2,6-difluorophenyl)-N-(2,3-dihydrobenzo[b][1,4]dioxin-6-yl)imidazo[1,2-a]pyridin-3-amine Amount made: 3.9 mg. LCMS *m*/*z* 380 [M+H]⁺, purity (UV/MS) 96/70.

N-(2,3-dihydrobenzo[b][1,4]dioxin-6-yl)-2-(2,6-dimethylphenyl)imidazo[1,2-a]pyridin-3-amine Amount made: 4.0 mg. LCMS *m*/*z* 372 [M+H]⁺, purity (UV/MS) 92/90.

4-(3-(2,3-dihydrobenzo[b][1,4]dioxin-6-ylamino)imidazo[1,2-a]pyridin-2-yl)-3-methoxyphenol Amount made: 6.8 mg. LCMS *m*/*z* 390 [M+H]⁺, purity (UV/MS) 96/80.

### 2-(3,5-dichloropyridin-4-yl)-N-(2,3-dihydrobenzo[b][1,4]dioxin-6-yl)imidazo[1,2-a]pyridin-3-amine

Amount made: 1.0 mg. LCMS *m*/*z* 413 [M+H]⁺, purity (UV/MS) 95/70.

4-(3-(2,3-dihydrobenzo[b][1,4]dioxin-6-ylamino)-5-methylimidazo[1,2-a]pyridin-2-yl)-2-methoxyphenol Amount made: 2.2 mg. LCMS *m*/*z* 404 [M+H]⁺, purity (UV/MS) 96/80.

N-(2,3-dihydrobenzo[b][1,4]dioxin-6-yl)-5-methyl-2-(pyridin-2-yl)imidazo[1,2-a]pyridin-3-amine Amount made: 2.0 mg. LCMS *m*/*z* 359 [M+H]⁺, purity (UV/MS) 1·00/100.

2-(2,6-dichlorophenyl)-N-(2,3-dihydrobenzo[b][1,4]dioxin-6-yl)-5-methylimidazo[1,2-a]pyridin-3-amine Amount made: 5.5 mg. LCMS *m*/*z* 426 [M+H]⁺, purity (UV/MS) 94/70.

N-(2,3-dihydrobenzo[b][1,4]dioxin-6-yl)-2-(2,6-dimethoxyphenyl)-5-methylimidazo[1,2-a]pyridin-3-amine Amount made: 14.6 mg. LCMS *m*/*z* 418 [M+H]⁺, purity (UV/MS) 100/90.

4-(3-(2,3-dihydrobenzo[b][1,4]dioxin-6-ylamino)-5-methylimidazo[1,2-a]pyridin-2-yl)-3-methoxyphenol Amount made: 7.4 mg. LCMS *m*/*z* 404 [M+H]⁺, purity (UV/MS) 100/80.

4-(3-(2,3-dihydrobenzo[b][1,4]dioxin-6-ylamino)-6-methylimidazo[1,2-a]pyridin-2-yl)-2-methoxyphenol Amount made: 2.8 mg. LCMS *m*/*z* 404 [M+H]⁺, purity (UV/MS) 98/80.

N-(2,3-dihydrobenzo[b][1,4]dioxin-6-yl)-6-methyl-2-(pyridin-2-yl)imidazo[1,2-a]pyridin-3-amine Amount made: 2.0 mg. LCMS *m*/*z* 359 [M+H]⁺, purity (UV/MS) 100/100.

2-(2,6-dichlorophenyl)-N-(2,3-dihydrobenzo[b][1,4]dioxin-6-yl)-6-methylimidazo[1,2-a]pyridin-3-amine Amount made: 4.7 mg. LCMS *m*/*z* 426 [M+H]⁺, purity (UV/MS) 98/80.

N-(2,3-dihydrobenzo[b][1,4]dioxin-6-yl)-2-(2,6-dimethoxyphenyl)-6-methylimidazo[1,2-a]pyridin-3-amine Amount made: 19.3 mg. LCMS *m*/*z* 418 [M+H]⁺, purity (UV/MS) 100/100.

2-(2-chloro-6-fluorophenyl)-N-(2,3-dihydrobenzo[b][1,4]dioxin-6-yl)-6-methylimidazo[1,2-a]pyridin-3-amine Amount made: 13.4 mg. LCMS *m*/*z* 410 [M+H]⁺, purity (UV/MS) 94/90.

2-(2,6-difluorophenyl)-N-(2,3-dihydrobenzo[b][1,4]dioxin-6-yl)-6-methylimidazo[1,2-a]pyridin-3-amine Amount made: 1.8 mg. LCMS *m*/*z* 394 [M+H]⁺, purity (UV/MS) 98/80.

N-(2,3-dihydrobenzo[b][1,4]dioxin-6-yl)-2-(2,6-dimethylphenyl)-6-methylimidazo[1,2-a]pyridin-3-amine Amount made: 3.7 mg. LCMS *m*/*z* 386 [M+H]⁺, purity (UV/MS) 96/70.

2-(2-chloro-6-nitrophenyl)-N-(2,3-dihydrobenzo[b][1,4]dioxin-6-yl)-6-methylimidazo[1,2-a]pyridin-3-amine Amount made: 4.8 mg. LCMS *m*/*z* 437 [M+H]⁺, purity (UV/MS) 97/90.

4-(3-(2,3-dihydrobenzo[b][1,4]dioxin-6-ylamino)-6-methylimidazo[1,2-a]pyridin-2-yl)-3-methoxyphenol Amount made: 17.3 mg. LCMS *m*/*z* 404 [M+H]⁺, purity (UV/MS) 98/90.

4-(3-(2,3-dihydrobenzo[b][1,4]dioxin-6-ylamino)-8-methylimidazo[1,2-a]pyridin-2-yl)-2-methoxyphenol Amount made: 4.7 mg. LCMS *m*/*z* 404 [M+H]⁺, purity (UV/MS) 99/80.

N-(2,3-dihydrobenzo[b][1,4]dioxin-6-yl)-8-methyl-2-(pyridin-2-yl)imidazo[1,2-a]pyridin-3-amine Amount made: 6.2 mg. LCMS *m*/*z* 359 [M+H]⁺, purity (UV/MS) 100/80.

N-(2,3-dihydrobenzo[b][1,4]dioxin-6-yl)-2-(2,6-dimethoxyphenyl)-8-methylimidazo[1,2-a]pyridin-3-amine Amount made: 9.5 mg. LCMS *m*/*z* 418 [M+H]⁺, purity (UV/MS) 100/100.

4-(3-(2,3-dihydrobenzo[b][1,4]dioxin-6-ylamino)-8-methylimidazo[1,2-a]pyridin-2-yl)-3-methoxyphenol Amount made: 6.7 mg. LCMS *m*/*z* 404 [M+H]⁺, purity (UV/MS) 95/90.

4-(3-(2,3-dihydrobenzo[b][1,4]dioxin-6-ylamino)imidazo[1,2-a]pyrazin-2-yl)-2-methoxyphenol Amount made: 2.1 mg. LCMS *m*/*z* 391 [M+H]⁺, purity (UV/MS) 100/100.

2-(2,6-dichlorophenyl-N-(2,3-dihydrobenzo[b][1,4]dioxin-6-yl)imidazo[1,2-a]pyrazin-3-amine Amount made: 1.4 mg. LCMS *m*/*z* 413 [M+H]⁺, purity (UV/MS) 85/70.

N-(2,3-dihydrobenzo[b][1,4]dioxin-6-yl)-2-(2,6-dimethoxyphenyl)imidazo[1,2-a]pyrazin-3-amine Amount made: 17.3 mg. LCMS *m*/*z* 405 [M+H]⁺, purity (UV/MS) 100/90.

2-(2,6-difluorophenyl)-N-(2,3-dihydrobenzo[b][1,4]dioxin-6-yl)imidazo[1,2-a]pyrazin-3-amine Amount made: 1.0 mg. LCMS *m*/*z* 381 [M+H]⁺, purity (UV/MS) 97/70.

N-(2,3-dihydrobenzo[b][1,4]dioxin-6-yl)-2-(2,6-dimethylphenyl)imidazo[1,2-a]pyrazin-3-amine Amount made: 2.1 mg. LCMS *m*/*z* 373 [M+H]⁺, purity (UV/MS) 100/100.

N-(2,3-dihydrobenzo[b][1,4]dioxin-6-yl)-2-(2-fluoro-6-methoxyphenyl)imidazo[1,2-a]pyrazin-3-amine Amount made: 1.0 mg. LCMS *m*/*z* 393 [M+H]⁺, purity (UV/MS) 93/90.

4-(3-(2,3-dihydrobenzo[b][1,4]dioxin-6-ylamino)imidazo[1,2-a]pyrimidin-2-yl)-2-methoxyphenol Amount made: 0.2 mg. LCMS *m*/*z* 391 [M+H]⁺, purity (UV/MS) 100/100.

N-(2,3-dihydrobenzo[b][1,4]dioxin-6-yl)-2-(pyridin-2-yl)imidazo[1,2-a]pyrimidin-3-amine Amount made: 0.7 mg. LCMS *m*/*z* 346 [M+H]⁺, purity (UV/MS) 97/70.

N-(2,3-dihydrobenzo[b][1,4]dioxin-6-yl)-2-(2,6-dimethoxyphenyl)imidazo[1,2-a]pyrimidin-3-amine Amount made: 8.3 mg. LCMS *m*/*z* 405 [M+H]⁺, purity (UV/MS) 100/90.

N-(2,3-dihydrobenzo[b][1,4]dioxin-6-yl)-2-(2,6-dimethylphenyl)imidazo[1,2-a]pyrimidin-3-amine Amount made: 0.4 mg. LCMS *m*/*z* 373 [M+H]⁺, purity (UV/MS) 100/100.

4-(3-(2,3-dihydrobenzo[b][1,4]dioxin-6-ylamino)imidazo[1,2-a]pyrimidin-2-yl)-3-methoxyphenol Amount made: 0.2 mg. LCMS *m*/*z* 391 [M+H]⁺, purity (UV/MS) 100/90.

N-(2,3-dihydrobenzo[b][1,4]dioxin-6-yl)-2-(2-fluoro-6-methoxyphenyl)imidazo[1,2-a]pyrimidin-3-amine Amount made: 0.3 mg. LCMS *m*/*z* 393 [M+H]⁺, purity (UV/MS) 100/100.

4-(3-(2,3-dihydrobenzo[b][1,4]dioxin-6-ylamino)imidazo[1,2-c]pyrimidin-2-yl)-2-methoxyphenol Amount made: 0.7 mg. LCMS *m*/*z* 391 [M+H]⁺, purity (UV/MS) 100/80.

N-(2,3-dihydrobenzo[b][1,4]dioxin-6-yl)-2-(2,6-dimethoxyphenyl)imidazo[1,2-c]pyrimidin-3-amine Amount made: 1.5 mg. LCMS *m*/*z* 405 [M+H]⁺, purity (UV/MS) 96/80.

4-(3-(2,3-dihydrobenzo[b][1,4]dioxin-6-ylamino)imidazo[1,2-c]pyrimidin-2-yl)-3-methoxyphenol Amount made: 1.5 mg. LCMS *m*/*z* 391 [M+H]⁺, purity (UV/MS) 94/90.

The following compounds were prepared as described in GP13:

4-(3-(benzo[d][1,3]dioxol-5-ylamino)-7-methylimidazo[1,2-a]pyridin-2-yl)-2-fluoro-6-methoxyphenol Amount made: 1.9 mg. LCMS *m*/*z* 408 [M+H]⁺, purity (UV/MS) 91/85.

3-(2-(2-chloro-6-fluorophenyl)imidazo[1,2-a]pyridin-3-ylamino)benzonitrile Amount made: 3.2 mg. LCMS *m*/*z* 363 [M+H]⁺, purity (UV/MS) 94/92.

4-(3-(benzo[d][1,3]dioxol-5-ylamino)imidazo[1,2-a]pyridin-2-yl)-2-fluoro-6-methoxyphenol Amount made: 3.2 mg. LCMS *m*/*z* 394 [M+H]⁺, purity (UV/MS) 95/64.

N-(benzo[d][1,3]dioxol-5-yl)-2-(2-chloro-6-fluorophenyl)-5-methylimidazo[1,2-a]pyridin-3-amine Amount made: 6.7 mg. LCMS *m*/*z* 396 [M+H]⁺, purity (UV/MS) 100/95.

4-(3-(benzo[d][1,3]dioxol-5-ylamino)-5-methylimidazo[1,2-a]pyridin-2-yl)-2-fluoro-6-methoxyphenol Amount made: 3.0 mg. LCMS *m*/*z* 408 [M+H]⁺, purity (UV/MS) 100/70.

4-(3-(benzo[d][1,3]dioxol-5-ylamino)-6-methylimidazo[1,2-a]pyridin-2-yl)-2-fluoro-6-methoxyphenol Amount made: 1.3 mg. LCMS *m*/*z* 408 [M+H]⁺, purity (UV/MS) 90/80.

N-(benzo[d][1,3]dioxol-5-yl)-2-(2-chloro-6-fluorophenyl)-8-methylimidazo[1,2-a]pyridin-3-amine Amount made: 1.6 mg. LCMS *m*/*z* 396 [M+H]⁺, purity (UV/MS) 95/64.

N-(2,3-dihydrobenzo[b][1,4]dioxin-6-yl-2-(3-fluoropyridin-2-yl)-7-methylimidazo[1,2-a]pyridin-3-amine Amount made: 1.0 mg. LCMS *m*/*z* 377 [M+H]⁺, purity (UV/MS) 100/90.

N-(2,3-dihydrobenzo[b][1,4]dioxin-6-yl)-2-(6-fluoropyridin-3-yl)-7-methylimidazo[1,2-a]pyridin-3-amine
Amount made: 1.9 mg. LCMS *m*/*z* 377 [M+H]⁺, purity (UV/MS) 63/50.

N-(2,3-dihydrobenzo[b][1,4]dioxin-6-yl)-2-(6-fluoropyridin-2-yl)-7-methylimidazo[1,2-a]pyridin-3-amine Amount made: 0.1 mg. LCMS *m*/*z* 377 [M+H]⁺, purity (UV/MS) 86/70.

4-(3-(2,3-dihydrobenzo[b][1,4]dioxin-6-ylamino)imidazo[1,2-a]pyridin-2-yl)-2-fluoro-6-methoxyphenol Amount made: 1.9 mg. LCMS *m*/*z* 408 [M+H]⁺, purity (UV/MS) 94/30.

N-(2,3-dihydrobenzo[b][1,4]dioxin-6-yl)-2-(3-fluoropyridin-2-yl)imidazo[1,2-a]pyridin-3-amine Amount made: 2.1 mg. LCMS *m*/*z* 363 [M+H]⁺, purity (UV/MS) 100/90.

N-(2,3-dihydrobenzo[b][1,4]dioxin-6-yl)-2-(6-fluoropyridin-3-yl)imidazo[1,2-a]pyridin-3-amine Amount made: 0.6 mg. LCMS *m*/*z* 363 [M+H]⁺, purity (UV/MS) 85/80.

N-(2,3-dihydrobenzo[b][1,4]dioxin-6-yl)-2-(6-fluoropyridin-2-yl)imidazo[1,2-a]pyridin-3-amine Amount made: 0.2 mg. LCMS *m*/*z* 363 [M+H]⁺, purity (UV/MS) 91/72.

4-(3-(2,3-dihydrobenzo[b][1,4]dioxin-6-ylamino)-5-methylimidazo[1,2-a]pyridin-2-yl)-2-fluoro-6-methoxyphenol Amount made: 0.3 mg. LCMS *m*/*z* 422 [M+H]⁺, purity (UV/MS) 87/60.

N-(2,3-dihydrobenzo[b][1,4]dioxin-6-yl)-2-(3-fluoropyridin-2-yl)-5-methylimidazo[1,2-a]pyridin-3-amine Amount made: 0.4 mg. LCMS *m*/*z* 377 [M+H]⁺, purity (UV/MS) 100/90.

N-(2,3-dihydrobenzo[b][1,4]dioxin-6-yl)-2-(6-fluoropyridin-2-yl)-5-methylimidazo[1,2-a]pyridin-3-amine Amount made: 0.2 mg. LCMS *m*/*z* 377 [M+H]⁺, purity (UV/MS) 66/50.

4-(3-(2,3-dihydrobenzo[b][1,4]dioxin-6-ylamino)-6-methylimidazo[1,2-a]pyridin-2-yl)-2-fluoro-6-methoxyphenol Amount made: 0.3 mg. LCMS *m*/*z* 422 [M+H]⁺, purity (UV/MS) 85/50.

N-(2,3-dihydrobenzo[b][1,4]dioxin-6-yl)-2-(3-fluoropyridin-2-yl)-6-methylimidazo[1,2-a]pyridin-3-amine
Amount made: 0.6 mg. LCMS *m*/*z* 377 [M+H]⁺, purity (UV/MS) 100/90.

N-(2,3-dihydrobenzo[b][1,4]dioxin-6-yl)-2-(6-fluoropyridin-2-yl)-6-methylimidazo[1,2-a]pyridin-3-amine Amount made: 0.1 mg. LCMS *m*/*z* 377 [M+H]⁺, purity (UV/MS) 70/50.

### Radiofluorination Methods

### General Procedure 14 (GP14)

### Synthesis of CR-132

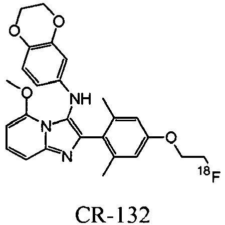

[¹⁸F]fluoroethyltosylate was prepared as described by Wester et al (J. Nucl. Med., 1999, 40, 205-212) and eluted from the dried tC18 Sep-Pak (lite) cartridge with anhydrous acetonitrile (0.5 mL) into a 1 mL glass Wheaton vial containing a stirred suspension of cesium carbonate (9 mg, 46 µmol), the hydroxyl precursor compound (2-3 mg, 5-7 µmol) and anhydrous acetonitrile (0.1 mL). The reaction mixture was heated to 135°C for 15 min. After cooling, the reaction mixture was diluted with water (0.5 mL) and the crude product applied to semi-preparative HPLC: Phenomenex Luna C18(2) column (100 x 10 mm i.d.), particle size 5 µm); mobile phase A: water, mobile phase B: acetonitrile; flow gradient: 3 mL/min; 0-1 min 40%B; 1-25 min 40-50%B; Wavelength 254nm; *t*_{R} CR-132 16 min, *t*_{R} [¹⁸F]fluoroethyltosylate 12 min. The CR-132 cut-peak was diluted to 10 mL with water and adsorbed on a tC 18 Sep-Pak (lite) cartridge. The cartridge was washed with water (5 mL), and CR-132 was eluted with ethanol (0.5 mL) and further formulated with PBS (5mL). The radiochemical yield was 14% non-decay corrected from the starting activity and the total reaction time was 180 minutes. Radiochemical purity was >95%.

### Synthesis of CR-133

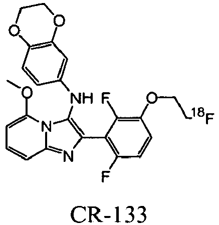

CR-133 was prepared according to GP14. The radiochemical yield was 9-14% non-decay corrected from the starting activity and the total reaction time was 180 minutes. Radiochemical purity was >95%.

### Synthesis of CR-134

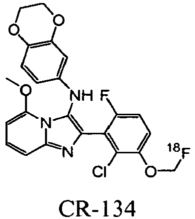

CR-134 was prepared according to GP14 wherein (¹⁸F]fluoromethyltosylate (Neal et al J. Label. Compd. Radiopharm., 2005, 48, 557-568) was used in place of [¹⁸F]fluoroethyltosylate. Radiochemical yield 9% non-decay corrected, total reaction time 148 minutes from ¹⁸F-water, radiochemical purity 98%, estimated specific activity 31GBq/µmol.

### General Procedure 15

### Synthesis of CR-135

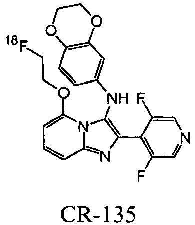

A mixture of Kryptofix 2.2.2 (2.5 mg, 7µmol), potassium bicarbonate (*ca*. 0.1 M, 35µL, 3.5µmol) and acetonitrile (0.5 mL) was added to [¹⁸F]F⁻ /H₂0 (*ca.* 400MBq, 100-300µL) in a COC reaction vessel. The solvent was removed by heating at 100°C under a stream of nitrogen for 15-20 minutes. The tosylate precursor compound (5 mg, 8 µmol) in acetonitrile (1mL) was labelled at 100°C/10mins. After cooling, the reaction solution was transferred by syringe to an empty vial, the reaction vessel was rinsed with water (1.5 mL) and combined with the crude product. The crude product was purified by semi-preparative HPLC: Phenomenex Luna C18(2) column (100 x 10 mm i.d.), particle size 5 µm); mobile phase A: aqueous 0.8% triethylamine (pH adjusted to 7.5 with H₃PO₄), mobile phase B: acetonitrile; flow gradient: 3 mL/min; 0-1 min 40%B; 1-25 min 40-95% B; Wavelength 254nm, *t*_{R} CR-135 15 min. The CR-135 cut-peak was diluted to a volume of *ca.* 10 mL with water and adsorbed on a tC18 Sep-Pak (lite) cartridge. The cartridge was washed with water (5 mL) before CR-135 was eluted using ethanol (0.5mL). The product was formulated with PBS (5mL). The radiochemical yield was 8% (n=1) non-decay corrected from the starting activity and the total reaction time was 90 minutes. Radiochemical purity was >95%.

### Example 2: CB2 Receptor Binding Assays

To show that CB2 compounds can block binding of a CB2 ligand to CB2 receptors the ability of compounds of Formula I to block binding of CB2 ligand CP 55,940 (2-[(1S,2R,5S)-5-hydroxy-2-(3-hydroxypropyl) cyclohexyl]-5-(2-methyloctan-2-yl)phenol; CAS No. 83002-04-4) was examined in HEK-293T cells as follows.

*Membrane preparation* - HEK-293T cells were cultured according to ATCC (Manassas, VA) guidelines and transfected with human CB2 cDNA (SEQ ID NO:1) (Genbank X74328), operably linked to the SV40 promoter, using Polyfect (Qiagen, Valencia, CA) or Fugene (Roche, Nutley, N.J.) according to manufacturer's instructions. 48 h after transfection cells were harvested in ice cold membrane buffer (20 mM HEPES, 6 mM MgCl₂, 1 mM EDTA, pH 7.2) using a cell scraper. Cells were transferred to a nitrogen cavitation chamber and a pressure of 900 bar was applied for 30 min. The pressure was released and the cell debris was collected and centrifuged at 1000 g at 4 °C for 10 min. The supernatant was collected and the spin was repeated until the supernatant was free of precipitate. Membranes were then pelleted by centrifugation at 12.000 g at 4 °C for 20 min. Membranes were resuspended in an appropriate amount of membrane buffer. The membrane concentration was determined using a BioRad (Hercules, CA) protein assay dye reagent according to manufacturer's instructions. Membranes were diluted to 1 mg/ml and aliquots snap-frozen in liquid nitrogen and store at -80°C.

*Binding assay* - 0.5- 10 µg of membranes were incubated in binding buffer (50 mM Tris, 0.5 mM EDTA, 0.1% BSA, pH 7.4) in the presence of 1.5 nM radioligand ([³H]-CP 55,940 Perkin Elmer) and varying concentrations of ligands (total volume 100 µL in a 96 well plate). Membranes were filtered onto a 96 well GF/B filterplate (Packard Bioscience, Shelton, CT) and washed with 500 mL wash buffer (25mM HEPES, 1 mM CaCl₂, 5 mM MgCl₂, 0.25M NaCl) using a Filtermate 196 Harvester (Packard Instruments, Downers Grove, IL). The filter plates were dried under a heat lamp before addition of 50 µL of scintillation fluid to each well (Microscint 20, Packard, Shelton, CT). Plates were counted on a Topcount NXT (Packard, Shelton, CT).

*Data Analysis* - Graphs were plotted and K_{D} values were determined by nonlinear regression analysis using Prism software (GraphPad version 4.0, San Diego, CA, USA).

### Table 1. Binding of CB2 compounds to native CB2 receptors

These results demonstrate that the compounds described herein bind with high affinity to native CB2 receptors.

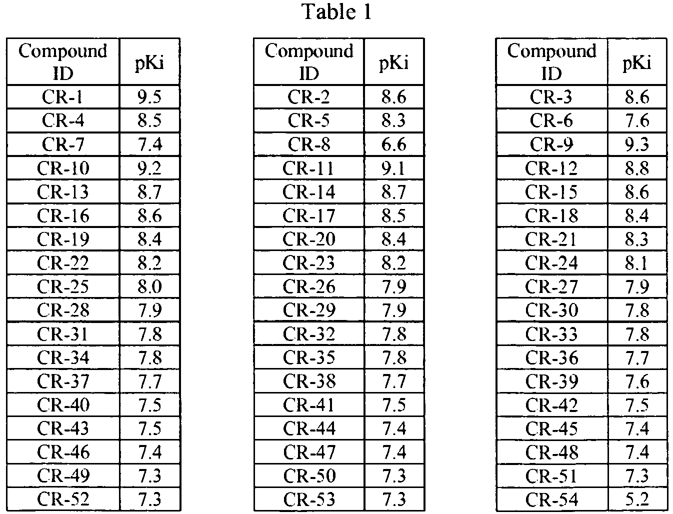

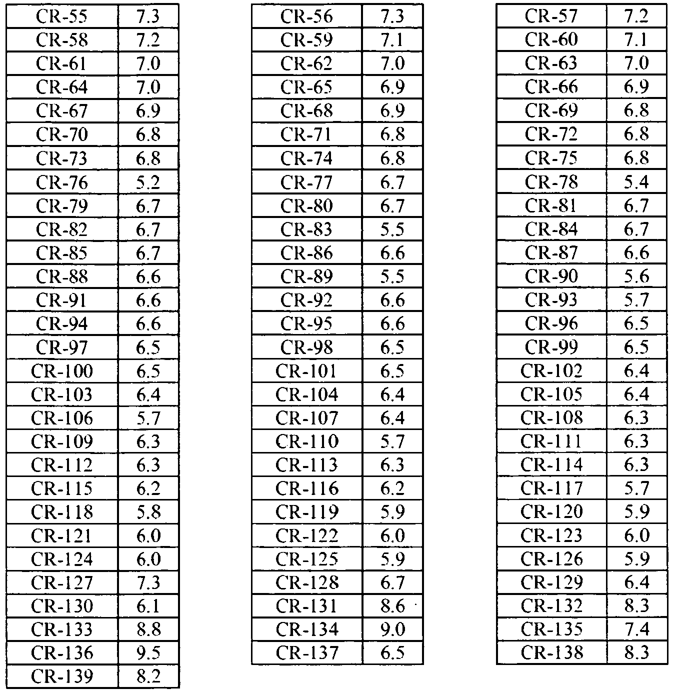

It will be appreciated that the CB2 receptor binding assay of the foregoing example may be used to identify compounds which are agonists, inverse agonists or antagonists of a CB2 receptor. The cannabinoid CB2 receptor used in the assay may consist essentially of SEQ ID NO:2. In further embodiments, the cannabinoid CB2 receptor used in the assay may have at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99% or greater than at least 99% amino acid identity with a full-length CB2 receptor.

### REFERENCES

The following references are referenced in the text and are incorporated by reference herein in their entirety.

Ashton JC, Rahman RM, Nair SM, Sutherland BA, Glass M, Appleton I. Cerebral hypoxia-ischemia and middle cerebral artery occlusion induce expression of the cannabinoid CB2 receptor in the brain. Neurosci Lett. 2007 Jan 29;412(2):114-7.

Beltramo M, Bernardini N, Bertorelli R, Campanella M, Nicolussi E, Fredduzzi S, Reggiani A. CB2 receptor-mediated antihyperalgesia: possible direct involvement of neural mechanisms. Eur J Neurosci. 2006 Mar;23(6):1530-8.

Benito C, Nunez E, Tolon RM, Carrier EJ, Rabano A, Hillard CJ, Romero J. Cannabinoid CB2 receptors and fatty acid amide hydrolase are selectively overexpressed in neuritic plaque-associated glia in Alzheimer's disease brains. J Neurosci. 2003 Dec 3;23(35):11136-41.

Benito C, Kim WK, Chavarria I, Hillard CJ, Mackie K, Tolon RM, Williams K, Romero J. A glial endogenous cannabinoid system is upregulated in the brains of macaques with simian immunodeficiency virus-induced encephalitis. J Neurosci. 2005 Mar 9;25(10):2530-6.

Benito C, Romero J, Tolon, R, Clemente D, Docagne F, Hillard, C, Guaza, C, Romero, J. Cannabinoid CB1 and CB2 Recptors and fatty acid amide hydrolase are specific markers of plaque cell subtypes in human multiple sclerosis. J Neurosci. 2007 Feb. 28, 27(9):2396-2402.

Cabral GA, Marciano-Cabral F. Cannabinoid receptors in microglia of the central nervous system: immune functional relevance. J Leukoc Biol. 2005 Dec;78(6):1192-7.

Carlisle SJ, Marciano-Cabral F, Staab A, Ludwick C, Cabral GA. Differential expression of the CB2 cannabinoid receptor by rodent macrophages and macrophage-like cells in relation to cell activation. Int Immunopharmacol. 2002 Jan;2(1):69-82.

Elmes SJ, Jhaveri MD, Smart D, Kendall DA, Chapman V. Cannabinoid CB2 receptor activation inhibits mechanically evoked responses of wide dynamic range dorsal horn neurons in naive rats and in rat models of inflammatory and neuropathic pain. Eur J Neurosci. 2004 Nov;20(9):2311-20.

Galiegue S, Mary S, Marchand J, Dussossoy D, Carriere D, Carayon P, Bouaboula M, Shire D, Le Fur G, Casellas P. Expression of central and peripheral cannabinoid receptors in human immune tissues and leukocyte subpopulations. Eur J Biochem. 1995 Aug 15;232(1):54-61.

Golech SA, McCarron RM, Chen Y, Bembry J, Lenz F, Mechoulam R, Shohami E, Spatz M. Human brain endothelium: coexpression and function of vanilloid and endocannabinoid receptors. Brain Res Mol Brain Res. 2004 Dec 6;132(1):87-92.

Gong JP, Onaivi ES, Ishiguro H, Liu QR, Tagliaferro PA, Brusco A, Uhl GR. Cannabinoid CB2 receptors: immunohistochemical localization in rat brain. Brain Res. 2006 Feb 3;1071(1):10-23

Howlett AC, Breivogel CS, Childers SR, Deadwyler SA, Hampson RE, Porrino LJ. Cannabinoid physiology and pharmacology: 30 years of progress. Neuropharmacology. 2004;47 Suppl 1:345-58

Ibrahim MM, Porreca F, Lai J, Albrecht PJ, Rice FL, Khodorova A, Davar G, Makriyannis A, Vanderah TW, Mata HP, Malan TP Jr. CB2 cannabinoid receptor activation produces antinociception by stimulating peripheral release of endogenous opioids. Proc Natl Acad Sci U S A. 2005 Feb 22;102(8):3093-8.

Karsak M, Cohen-Solal M, Freudenberg J, Ostertag A, Morieux C, Komak U, Essig J, Erxlebe E, Bab I, Kubisch C, de Vernejoul MC, Zimmer A. Cannabinoid receptor type 2 gene is associated with human osteoporosis. Hum Mol Genet. 2005 Nov 15;14(22):3389-96.

Klegeris A, Bissonnette CJ, McGeer PL. Reduction of human monocytic cell neurotoxicity and cytokine secretion by ligands of the cannabinoid-type CB2 receptor. Br J Pharmacol. 2003 Jun;139(4):775-86.

Lee SF, Newton C, Widen R, Friedman H, Klein TW. Downregulation of cannabinoid receptor 2 (CB2) messenger RNA expression during in vitro stimulation of murine splenocytes with lipopolysaccharide. Adv Exp Med Biol. 2001;493:223-8.

Martin RS, Luong LA, Welsh NJ, Eglen RM, Martin GR, MacLennan SJ. Effects of cannabinoid receptor agonists on neuronally-evoked contractions of urinary bladder tissues isolated from rat, mouse, pig, dog, monkey and human. Br J Pharmacol. 2000 Apr;129(8):1707-15.

Munro S, Thomas KL, Abu-Shaar M. Molecular characterization of a peripheral receptor for cannabinoids. Nature. 1993 Sep 2;365(6441):61-5.

Ofek O, Karsak M, Leclerc N, Fogel M, Frenkel B, Wright K, Tam J, Attar-Namdar M, Kram V, Shohami E, Mechoulam R, Zimmer A, Bab I. Peripheral cannabinoid receptor, CB2, regulates bone mass. Proc Natl Acad Sci U S A. 2006 Jan 17;103(3):696-701.

Onaivi ES, Ishiguro H, Gong JP, Patel S, Perchuk A, Meozzi PA, Myers L, Mora Z, Tagliaferro P, Gardner E, Brusco A, Akinshola BE, Liu QR, Hope B, Iwasaki S, Arinami T, Teasenfitz L, Uhl GR. Discovery of the presence and functional expression of cannabinoid CB2 receptors in brain. Ann N Y Acad Sci. 2006 Aug;1074:514-36.

Palazuelos J, Aguado T, Egia A, Mechoulam R, Guzman M, Galve-Roperh I. Non-psychoactive CB2 cannabinoid agonists stimulate neural progenitor proliferation. FASEB J. 2006 Nov;20(13):2405-7.

Patel HJ, Birrell MA, Crispino N, Hele DJ, Venkatesan P, Barnes PJ, Yacoub MH, Belvisi MG. Inhibition of guinea-pig and human sensory nerve activity and the cough reflex in guinea-pigs by cannabinoid (CB2) receptor activation. Br J Pharmacol. 2003 Sep;140(2):261-8.

Ross RA, Coutts AA, McFarlane SM, Anavi-Goffer S, Irving AJ, Pertwee RG, MacEwan DJ, Scott RH. Actions of cannabinoid receptor ligands on rat cultured sensory neurones: implications for antinociception. Neuropharmacology. 2001;40(2):221-32.

Skaper SD, Buriani A, Dal Toso R, Petrelli L, Romanello S, Facci L, Leon A.The ALIAmide palmitoylethanolamide and cannabinoids, but not anandamide, are protective in a delayed postglutamate paradigm of excitotoxic death in cerebellar granule neurons. Proc Natl Acad Sci U S A. 1996 Apr 30;93(9):3984-9.

Sipe JC, Arbour N, Gerber A, Beutler E. Reduced endocannabinoid immune modulation by a common cannabinoid 2 (CB2) receptor gene polymorphism: possible risk for autoimmune disorders. J Leukoc Biol. 2005 Jul;78(1):231-8.

Stander S, Schmelz M, Metze D, Luger T, Rukwied R. Distribution of cannabinoid receptor 1 (CB1) and 2 (CB2) on sensory nerve fibers and adnexal structures in human skin. J Dermatol Sci. 2005 Jun;38(3):177-88.

Ueda Y, Miyagawa N, Matsui T, Kaya T, Iwamura H. Involvement of cannabinoid CB(2) receptor-mediated response and efficacy of cannabinoid CB(2) receptor inverse agonist, JTE-907, in cutaneous inflammation in mice. Eur J Pharmacol. 2005 Sep 27;520(1-3):164-71.

Van Sickle MD, Duncan M, Kingsley PJ, Mouihate A, Urbani P, Mackie K, Stella N, Makriyannis A, Piomelli D, Davison JS, Marnett LJ, Di Marzo V, Pittman QJ, Patel KD, Sharkey KA. Identification and functional characterization of brainstem cannabinoid CB2 receptors. Science. 2005 Oct 14;310(5746):329-32.

Walczak JS, Pichette V, Leblond F, Desbiens K, Beaulieu P.Behavioral, pharmacological and molecular characterization of the saphenous nerve partial ligation: a new model of neuropathic pain. Neuroscience. 2005;132(4):1093-102.

Whiteside GT, Lee GP, Valenzano KJ. The role of the cannabinoid CB2 receptor in pain transmission and therapeutic potential of small molecule CB2 receptor agonists. Curr Med Chem. 2007;14(8):917-36.

Wotherspoon G, Fox A, McIntyre P, Colley S, Bevan S, Winter J. Peripheral nerve injury induces cannabinoid receptor 2 protein expression in rat sensory neurons. Neuroscience. 2005;135(1):235-45.

Yoshihara S, Morimoto H, Yamada Y, Abe T, Arisaka O. Cannabinoid receptor agonists inhibit sensory nerve activation in guinea pig airways. Am J Respir Crit Care Med. 2004 Nov 1;170(9):941-6.

Zhang J, Hoffert C, Vu HK, Groblewski T, Ahmad S, O'Donnell D. Induction of CB2 receptor expression in the rat spinal cord of neuropathic but not inflammatory chronic pain models. Eur J Neurosci. 2003 Jun;17(12):2750-4.

## Claims

1. A compound of Formula I: 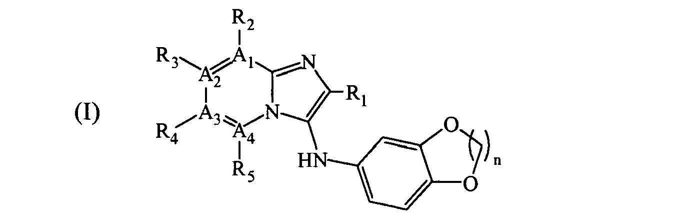 or a pharmaceutically acceptable salt thereof, wherein:
a) the 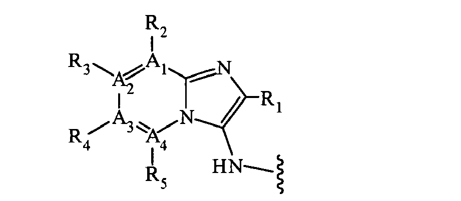 moiety is selected from the group consisting of 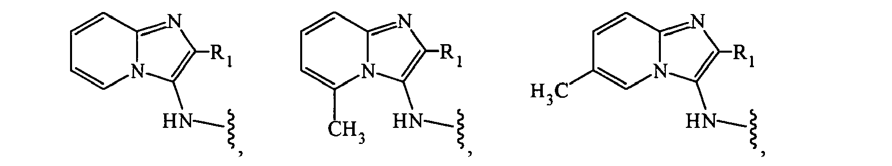 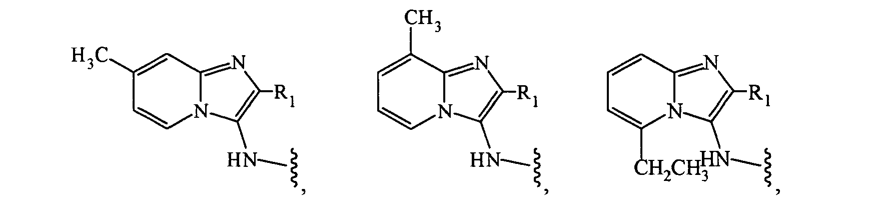 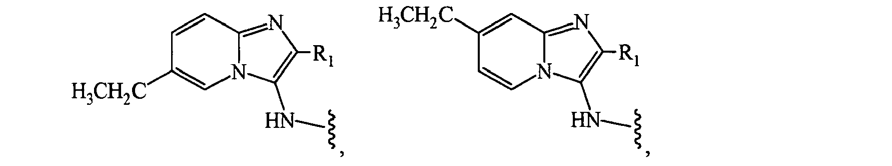 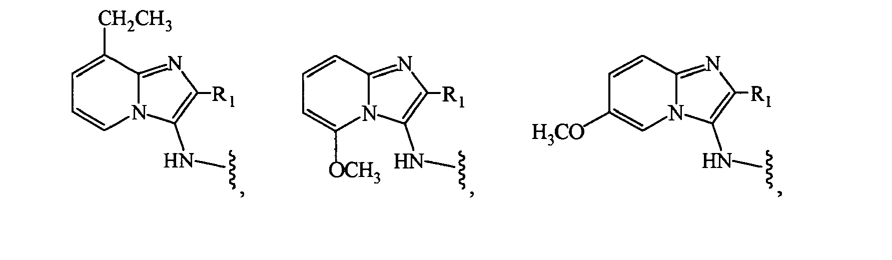 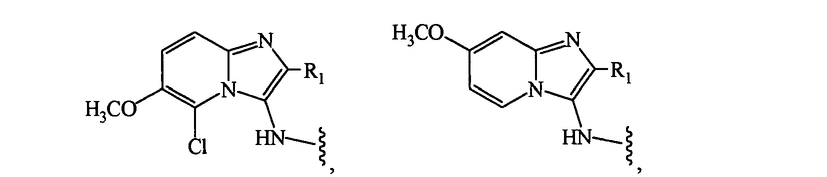 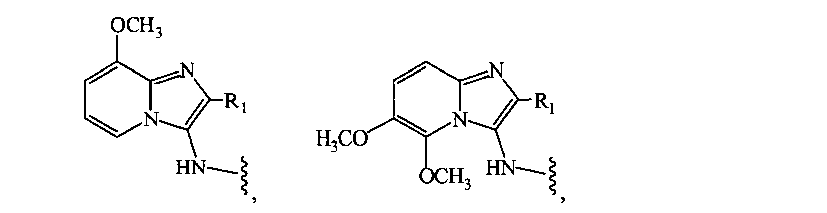 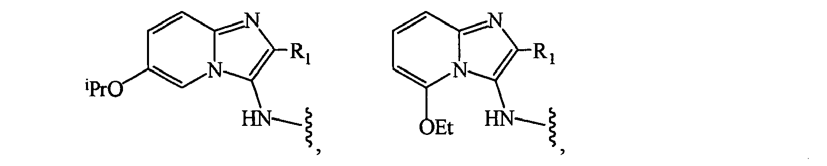 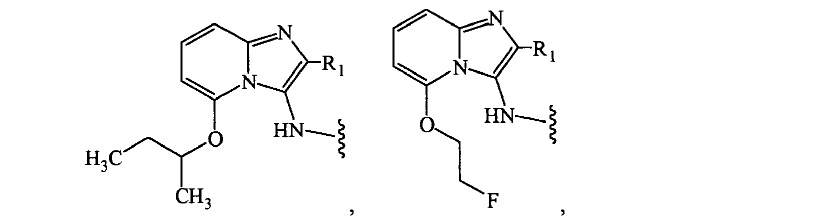 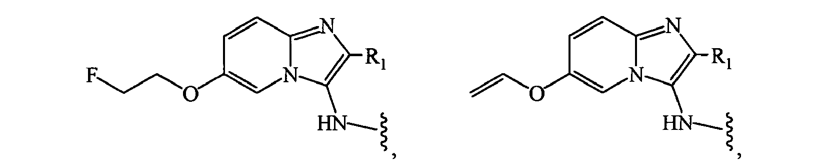 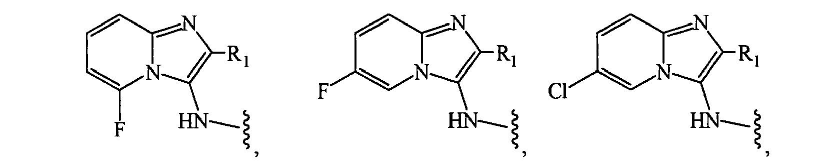 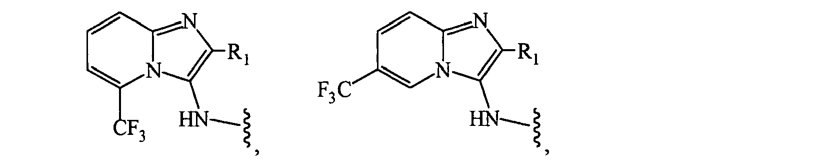 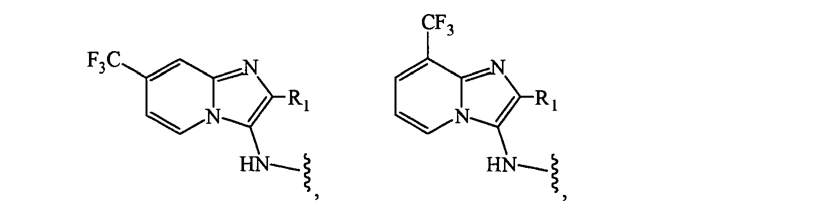 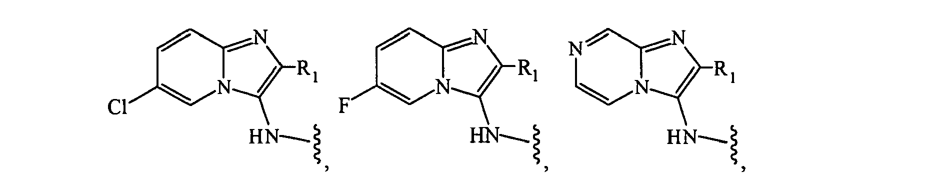 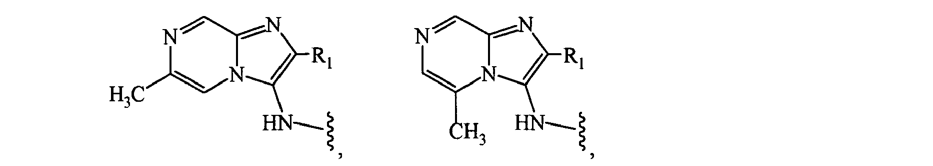 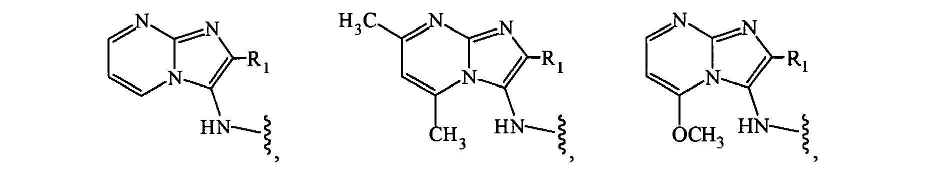 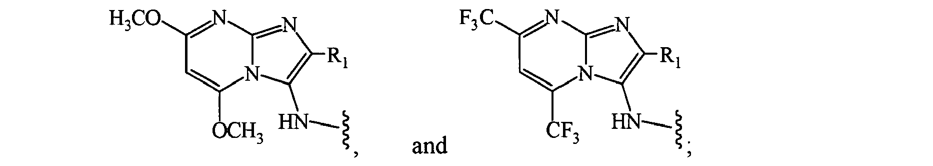
b) R₁ is selected from the group consisting of optionally substituted aryl, optionally substituted heteroaryl, optionally substituted carbocyclic ring, and optionally substituted heterocyclic ring;
c) n is 1 or 2; and,
d) at least one atom in the compound is a radioisotope;
and wherein, when a group of the compound of Formula I is described as optionally substituted, said group is unsubstituted, or substituted with one or more substituents independently selected from alkyl, alkenyl, alkynyl, cycloalkyl, cycloalkenyl, cycloalkynyl, aryl, heteroaryl, heteroalicyclyl, aralkyl, heteroaralkyl, (hetereoalicyclyl)alkyl, hydroxy, alkoxy, aryloxy, acyl, ester, mercapto, alkylthio, arylthio, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, and amino, including mono- and di-substituted amino groups.

2. The compound of claim 1, wherein the radioisotope is an isotope of hydrogen, carbon, nitrogen, oxygen, or halogen.

3. The compound of claim 2, wherein the halogen is fluorine or iodine.

4. The compound of claim 1 wherein the radioisotope is selected from ¹²³I, ¹¹C and ¹⁸F.

5. The compound of claim 4 wherein the radioisotope is selected from ¹¹C and ¹⁸F.

6. The compound of claim 4 wherein the radioisotope is ¹²³I.

7. The compound of claim 1, wherein R₁ is optionally substituted heteroaryl.

8. The compound of claim 7, wherein the optionally substituted heteroaryl is selected from the group consisting of furan, thiophene, phthalazinone, pyrrole, oxazole, thiazole, imidazole, pyrazole, isoxazole, isothiazole, triazole, thiadiazole, pyran, pyridine, pyridazine, pyrimidine, pyrazine and triazine.

9. The compound of claim 7, wherein the optionally substituted heteroaryl is pyridyl or thiophenyl.

10. The compound of claim 1, wherein R₁ is optionally substituted aryl.

11. The compound of claim 10, wherein the optionally substituted aryl is phenyl.

12. A pharmaceutical composition comprising the compound of any one of claims 1-11, and a physiologically acceptable component such as a carrier, a diluent, a salt or an excipient, or a combination thereof.

13. The compound of claim 5 for use in a method of PET imaging a first area of a tissue of a subject, the method comprising:
administering to the subject a pharmaceutical composition comprising a compound of Formula I as defined in claim 5;
measuring the signal emitted by the radioisotope from the first area of the tissue; and
comparing the amount of signal emitted from the first area of the tissue to an amount of signal emitted from a control sample.

14. The compound of claim 6 for use in a method of SPECT imaging a first area of a tissue of a subject, the method comprising:
administering to the subject a pharmaceutical composition comprising a compound of Formula I as defined in claim 6;
measuring the signal emitted by the radioisotope from the first area of the tissue; and
comparing the amount of signal emitted from the first area of the tissue to an amount of signal emitted from a control sample.

15. A method of determining a distribution of CB2 receptors in a tissue comprising administering a radiolabeled compound of Formula I as defined in Claim 1 to the tissue in vitro and obtaining an image of the tissue.

## Patentansprüche

1. Verbindung der Formel I: 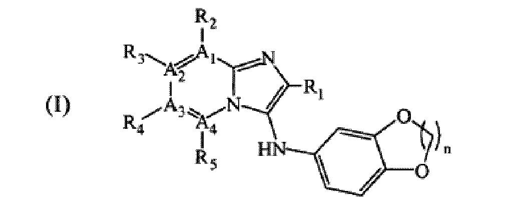 oder ein pharmazeutisch unbedenkliches Salz derselben, wobei:
a) die Einheit 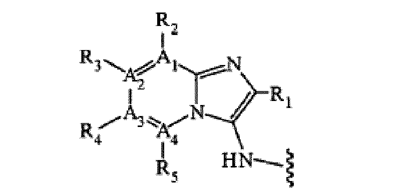 aus jener Gruppe gewählt ist, die besteht aus 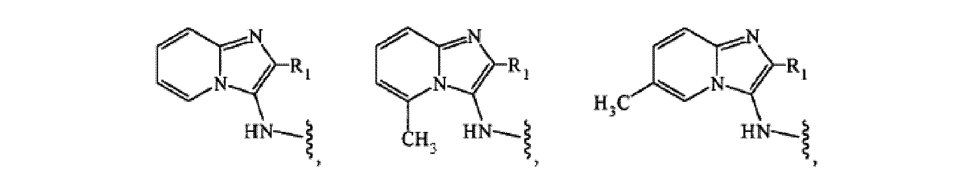 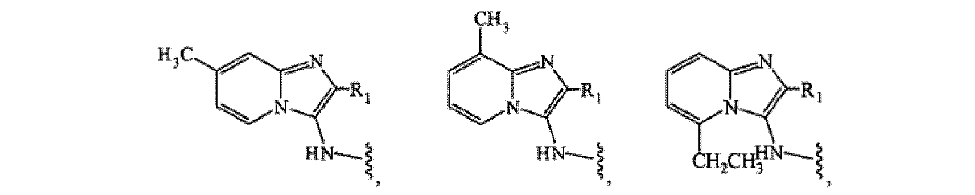 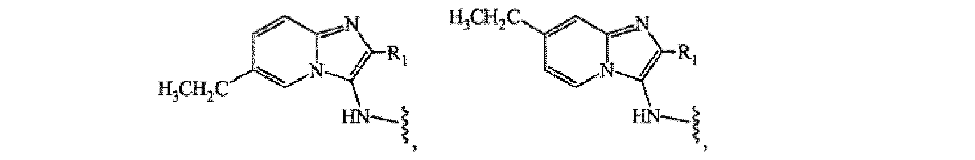 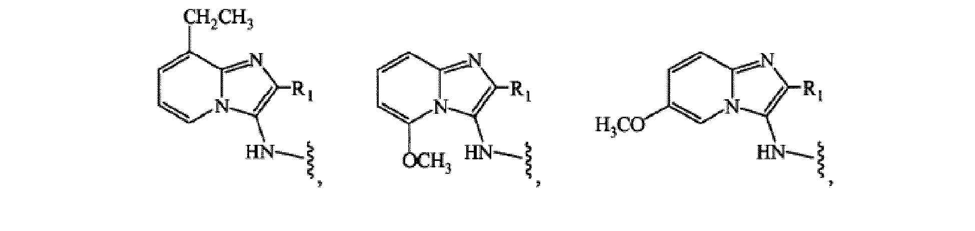 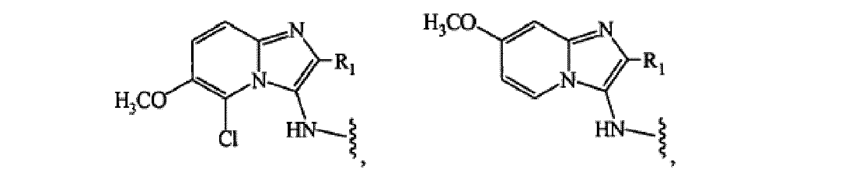 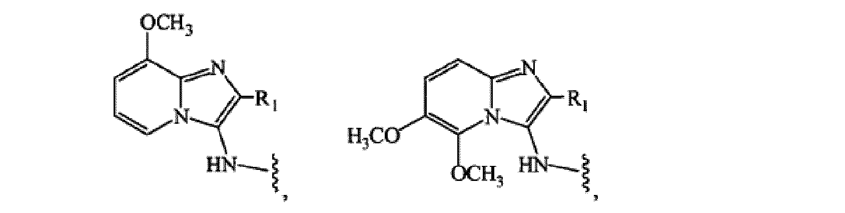 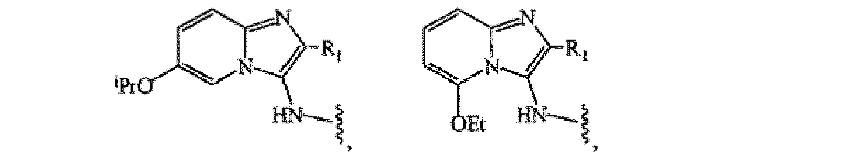 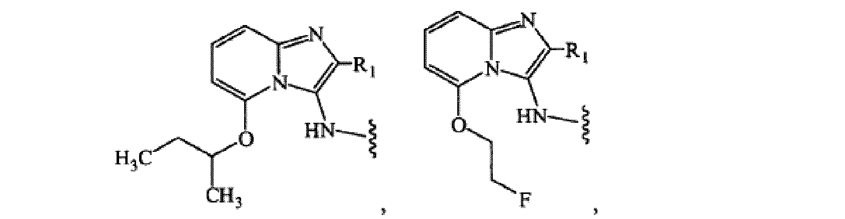 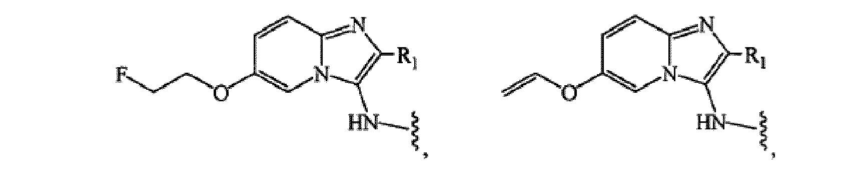 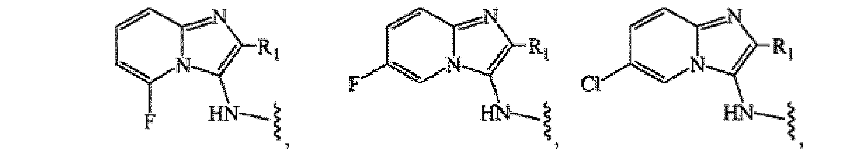 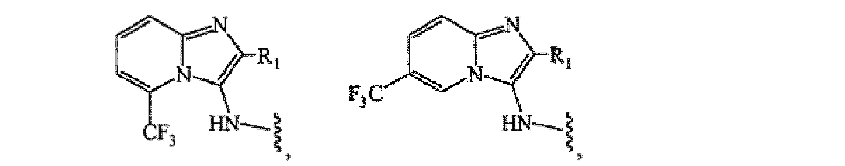 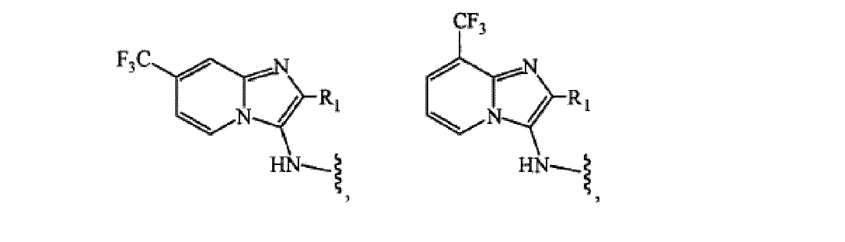 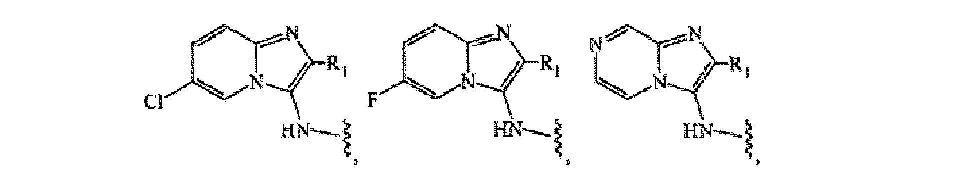 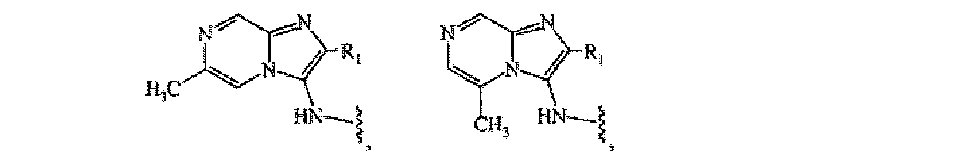 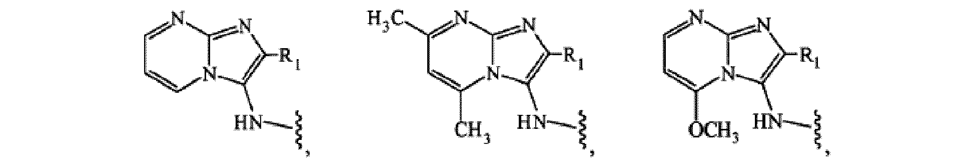 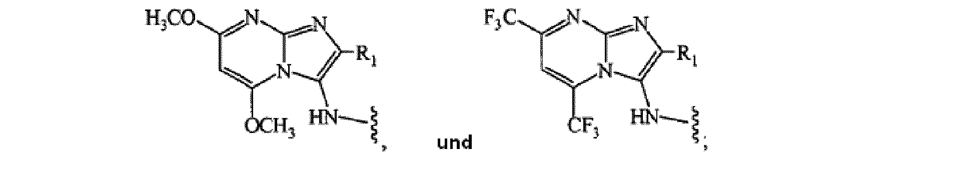
b) R₁ aus jener Gruppe gewählt ist, die besteht aus gegebenenfalls substituiertem Aryl, gegebenenfalls substituiertem Heteroaryl, einem gegebenenfalls substituierten carbocyclischen Ring und einem gegebenenfalls substituierten heterocyclischen Ring;
c) n für 1 oder 2 steht; und
d) zumindest ein Atom in der Verbindung ein Radioisotop ist;
und wobei, wenn eine Gruppe der Verbindung der Formel I als gegebenenfalls substituiert beschrieben ist, die Gruppe unsubstituiert ist, oder substituiert ist mit einem oder mehreren Substituenten, die unabhängig gewählt sind aus Alkyl, Alkenyl, Alkinyl, Cycloalkyl, Cycloalkenyl, Cycloalkinyl, Aryl, Heteroaryl, Heteroalicyclyl, Aralkyl, Heteroaralkyl, (Heteroalicyclyl)alkyl, Hydroxy, Alkoxy, Aryloxy, Acyl, Ester, Mercapto, Alkylthio, Arylthio, Cyano, Halogen, Carbonyl, Thiocarbonyl, O-Carbamyl, N-Carbamyl, O-Thiocarbamyl, N-Thiocarbamyl, C-Amido, N-Amido, S-Sulfonamido, N-Sulfonamido, C-Carboxy, geschütztes C-Carboxy, O-Carboxy, Isocyanato, Thiocyanato, Isothiocyanato, Nitro, Silyl, Sulfenyl, Sulfinyl, Sulfonyl, Haloalkyl, Haloalkoxy, Trihalomethansulfonyl, Trihalomethansulfonamido und Amino, einschließlich mono- und disubstituierter Aminogruppen.

2. Verbindung nach Anspruch 1, wobei das Radioisotop ein Isotop von Wasserstoff, Kohlenstoff, Stickstoff, Sauerstoff oder einem Halogen ist.

3. Verbindung nach Anspruch 2, wobei das Halogen Fluor oder lod ist.

4. Verbindung nach Anspruch 1, wobei das Radioisotop aus ¹²³I, ¹¹C und ¹⁸F gewählt ist.

5. Verbindung nach Anspruch 4, wobei das Radioisotop aus ¹¹C und ¹⁸F gewählt ist.

6. Verbindung nach Anspruch 4, wobei das Radioisotop ¹²³I ist.

7. Verbindung nach Anspruch 1, wobei R₁ gegebenenfalls substituiertes Heteroaryl ist.

8. Verbindung nach Anspruch 7, wobei das gegebenenfalls substituierte Heteroaryl gewählt ist aus jener Gruppe, die besteht aus Furan, Thiophen, Phthalazinon, Pyrrol, Oxazol, Thiazol, Imidazol, Pyrazol, Isoxazol, Isothiazol, Triazol, Thiadiazol, Pyran, Pyridin, Pyridazin, Pyrimidin, Pyrazin und Triazin.

9. Verbindung nach Anspruch 7, wobei das gegebenenfalls substituierte Heteroaryl Pyridyl oder Thiophenyl ist.

10. Verbindung nach Anspruch 1, wobei R₁ gegebenenfalls substituiertes Aryl ist.

11. Verbindung nach Anspruch 10, wobei das gegebenenfalls substituierte Aryl Phenyl ist.

12. Pharmazeutische Zusammensetzung, umfassend die Verbindung nach einem der Ansprüche 1 - 11, und eine physiologisch unbedenkliche Komponente, wie einen Träger, ein Verdünnungsmittel, ein Salz oder einen Exzipienten, oder eine Kombination davon.

13. Verbindung nach Anspruch 5 zur Verwendung in einem Verfahren zur PET-Bildgebung eines ersten Bereichs von einem Gewebe eines Patienten, wobei das Verfahren umfasst:
Verabreichen, an den Patienten, einer pharmazeutischen Zusammensetzung,
die eine Verbindung der Formel I, wie in Anspruch 5 definiert, umfasst;
Messen des Signals, das vom Radioisotop aus dem ersten Bereich des Gewebes ausgesendet wird; und
Vergleichen der aus dem ersten Bereich des Gewebes ausgesendeten Signalstärke mit einer aus einer Kontrollprobe ausgesendeten Signalstärke.

14. Verbindung nach Anspruch 6 zur Verwendung in einem Verfahren zur SPECT-Bildgebung eines ersten Bereichs von einem Gewebe eines Patienten, wobei das Verfahren umfasst:
Verabreichen, an den Patienten, einer pharmazeutischen Zusammensetzung,
die eine Verbindung der Formel I, wie in Anspruch 6 definiert, umfasst;
Messen des Signals, das vom Radioisotop aus dem ersten Bereich des Gewebes ausgesendet wird; und
Vergleichen der aus dem ersten Bereich des Gewebes ausgesendeten Signalstärke mit einer aus einer Kontrollprobe ausgesendeten Signalstärke.

15. Verfahren zur Bestimmung einer Verteilung von CB2-Rezeptoren in einem Gewebe, umfassend das Verabreichen einer radioaktiv markierten Verbindung der Formel I, wie in Anspruch 1 definiert, an das Gewebe *in vitro* und das Erhalten eines Abbilds des Gewebes.

## Revendications

1. Composé selon la formule I : 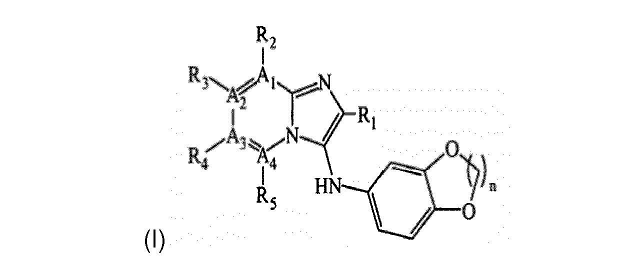 ou un sel pharmaceutiquement acceptable de celui-ci, dans lequel :
a) la fraction 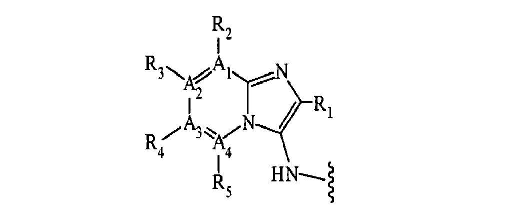 est sélectionnée à partir du groupe constitué par : 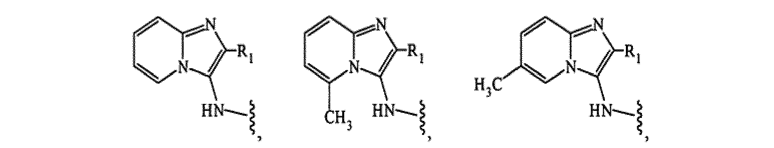 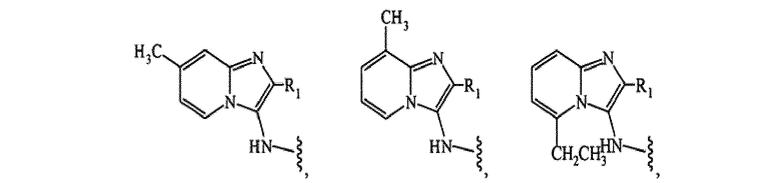 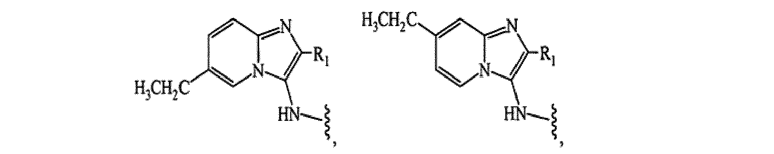 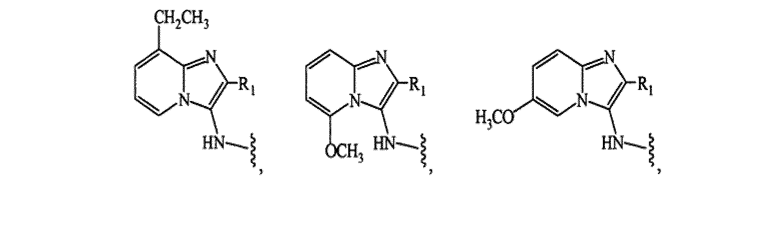 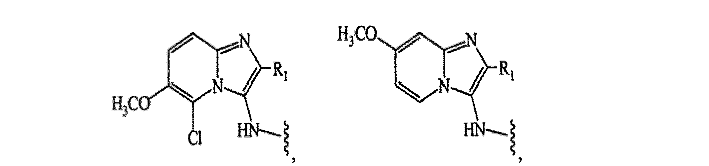 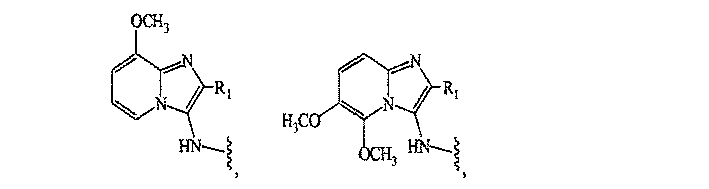 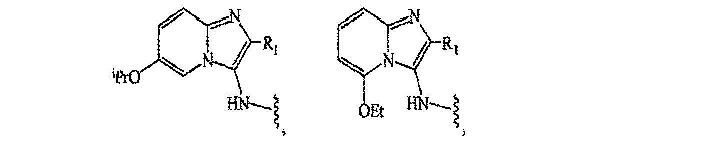 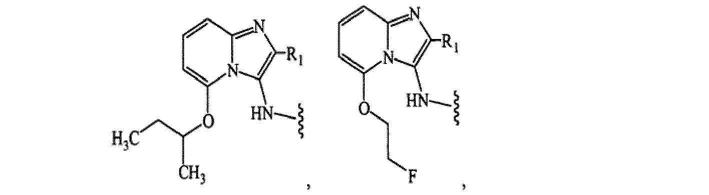 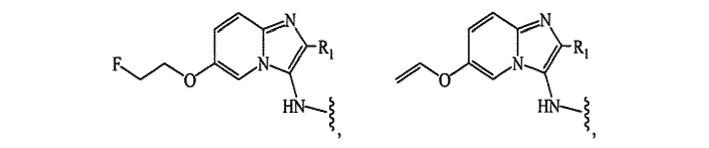 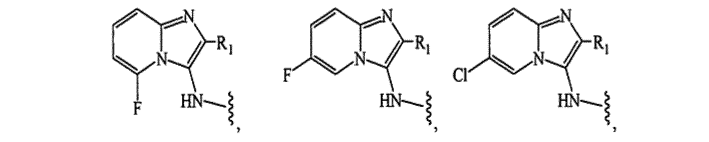 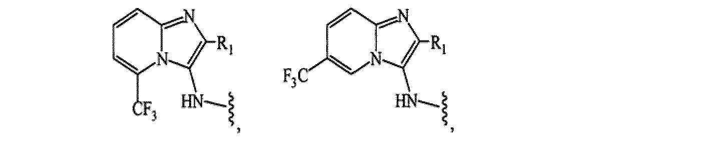 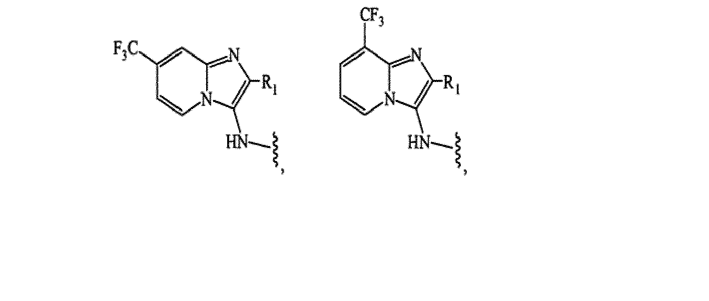 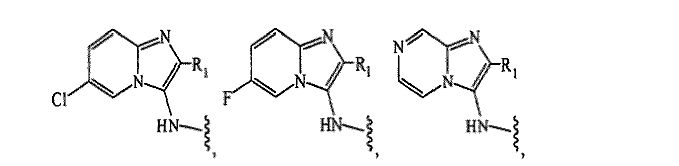 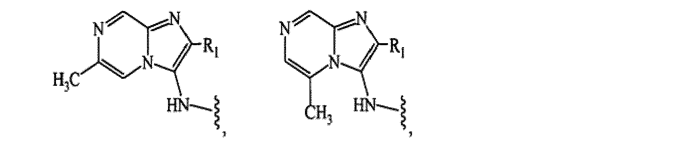 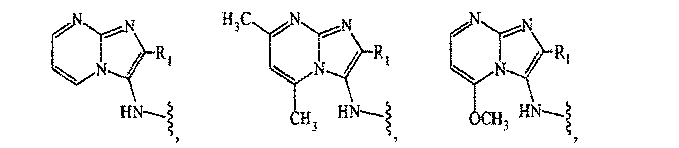 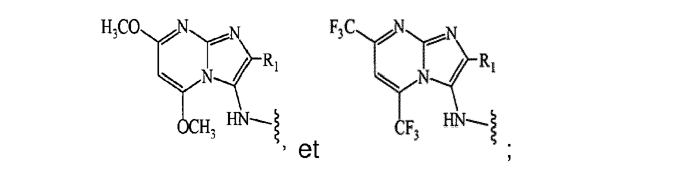
b) R₁ est sélectionné à partir du groupe constitué par un aryle éventuellement substitué, un hétéro-aryle éventuellement substitué, un composé carbocyclique éventuellement substitué et un composé hétérocyclique éventuellement substitué ;
c) n est égal à 1 ou 2 ; et,
d) au moins un atome dans le composé est un radio-isotope ;
et dans lequel, lorsqu'un groupe du composé de formule I est décrit comme étant éventuellement substitué, ledit groupe est soit non substitué, soit substitué par un ou plusieurs substituants sélectionnés de manière indépendante à partir des alkyle, alkényle, alkynyle, cycloalkyle, cycloalkényle, cycloalkynyle, aryle, hétéroaryle, hétéroalicyclyle, aralkyle, hétéroaralkyle, (hétéroalicyclyle)alkyle, hydroxy, alkoxy, aryloxy, acyle, ester, mercapto, alkylthio, arylthio, cyano, halogène, carbonyle, thiocarbonyle, O-carbamyle, N-carbamyle, O-thiocarbamyle, N-thiocarbamyle, C-amido, N-amido, S-sulfonamido, N-sulfonamido, C-carboxy, C-carboxy protégé, O-carboxy, isocyanato, thiocyanato, isothiocyanato, nitro, silyle, sulfényle, sulfinyle, sulfonyle, haloalkyle, haloalkoxy, trihalométhanesulfonyle, trihalométhanesulfonamido, et amino, comprenant les groupes animés mono- et di-substitués.

2. Composé selon la revendication 1, dans lequel le radio-isotope est un isotope d'hydrogène, de carbone, d'azote, d'oxygène, ou d'un halogène.

3. Composé selon la revendication 2, dans lequel l'halogène est du fluor ou de l'iode.

4. Composé selon la revendication 1, dans lequel le radio-isotope est sélectionné à partir de ¹²³I, 11C et ¹⁸F.

5. Composé selon la revendication 4, dans lequel le radio-isotope est sélectionné à partir de ¹¹C et ¹⁸F.

6. Composé selon la revendication 4, dans lequel le radio-isotope est le ¹²³I.

7. Composé selon la revendication 1, dans lequel R₁ est un hétéroaryle éventuellement substitué.

8. Composé selon la revendication 7, dans lequel l'hétéroaryle éventuellement substitué est sélectionné à partir du groupe constitué par les furane, thiophène, phthalazinone, pyrrole, oxazole, thiazole, imidazol, pyrazole, isoxazole, isothiazole, triazole, thiadiazole, pyrane, pyridine, pyridazine, pyrimidine, pyrazine et triazine.

9. Composé selon la revendication 7, dans lequel l'hétéroaryle éventuellement substitué est de la pyridyle ou du thiophényle.

10. Composé selon la revendication 1, dans lequel R₁ est un aryle éventuellement substitué.

11. Composé selon la revendication 10, dans lequel l'aryle éventuellement substitué est du phényle.

12. Composition pharmaceutique comprenant le composé selon l'une quelconque des revendications 1 à 11, et un composant physiologiquement acceptable tel qu'un support, un diluant, un sel ou un excipient, ou une association de ceux-ci.

13. Composé selon la revendication 5 destiné à une utilisation dans un procédé d'imagerie PET d'une première zone d'un tissu d'un sujet, le procédé comprenant :
l'administration au sujet d'une composition pharmaceutique comprenant un composé de formule I selon la revendication 5 ;
la mesure du signal émis par le radio-isotope à partir de la première zone du tissu ; et
la comparaison de l'intensité de signal émis à partir de la première zone du tissu à une intensité de signal émis à partir d'un échantillon de contrôle.

14. Composé selon la revendication 6, destiné à une utilisation dans un procédé d'imagerie SPECT d'une première zone d'un tissu d'un sujet, le procédé comprenant :
l'administration au sujet d'une composition pharmaceutique comprenant un composé de formule I selon la revendication 6 ;
la mesure du signal émis par le radio-isotope à partir de la première zone du tissu ; et
la comparaison de l'intensité du signal émis à partir de la première zone du tissu à une intensité de signal émis à partir d'un échantillon de contrôle.

15. Procédé de détermination d'une distribution de récepteurs de CB2 sur un tissu comprenant l'administration d'un composé radiomarqué de formule I selon la revendication 1 dans le tissu in vitro et l'obtention d'une image du tissu.
